# EUROPEAN PATENT APPLICATION

(11) **EP 3 872 084 A1**
(43) Date of publication of application: **01.09.2021**
(21) Application number: 20159879.4
(22) Date of filing: 27.02.2020
(51) Int. Cl.: C07K 7/08

(54) **EPI-X4 BASED PEPTIDES AND DERIVATIVES THEREOF**

(71) Applicant: Universität Ulm, 89081 Ulm (DE); Baden-Württemberg Stiftung gGmbH, 70174 Stuttgart (DE); German University in Cairo, 11835 Cairo (EG); Aarhus University, 8000 Aarhus C (DK); Universität Duisburg-Essen, 47057 Duisburg (DE)
(72) Inventor: MÜNCH, Jan, 89233 Neu-Ulm (DE); KIRCHHOFF, Frank, 89079 Ulm-Einsingen (DE); HARMS, Mirja, 89073 Ulm (DE); ABADI, Ashraf H., 11835 Cairo (EG); HABIB, Monika Maged Wadie, Cairo (EG); ZELIKIN, Alexander, 8200 Aarhus N (DK); CARMALI, Sheiliza, 8000 Aarhus C (DK); SANCHEZ GARCIA, Elsa, 44797 Bochum (DE); SOKKAR, Pandian, 625520 Tamil Nadu (IN)
(74) Representative: PATERIS Patentanwälte PartmbB

(57) **Abstract**

A peptide derivative selected from a list of peptide derivatives with a sequence derived from human serum albumin fragment EPI-X4 is provided, which binds to CXCR4 with about 1000-fold stronger efficiency than EPI-X4. The peptide derivatives comprise length variants as well as modifications by length variants, D-amino acids, coupling of fatty acids, cholesterol or polymers, acetyl substitutions of amino groups, aminations of carboxyl groups. Further provided are pharmaceutical compositions of the peptide derivative.

SEQUENCE LISTING

<110> Universität Ulm et al.

<120> EPI-X4 based peptides and derivatives thereof with antagonistic
activity against natural CXCR4

<130> K30054

<160> 102

<170> BiSSAP 1.3.6

<210> 1
<211> 16
<212> PRT
<213> Artificial Sequence

<220>
<223> modified peptide derived from HSA Sequence

<400> 1
Leu Val Arg Tyr Thr Lys Lys Val Pro Gln Val Ser Thr Pro Thr Leu
1               5                   10                  15

<210> 2
<211> 12
<212> PRT
<213> Artificial Sequence

<220>
<223> modified peptide derived from HSA Sequence

<400> 2
Ile Val Arg Trp Ser Lys Lys Val Pro Cys Val Ser
1               5                   10

<210> 3
<211> 12
<212> PRT
<213> Artificial Sequence

<220>
<223> modified peptide derived from HSA Sequence

<400> 3
Ile Leu Arg Trp Ser Lys Lys Val Pro Cys Val Ser
1               5                   10

<210> 4
<211> 12
<212> PRT
<213> Artificial Sequence

<220>
<223> modified peptide derived from HSA Sequence

<400> 4
Ile Phe Arg Trp Ser Lys Lys Val Pro Cys Val Ser
1               5                   10

<210> 5
<211> 12
<212> PRT
<213> Artificial Sequence

<220>
<223> modified peptide derived from HSA Sequence

<400> 5
Ile Val Arg Trp Ser Arg Lys Val Pro Cys Val Ser
1               5                   10

<210> 6
<211> 12
<212> PRT
<213> Artificial Sequence

<220>
<223> modified peptide derived from HSA Sequence

<400> 6
Ile Val Arg Trp Ser His Lys Val Pro Cys Val Ser
1               5                   10

<210> 7
<211> 12
<212> PRT
<213> Artificial Sequence

<220>
<223> modified peptide derived from HSA Sequence

<400> 7
Ile Val Arg Trp Ser Lys Lys Leu Pro Cys Val Ser
1               5                   10

<210> 8
<211> 12
<212> PRT
<213> Artificial Sequence

<220>
<223> modified peptide derived from HSA Sequence

<400> 8
Ile Val Arg Trp Ser Lys Lys Ile Pro Cys Val Ser
1               5                   10

<210> 9
<211> 12
<212> PRT
<213> Artificial Sequence

<220>

<223> modified peptide derived from HSA Sequence

<400> 9
Ile Val Arg Trp Ser Lys Lys Phe Pro Cys Val Ser
1               5                   10

<210> 10
<211> 12
<212> PRT
<213> Artificial Sequence

<220>
<223> modified peptide derived from HSA Sequence

<400> 10
Ile Leu Arg Trp Ser Arg Lys Val Pro Cys Val Ser
1               5                   10

<210> 11
<211> 12
<212> PRT
<213> Artificial Sequence

<220>
<223> modified peptide derived from HSA Sequence

<400> 11
Ile Leu Arg Trp Ser His Lys Val Pro Cys Val Ser
1               5                   10

<210> 12
<211> 12
<212> PRT
<213> Artificial Sequence

<220>
<223> modified peptide derived from HSA Sequence

<400> 12
Ile Phe Arg Trp Ser Arg Lys Val Pro Cys Val Ser
1               5                   10

<210> 13
<211> 12
<212> PRT
<213> Artificial Sequence

<220>
<223> modified peptide derived from HSA Sequence

<400> 13
Ile Phe Arg Trp Ser His Lys Val Pro Cys Val Ser
1               5                   10

<210> 14
<211> 12
<212> PRT
<213> Artificial Sequence

<220>
<223> modified peptide derived from HSA Sequence

<400> 14
Ile Val Arg Trp Ser Lys Lys Met Pro Cys Val Ser
1               5                   10

<210> 15
<211> 12
<212> PRT
<213> Artificial Sequence


<220>
<223> modified peptide derived from HSA Sequence

<400> 15
Ile Leu Arg Trp Ser Arg Lys Met Pro Cys Val Ser
1               5                   10

<210> 16
<211> 12
<212> PRT
<213> Artificial Sequence


<220>
<223> modified peptide derived from HSA Sequence

<220>
<221> NON_STD
<222> 11
<223> Xaa = D-Val

<400> 16
Ile Val Arg Trp Ser Lys Lys Val Pro Cys Xaa Ser
1               5                   10

<210> 17
<211> 12
<212> PRT
<213> Artificial Sequence


<220>
<223> modified peptide derived from HSA Sequence

<220>
<221> NON_STD
<222> 11
<223> Xaa = D-Val

<400> 17
Ile Leu Arg Trp Ser Arg Lys Val Pro Cys Xaa Ser
1               5                   10

<210> 18
<211> 12
<212> PRT
<213> Artificial Sequence

<220>
<223> modified peptide derived from HSA Sequence

<400> 18
Ile Ile Arg Trp Ser Arg Lys Met Pro Cys Val Ser
1               5                   10

<210> 19
<211> 12
<212> PRT
<213> Artificial Sequence


<220>
<223> modified peptide derived from HSA Sequence

<400> 19
Ile Met Arg Trp Ser Arg Lys Met Pro Cys Val Ser
1               5                   10

<210> 20
<211> 12
<212> PRT
<213> Artificial Sequence


<220>
<223> modified peptide derived from HSA Sequence

<400> 20
Ile Leu Arg Trp Ser Arg Lys Met Pro Cys Leu Ser
1               5                   10

<210> 21
<211> 12
<212> PRT
<213> Artificial Sequence


<220>
<223> modified peptide derived from HSA Sequence

<400> 21
Ile Leu Arg Trp Ser Arg Lys Met Pro Cys Phe Ser
1               5                   10

<210> 22
<211> 12
<212> PRT
<213> Artificial Sequence


<220>
<223> modified peptide derived from HSA Sequence

<400> 22
Ile Leu Arg Trp Ser Arg Lys Met Pro Cys Met Ser
1               5                   10

<210> 23
<211> 12
<212> PRT
<213> Artificial Sequence

<220>
<223> modified peptide derived from HSA Sequence

<400> 23
Ile Leu Arg Trp Ser Arg Lys Leu Pro Cys Val Ser
1               5                   10

<210> 24
<211> 12
<212> PRT
<213> Artificial Sequence



<220>
<223> modified peptide derived from HSA Sequence

<400> 24
Ile Leu Arg Trp Ser Arg Lys Phe Pro Cys Val Ser
1               5                   10

<210> 25
<211> 12
<212> PRT
<213> Artificial Sequence



<220>
<223> modified peptide derived from HSA Sequence

<400> 25
Ile Leu Arg Trp Ser Arg Lys Val Pro Ser Val Ser
1               5                   10

<210> 26
<211> 12
<212> PRT
<213> Artificial Sequence



<220>
<223> modified peptide derived from HSA Sequence

<400> 26
Ile Leu Arg Trp Ser Arg Lys Met Pro Ser Val Ser
1               5                   10

<210> 27
<211> 12
<212> PRT
<213> Artificial Sequence



<220>
<221> MOD_RES
<222> 1
<223> ACETYLATION

<220>
<223> modified peptide derived from HSA Sequence

<400> 27

Ser Leu Arg Trp Ser Arg Lys Met Pro Cys Val Ser
1               5                   10

<210> 28
<211> 12
<212> PRT
<213> Artificial Sequence

<220>
<221> NON_STD
<222> 1
<223> Xaa = acetylated D-Ser

<220>
<223> modified peptide derived from HSA Sequence

<400> 28
Xaa Leu Arg Trp Ser Arg Lys Met Pro Cys Val Ser
1               5                   10

<210> 29
<211> 12
<212> PRT
<213> Artificial Sequence

<220>
<223> modified peptide derived from HSA Sequence

<400> 29
Met Leu Arg Trp Ser Arg Lys Met Pro Cys Val Ser
1               5                   10

<210> 30
<211> 12
<212> PRT
<213> Artificial Sequence

<220>
<221> NON_STD
<222> 1
<223> Xaa = D-Met

<220>
<223> modified peptide derived from HSA Sequence

<400> 30
Xaa Leu Arg Trp Ser Arg Lys Met Pro Cys Val Ser
1               5                   10

<210> 31
<211> 12
<212> PRT
<213> Artificial Sequence

<220>
<221> NON_STD
<222> 1
<223> Xaa = D-Leu

<220>
<223> modified peptide derived from HSA Sequence

<400> 31
Xaa Leu Arg Trp Ser Arg Lys Met Pro Cys Val Ser
1               5                   10

<210> 32
<211> 12
<212> PRT
<213> Artificial Sequence


<220>
<221> NON_STD
<222> 1
<223> Xaa = D-Phe

<220>
<223> modified peptide derived from HSA Sequence

<400> 32
Xaa Leu Arg Trp Ser Arg Lys Met Pro Cys Val Ser
1               5                   10

<210> 33
<211> 12
<212> PRT
<213> Artificial Sequence


<220>
<223> modified peptide derived from HSA Sequence

<400> 33
Gly Leu Arg Trp Ser Arg Lys Met Pro Cys Val Ser
1               5                   10

<210> 34
<211> 12
<212> PRT
<213> Artificial Sequence


<220>
<221> MOD_RES
<222> 1
<223> ACETYLATION

<220>
<223> modified peptide derived from HSA Sequence

<400> 34
Ser Met Arg Trp Ser Arg Lys Met Pro Cys Val Ser
1               5                   10

<210> 35
<211> 12
<212> PRT
<213> Artificial Sequence


<220>

<221> NON_STD
<222> 1
<223> Xaa = acetylated D-Ser

<220>
<223> modified peptide derived from HSA Sequence

<400> 35
Xaa Met Arg Trp Ser Arg Lys Met Pro Cys Val Ser
1               5                   10

<210> 36
<211> 12
<212> PRT
<213> Artificial Sequence


<220>
<223> modified peptide derived from HSA Sequence

<400> 36
Met Met Arg Trp Ser Arg Lys Met Pro Cys Val Ser
1               5                   10

<210> 37
<211> 12
<212> PRT
<213> Artificial Sequence


<220>
<221> NON_STD
<222> 1
<223> Xaa = D-Met

<220>
<223> modified peptide derived from HSA Sequence

<400> 37
Xaa Met Arg Trp Ser Arg Lys Met Pro Cys Val Ser
1               5                   10

<210> 38
<211> 12
<212> PRT
<213> Artificial Sequence


<220>
<221> NON_STD
<222> 1
<223> Xaa = D-Leu

<220>
<223> modified peptide derived from HSA Sequence

<400> 38
Xaa Met Arg Trp Ser Arg Lys Met Pro Cys Val Ser
1               5                   10

<210> 39
<211> 12
<212> PRT

<213> Artificial Sequence

<220>
<221> NON_STD
<222> 1
<223> Xaa = D-Phe

<220>
<223> modified peptide derived from HSA Sequence

<400> 39
Xaa Met Arg Trp Ser Arg Lys Met Pro Cys Val Ser
1               5                   10

<210> 40
<211> 12
<212> PRT
<213> Artificial Sequence

<220>
<221> NON_STD
<222> 1
<223> Xaa = D-Gly

<220>
<223> modified peptide derived from HSA Sequence

<400> 40
Xaa Met Arg Trp Ser Arg Lys Met Pro Cys Val Ser
1               5                   10

<210> 41
<211> 12
<212> PRT
<213> Artificial Sequence

<220>
<223> modified peptide derived from HSA Sequence

<400> 41
Met Leu Arg Trp Ser Arg Lys Leu Pro Cys Val Ser
1               5                   10

<210> 42
<211> 12
<212> PRT
<213> Artificial Sequence

<220>
<221> NON_STD
<222> 1
<223> Xaa = D-Met

<220>
<223> modified peptide derived from HSA Sequence

<400> 42
Xaa Leu Arg Trp Ser Arg Lys Leu Pro Cys Val Ser
1               5                   10

<210> 43
<211> 12
<212> PRT
<213> Artificial Sequence

<220>
<223> modified peptide derived from HSA Sequence

<220>
<221> NON_STD
<222> 2
<223> Xaa = D-Leu

<400> 43
Ile Xaa Arg Trp Ser Arg Lys Leu Pro Cys Val Ser
1               5                   10

<210> 44
<211> 12
<212> PRT
<213> Artificial Sequence

<220>
<223> modified peptide derived from HSA Sequence

<220>
<221> NON_STD
<222> 2
<223> Xaa = D-Leu

<400> 44
Ile Xaa Arg Trp Ser Arg Lys Met Pro Cys Val Ser
1               5                   10

<210> 45
<211> 12
<212> PRT
<213> Artificial Sequence

<220>
<223> modified peptide derived from HSA Sequence

<220>
<221> NON_STD
<222> 2
<223> Xaa = D-Leu

<400> 45
Met Xaa Arg Trp Ser Arg Lys Leu Pro Cys Val Ser
1               5                   10

<210> 46
<211> 12
<212> PRT
<213> Artificial Sequence

<220>
<223> modified peptide derived from HSA Sequence

<220>
<221> NON_STD
<222> 2
<223> Xaa = D-Leu

<400> 46
Met Xaa Arg Trp Ser Arg Lys Met Pro Cys Val Ser
1               5                   10

<210> 47
<211> 9
<212> PRT
<213> Artificial Sequence

<220>
<223> modified peptide derived from HSA Sequence

<220>
<221> MOD_RES
<222> 9
<223> Amination

<400> 47
Ile Val Arg Trp Ser Lys Lys Val Pro
1               5

<210> 48
<211> 7
<212> PRT
<213> Artificial Sequence

<220>
<223> modified peptide derived from HSA Sequence

<220>
<221> MOD_RES
<222> 7
<223> Amination

<400> 48
Ile Val Arg Trp Ser Lys Lys
1               5

<210> 49
<211> 9
<212> PRT
<213> Artificial Sequence

<220>
<223> modified peptide derived from HSA Sequence

<220>
<221> MOD_RES
<222> 9
<223> Amination

<400> 49
Ile Leu Arg Trp Ser Arg Lys Leu Pro
1               5

<210> 50
<211> 7
<212> PRT
<213> Artificial Sequence

<220>
<223> modified peptide derived from HSA Sequence

<220>
<221> MOD_RES
<222> 7
<223> Amination

<400> 50
Ile Leu Arg Trp Ser Arg Lys
1               5

<210> 51
<211> 12
<212> PRT
<213> Artificial Sequence

<220>
<223> modified peptide derived from HSA Sequence

<400> 51
Ile Leu Arg Trp Ser Arg Lys Leu Pro Ser Val Ser
1               5                   10

<210> 52
<211> 12
<212> PRT
<213> Artificial Sequence

<220>
<221> NON_STD
<222> 1
<223> Xaa = D-Leu

<220>
<223> modified peptide derived from HSA Sequence

<220>
<221> MOD_RES
<222> 7
<223> Palmitic acid with glutamate linker

<400> 52
Xaa Leu Arg Trp Ser Arg Lys Met Pro Cys Val Ser
1               5                   10

<210> 53
<211> 12
<212> PRT
<213> Artificial Sequence

<220>
<221> NON_STD

<222> 1
<223> Xaa = D-Leu

<220>
<223> modified peptide derived from HSA Sequence

<220>
<221> MOD_RES
<222> 7
<223> Palmitic acid

<400> 53
Xaa Leu Arg Trp Ser Arg Lys Met Pro Cys Val Ser
1               5                   10

<210> 54
<211> 12
<212> PRT
<213> Artificial Sequence

<220>
<223> modified peptide derived from HSA Sequence

<220>
<221> MOD_RES
<222> 7
<223> Palmitic acid with Glutamate linker

<400> 54
Ile Leu Arg Trp Ser Arg Lys Leu Pro Cys Val Ser
1               5                   10

<210> 55
<211> 12
<212> PRT
<213> Artificial Sequence

<220>
<223> modified peptide derived from HSA Sequence

<220>
<221> MOD_RES
<222> 7
<223> Palmitic acid

<400> 55
Ile Leu Arg Trp Ser Arg Lys Leu Pro Cys Val Ser
1               5                   10

<210> 56
<211> 12
<212> PRT
<213> Artificial Sequence

<220>
<223> modified peptide derived from HSA Sequence

<220>
<221> MOD_RES
<222> 11

<223> Palmitic acid with Glutamate linker

<400> 56
Ile Leu Arg Trp Ser Arg Lys Leu Pro Cys Lys Ser
1               5                   10

<210> 57
<211> 12
<212> PRT
<213> Artificial Sequence

<220>
<223> modified peptide derived from HSA Sequence

<400> 57
Ile Tyr Arg Trp Ser Arg Lys Met Pro Cys Leu Ser
1               5                   10

<210> 58
<211> 12
<212> PRT
<213> Artificial Sequence

<220>
<223> modified peptide derived from HSA Sequence

<220>
<221> MOD_RES
<222> 7
<223> Palmitic acid with Glutamate linker

<400> 58
Ile Leu Arg Trp Ser Arg Lys Met Pro Cys Leu Ser
1               5                   10

<210> 59
<211> 12
<212> PRT
<213> Artificial Sequence

<220>
<223> modified peptide derived from HSA Sequence

<220>
<221> MOD_RES
<222> 7
<223> Palmitic acid

<400> 59
Ile Leu Arg Trp Ser Arg Lys Met Pro Cys Leu Ser
1               5                   10

<210> 60
<211> 12
<212> PRT
<213> Artificial Sequence

<220>
<223> modified peptide derived from HSA Sequence

<400> 60
Ile Val Arg Trp Ser Lys Lys Val Pro Ser Val Ser
1               5                   10

<210> 61
<211> 7
<212> PRT
<213> Artificial Sequence

<220>
<223> modified peptide derived from HSA Sequence

<220>
<221> MOD_RES
<222> 6
<223> Palmitic acid

<220>
<221> MOD_RES
<222> 7
<223> Amination

<400> 61
Ile Val Arg Trp Ser Lys Lys
1               5

<210> 62
<211> 7
<212> PRT
<213> Artificial Sequence

<220>
<223> modified peptide derived from HSA Sequence

<220>
<221> MOD_RES
<222> 7
<223> Palmitic acid

<220>
<221> MOD_RES
<222> 7
<223> Amination

<400> 62
Ile Val Arg Trp Ser Lys Lys
1               5

<210> 63
<211> 9
<212> PRT
<213> Artificial Sequence

<220>
<223> modified peptide derived from HSA Sequence

<220>
<221> MOD_RES
<222> 6

<223> Palmitic acid

<220>
<221> MOD_RES
<222> 9
<223> Amination

<400> 63
Ile Val Arg Trp Ser Lys Lys Val Pro
1               5

<210> 64
<211> 9
<212> PRT
<213> Artificial Sequence

<220>
<223> modified peptide derived from HSA Sequence

<220>
<221> MOD_RES
<222> 7
<223> Palmitic acid

<220>
<221> MOD_RES
<222> 9
<223> Amination

<400> 64
Ile Val Arg Trp Ser Lys Lys Val Pro
1               5

<210> 65
<211> 12
<212> PRT
<213> Artificial Sequence

<220>
<223> modified peptide derived from HSA Sequence

<220>
<221> MOD_RES
<222> 6
<223> Palmitic acid

<400> 65
Ile Val Arg Trp Ser Lys Lys Val Pro Cys Val Ser
1               5                   10

<210> 66
<211> 12
<212> PRT
<213> Artificial Sequence

<220>
<223> modified peptide derived from HSA Sequence

<220>
<221> MOD_RES

<222> 7
<223> Palmitic acid

<400> 66
Ile Val Arg Trp Ser Lys Lys Val Pro Cys Val Ser
1               5                   10

<210> 67
<211> 7
<212> PRT
<213> Artificial Sequence

<220>
<223> modified peptide derived from HSA Sequence

<220>
<221> MOD_RES
<222> 7
<223> Palmitic acid

<220>
<221> MOD_RES
<222> 7
<223> Amination

<400> 67
Ile Leu Arg Trp Ser Arg Lys
1               5

<210> 68
<211> 9
<212> PRT
<213> Artificial Sequence

<220>
<223> modified peptide derived from HSA Sequence

<220>
<221> MOD_RES
<222> 7
<223> Palmitic acid

<220>
<221> MOD_RES
<222> 9
<223> Amination

<400> 68
Ile Leu Arg Trp Ser Arg Lys Leu Pro
1               5

<210> 69
<211> 8
<212> PRT
<213> Artificial Sequence

<220>
<223> modified peptide derived from HSA Sequence

<220>

<221> MOD_RES
<222> 7
<223> Palmitic acid

<220>
<221> MOD_RES
<222> 8
<223> Amination

<400> 69
Ile Leu Arg Trp Ser Arg Lys Leu
1               5

<210> 70
<211> 7
<212> PRT
<213> Artificial Sequence


<220>
<221> NON_STD
<222> 1
<223> Xaa = D-Ile

<220>
<223> modified peptide derived from HSA Sequence

<220>
<221> MOD_RES
<222> 7
<223> Amination

<400> 70
Xaa Leu Arg Trp Ser Arg Lys
1               5

<210> 71
<211> 9
<212> PRT
<213> Artificial Sequence


<220>
<221> NON_STD
<222> 1
<223> Xaa = D-Ile

<220>
<223> modified peptide derived from HSA Sequence

<220>
<221> MOD_RES
<222> 9
<223> Amination

<400> 71
Xaa Leu Arg Trp Ser Arg Lys Leu Pro
1               5

<210> 72
<211> 9
<212> PRT
<213> Artificial Sequence

<220>
<221> NON_STD
<222> 1
<223> Xaa = Ile

<220>
<223> modified peptide derived from HSA Sequence

<220>
<221> MOD_RES
<222> 7
<223> Palmitic acid

<220>
<221> MOD_RES
<222> 9
<223> Amination

<400> 72
Xaa Leu Arg Trp Ser Arg Lys Leu Pro
1               5

<210> 73
<211> 12
<212> PRT
<213> Artificial Sequence


<220>
<221> NON_STD
<222> 1
<223> Xaa = D-Leu

<220>
<223> modified peptide derived from HSA Sequence

<220>
<221> MOD_RES
<222> 7
<223> Palmitic acid

<400> 73
Xaa Met Arg Trp Ser Arg Lys Met Pro Cys Val Ser
1               5                   10

<210> 74
<211> 12
<212> PRT
<213> Artificial Sequence


<220>
<223> modified peptide derived from HSA Sequence

<220>
<221> NON_STD
<222> 2
<223> Xaa = D-Leu

<220>
<221> MOD_RES

<222> 7
<223> Palmitic acid

<400> 74
Met Xaa Arg Trp Ser Arg Lys Leu Pro Cys Val Ser
1               5                   10

<210> 75
<211> 9
<212> PRT
<213> Artificial Sequence

<220>
<221> NON_STD
<222> 1
<223> Xaa = D-Leu

<220>
<223> modified peptide derived from HSA Sequence

<220>
<221> MOD_RES
<222> 7
<223> Palmitic acid

<220>
<221> MOD_RES
<222> 9
<223> Amination

<400> 75
Xaa Met Arg Trp Ser Arg Lys Met Pro
1               5

<210> 76
<211> 9
<212> PRT
<213> Artificial Sequence

<220>
<223> modified peptide derived from HSA Sequence

<220>
<221> NON_STD
<222> 2
<223> Xaa = D-Leu

<220>
<221> MOD_RES
<222> 7
<223> Palmitic acid

<220>
<221> MOD_RES
<222> 9
<223> Amination

<400> 76
Met Xaa Arg Trp Ser Arg Lys Leu Pro
1               5

<210> 77
<211> 7
<212> PRT
<213> Artificial Sequence

<220>
<221> NON_STD
<222> 1
<223> Xaa = D-Leu

<220>
<223> modified peptide derived from HSA Sequence

<220>
<221> MOD_RES
<222> 7
<223> Palmitic acid

<220>
<221> MOD_RES
<222> 7
<223> Amination

<400> 77
Xaa Met Arg Trp Ser Arg Lys
1               5

<210> 78
<211> 7
<212> PRT
<213> Artificial Sequence

<220>
<223> modified peptide derived from HSA Sequence

<220>
<221> NON_STD
<222> 2
<223> Xaa = D-Leu

<220>
<221> MOD_RES
<222> 7
<223> Palmitic acid

<220>
<221> MOD_RES
<222> 7
<223> Amination

<400> 78
Met Xaa Arg Trp Ser Arg Lys
1               5

<210> 79
<211> 12
<212> PRT
<213> Artificial Sequence

<220>

<223> modified peptide derived from HSA Sequence

<220>
<221> MOD_RES
<222> 7
<223> Stearic acid

<400> 79
Ile Leu Arg Trp Ser Arg Lys Leu Pro Cys Val Ser
1               5                   10

<210> 80
<211> 12
<212> PRT
<213> Artificial Sequence


<220>
<223> modified peptide derived from HSA Sequence

<220>
<221> MOD_RES
<222> 7
<223> Oleic acid

<400> 80
Ile Leu Arg Trp Ser Arg Lys Leu Pro Cys Val Ser
1               5                   10

<210> 81
<211> 12
<212> PRT
<213> Artificial Sequence


<220>
<223> modified peptide derived from HSA Sequence

<220>
<221> MOD_RES
<222> 7
<223> Cholesterol

<400> 81
Ile Leu Arg Trp Ser Arg Lys Leu Pro Cys Val Ser
1               5                   10

<210> 82
<211> 12
<212> PRT
<213> Artificial Sequence


<220>
<221> MOD_RES
<222> 1
<223> ACETYLATION

<220>
<223> modified peptide derived from HSA Sequence

<400> 82
Ile Leu Arg Trp Ser Arg Lys Leu Pro Cys Val Ser

1                 5                   10

<210> 83
<211> 12
<212> PRT
<213> Artificial Sequence

<220>
<221> MOD_RES
<222> 1
<223> ACETYLATION

<220>
<221> NON_STD
<222> 1
<223> Xaa = D-Ile

<220>
<223> modified peptide derived from HSA Sequence

<400> 83
Xaa Leu Arg Trp Ser Arg Lys Leu Pro Cys Val Ser
1                 5                   10

<210> 84
<211> 12
<212> PRT
<213> Artificial Sequence

<220>
<221> MOD_RES
<222> 1
<223> ACETYLATION

<220>
<223> modified peptide derived from HSA Sequence

<400> 84
Met Leu Arg Trp Ser Arg Lys Leu Pro Cys Val Ser
1                 5                   10

<210> 85
<211> 12
<212> PRT
<213> Artificial Sequence

<220>
<221> MOD_RES
<222> 1
<223> ACETYLATION

<220>
<221> NON_STD
<222> 1
<223> Xaa = D-Met

<220>
<223> modified peptide derived from HSA Sequence

<400> 85

Xaa Leu Arg Trp Ser Arg Lys Leu Pro Cys Val Ser
1               5                   10

<210> 86
<211> 12
<212> PRT
<213> Artificial Sequence

<220>
<223> modified peptide derived from HSA Sequence

<400> 86
Val Leu Arg Trp Ser Arg Lys Leu Pro Cys Val Ser
1               5                   10

<210> 87
<211> 12
<212> PRT
<213> Artificial Sequence

<220>
<221> NON_STD
<222> 1
<223> D-Val

<220>
<223> modified peptide derived from HSA Sequence

<400> 87
Xaa Leu Arg Trp Ser Arg Lys Leu Pro Cys Val Ser
1               5                   10

<210> 88
<211> 8
<212> PRT
<213> Artificial Sequence

<220>
<223> modified peptide derived from HSA Sequence

<220>
<221> MOD_RES
<222> 8
<223> Palmitic acid with glutamate linker

<220>
<221> MOD_RES
<222> 8
<223> Amination

<400> 88
Ile Leu Arg Trp Ser Arg Lys Lys
1               5

<210> 89
<211> 7
<212> PRT
<213> Artificial Sequence

<220>
<223> modified peptide derived from HSA Sequence

<220>
<221> MOD_RES
<222> 7
<223> Palmitic acid with glutamate linker

<220>
<221> MOD_RES
<222> 7
<223> Amination

<400> 89
Ile Leu Arg Trp Ser Arg Lys
1               5

<210> 90
<211> 9
<212> PRT
<213> Artificial Sequence

<220>
<223> modified peptide derived from HSA Sequence

<220>
<221> MOD_RES
<222> 9
<223> Palmitic acid with glutamate linker

<220>
<221> MOD_RES
<222> 9
<223> Amination

<400> 90
Ile Leu Arg Trp Ser Arg Lys Leu Lys
1               5

<210> 91
<211> 7
<212> PRT
<213> Artificial Sequence

<220>
<221> NON_STD
<222> 1
<223> Xaa = D-Leu

<220>
<223> modified peptide derived from HSA Sequence

<220>
<221> MOD_RES
<222> 7
<223> Palmitic acid with glutamate linker

<220>
<221> MOD_RES
<222> 7
<223> Amination

<400> 91
Xaa Met Arg Trp Ser Arg Lys
1               5

<210> 92
<211> 8
<212> PRT
<213> Artificial Sequence

<220>
<221> NON_STD
<222> 1
<223> D-Leu

<220>
<223> modified peptide derived from HSA Sequence

<220>
<221> MOD_RES
<222> 8
<223> Palmitic acid with glutamate linker

<220>
<221> MOD_RES
<222> 8
<223> Amination

<400> 92
Xaa Met Arg Trp Ser Arg Lys Lys
1               5

<210> 93
<211> 7
<212> PRT
<213> Artificial Sequence

<220>
<221> NON_STD
<222> 1
<223> Xaa = D-Met

<220>
<223> modified peptide derived from HSA Sequence

<220>
<221> MOD_RES
<222> 7
<223> Palmitic acid with glutamate linker

<220>
<221> MOD_RES
<222> 7
<223> Amination

<400> 93
Xaa Leu Arg Trp Ser Arg Lys
1               5

<210> 94
<211> 8

<212> PRT
<213> Artificial Sequence

<220>
<221> NON_STD
<222> 1
<223> Xaa = D-Met

<220>
<223> modified peptide derived from HSA Sequence

<220>
<221> MOD_RES
<222> 8
<223> Palmitic acid with glutamate linker

<220>
<221> MOD_RES
<222> 8
<223> Amination

<400> 94
Xaa Leu Arg Trp Ser Arg Lys Lys
1               5

<210> 95
<211> 12
<212> PRT
<213> Artificial Sequence

<220>
<223> modified peptide derived from HSA Sequence

<220>
<221> MOD_RES
<222> 7
<223> Stearic acid with glutamate linker

<400> 95
Ile Leu Arg Trp Ser Arg Lys Leu Pro Cys Val Ser
1               5                   10

<210> 96
<211> 13
<212> PRT
<213> Artificial Sequence

<220>
<223> modified peptide derived from HSA Sequence

<220>
<221> MOD_RES
<222> 8
<223> Stearic acid with glutamate linker

<400> 96
Ile Leu Arg Trp Ser Arg Lys Lys Leu Pro Cys Val Ser
1               5                   10

<210> 97

<211> 12
<212> PRT
<213> Artificial Sequence

<220>
<223> modified peptide derived from HSA Sequence

<220>
<221> MOD_RES
<222> 7
<223> ACETYLATION

<400> 97
Ile Leu Arg Trp Ser Arg Lys Leu Pro Cys Val Ser
1               5                   10

<210> 98
<211> 12
<212> PRT
<213> Artificial Sequence

<220>
<223> modified peptide derived from HSA Sequence

<220>
<221> MOD_RES
<222> 7
<223> Decanoic acid with glutamate linker

<400> 98
Ile Leu Arg Trp Ser Arg Lys Leu Pro Cys Val Ser
1               5                   10

<210> 99
<211> 12
<212> PRT
<213> Artificial Sequence

<220>
<223> modified peptide derived from HSA Sequence

<220>
<221> MOD_RES
<222> 7
<223> Myristic acid with glutamate linker

<400> 99
Ile Leu Arg Trp Ser Arg Lys Leu Pro Cys Val Ser
1               5                   10

<210> 100
<211> 12
<212> PRT
<213> Artificial Sequence

<220>
<223> modified peptide derived from HSA Sequence

<220>

<221> MOD_RES
<222> 7
<223> Stearic acid with glutamate linker

<400> 100
Ile Leu Arg Trp Ser Arg Lys Leu Pro Cys Val Ser
1               5                   10

<210> 101
<211> 13
<212> PRT
<213> Artificial Sequence


<220>
<223> modified peptide derived from HSA Sequence

<220>
<221> MOD_RES
<222> 13
<223> dodecane tetraacetic acid

<400> 101
Ile Leu Arg Trp Ser Arg Lys Leu Pro Cys Val Ser Lys
1               5                   10

<210> 102
<211> 13
<212> PRT
<213> Artificial Sequence


<220>
<223> modified peptide derived from HSA Sequence

<220>
<221> MOD_RES
<222> 13
<223> dodecane tetraacetic acid

<400> 102
Ile Leu Arg Trp Ser Arg Lys Leu Pro Ser Val Ser Lys
1               5                   10

## Description

### Field of the invention

The present invention concerns peptides and derivates thereof binding to C-X-C chemokine receptor type 4, therapeutic uses of the peptides and methods for manufacturing the peptides of the invention.

### Background of the invention

CXC chemokine receptor type 4 (CXCR4) is expressed in many cells of the hematopoietic system, particularly stem cells and tumor cells. CXCR4 is a G protein-coupled receptor (GPCR) with stromal cell-derived factor-1 (SDF-1 or CXCL12) as sole chemokine ligand. CXCR4 is involved in multiple developmental and physiological processes including stem cell homing to the liver and bone marrow as well as participation in organogenesis and healing processes of the organs and wounds. In terms of pathophysiology, CXCR4 plays a role in various disease processes, including tumor growth, cancer cell metastasis, and inflammation. In addition, CXCR4 is a major co-receptor for HIV-1 entry into target cells.

Its involvement in many processes makes CXCR4 an attractive target in intervening with cancer cell proliferation, differentiation, and metastasis as well as inflammatory diseases. So far only one CXCR4 antagonist has obtained clinical approval (AMD3100, Hendrix et al., 2000) but only to mobilize hematopoietic stem cells in cancer patients with lymphoma and multiple myeloma.

A peptide derived from Human Serum Albumin amino acid sequence (EPI-X4 (SEQ ID NO.: 1)) has been shown to bind CXCR4, thereby inhibiting the binding of natural ligand CXCL12 (EP 2 162 462 B1). Peptides derived from EPI-X4 (SEQ ID NO.: 1) have been shown to bind CXCR4 more effectively than the original peptide (EP 3 007 717 A1). Yet, effective inhibition of binding of CXCL12 requests higher nanomolar values of these peptides, and the half-life period of the peptides is limited due to degradation by protease activity. There is a need for CXCR4 antagonists binding CXCR4 more effectively and with an increased blood stability and pharmacokinetic properties.

### Summary

A first aspect of the present invention is related to a peptide consisting of one of the following amino acid sequences, selected from any of the groups 1 to 10, wherein
- Group 1 consists of

| | |
|---|---|
| ILRWSRKMPCVS | (SEQ ID NO.: 15, JM#13) |
| IMRWSRKMPCVS | (SEQ ID NO.: 19, JM#17) |
| ILRWSRKMPCLS | (SEQ ID NO.: 20, JM#18) |
| ILRWSRKMPCMS | (SEQ ID NO.: 22, JM#20) |
| ILRWSRKLPCVS | (SEQ ID NO.: 23, JM#21) |
| ILRWSRKFPCVS | (SEQ ID NO.: 24, JM#22) |
| ILRWSRK(Pal)LPCVS | (SEQ ID NO.: 55, JM#144) |
| ILRWSRK(Glu-Pal)-NH2 | (SEQ ID NO.: 89, JM#192) |
| d-LMRWSRK(Glu-Pal)-NH2 | (SEQ ID NO.: 91, JM#194) |

- Group 2 consists of

| | |
|---|---|
| ILRWSRKMPCFS | (SEQ ID NO.: 21, JM#19) |
| ILRWSRK(Pal)L-NH2 | (SEQ ID NO.: 69, JM#172) |
| d-LMRWSRK(Pal)MP-NH2 | (SEQ ID NO.: 75, JM#178) |
| d-LMRWSRK(Pal)-NH2 | (SEQ ID NO.: 77, JM#180) |
| ILRWSRK(Ole)LPCVS | (SEQ ID NO.: 80, JM#183) |

- Group 3 consists of

| | |
|---|---|
| d-LLRWSRK(Pal)MPCVS | (SEQ ID NO.: 53, JM#141) |
| IVRWSK(Pal)KVP-NH2 | (SEQ ID NO.: 63, JM#166) |
| IVRWSKK(Pal)VP-NH2 | (SEQ ID NO.: 64, JM#167) |
| IVRWSKK(Pal)VPCVS | (SEQ ID NO.: 66, JM#169) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRKK(Glu-Pal)-NH2 | (SEQ ID NO.: 88, JM#191) |
| ILRWSRKLK(Glu-Pal)-NH2 | (SEQ ID NO.: 90, JM#193) |
| ILRWSRKK(Glu-Ste)LPCVS | (SEQ ID NO.: 96, JM#199) |
| ILRWSRK(Glu-Dec)LPCVS | (SEQ ID NO.: 98, JM#203) |
| ILRWSRK(Glu-Ste)LPCVS | (SEQ ID NO.: 100, JM#205) |

- Group 4 consists of

| | |
|---|---|
| ILRWSRKVPCVS | (SEQ ID NO.: 10, JM#8) |
| IFRWSRKVPCVS | (SEQ ID NO.: 12, JM#10) |
| MLRWSRKMPCVS | (SEQ ID NO.: 29, JM#27) |
| MMRWSRKMPCVS | (SEQ ID NO.: 36, JM#34) |
| MLRWSRKLPCVS | (SEQ ID NO.: 41, JM#39) |
| ILRWSRKLPSVS | (SEQ ID NO.: 51, JM#122) |
| d-LLRWSRK(Glu-Pal)MPCVS | (SEQ ID NO.: 52, JM#140) |
| ILRWSRK(Pal)MPCLS | (SEQ ID NO.: 59, JM#149) |
| d-LMRWSRKK(Glu-Pal)-NH2 | (SEQ ID NO.: 92, JM#195) |
| ILRWSRK-AcLPCVS | (SEQ ID NO.: 97, JM#200) |

- Group 5 consisting of

| | |
|---|---|
| ILRWSKKVPCVS | (SEQ ID NO.: 3, JM#1) |
| IFRWSKKVPCVS | (SEQ ID NO.: 4, JM#2) |
| IVRWSRKVPCVS | (SEQ ID NO.: 5, JM#3) |
| IVRWSHKVPCVS | (SEQ ID NO.: 6, JM#4) |
| IVRWSKKLPCVS | (SEQ ID NO.: 7, JM#5) |
| IVRWSKKIPCVS | (SEQ ID NO.: 8, JM#6) |
| IVRWSKKFPCVS | (SEQ ID NO.: 9, JM#7) |
| ILRWSHKVPCVS | (SEQ ID NO.: 11, JM#9) |
| IFRWSHKVPCVS | (SEQ ID NO.: 13, JM#11) |
| IVRWSKKMPCVS | (SEQ ID NO.: 14, JM#12) |
| IVRWSKKVPCd-VS | (SEQ ID NO.: 16, JM#14) |
| ILRWSRKVPCd-VS | (SEQ ID NO.: 17, JM#15) |
| IIRWSRKMPCVS | (SEQ ID NO.: 18, JM#16) |
| ILRWSRKVPSVS | (SEQ ID NO.: 25, JM#23) |
| ILRWSRKMPSVS | (SEQ ID NO.: 26, JM#24) |
| Ac-SLRWSRKMPCVS | (SEQ ID NO.: 27, JM#25) |
| d-Ac-SLRWSRKMPCVS | (SEQ ID NO.: 28, JM#26) |
| d-MLRWSRKMPCVS | (SEQ ID NO.: 30, JM#28) |
| d-LLRWSRKMPCVS | (SEQ ID NO.: 31, JM#29) |
| d-FLRWSRKMPCVS | (SEQ ID NO.: 32, JM#30) |
| GLRWSRKMPCVS | (SEQ ID NO.: 33, JM#31) |
| Ac-SMRWSRKMPCVS | (SEQ ID NO.: 34, JM#32) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-FMRWSRKMPCVS | (SEQ ID NO.: 39, JM#37) |
| d-GMRWSRKMPCVS | (SEQ ID NO.: 40, JM#38) |
| d-MLRWSRKLPCVS | (SEQ ID NO.: 42, JM#40) |
| Id-LRWSRKLPCVS | (SEQ ID NO.: 43, JM#41) |
| Id-LRWSRKMPCVS | (SEQ ID NO.: 44, JM#42) |
| Md-LRWSRKLPCVS | (SEQ ID NO.: 45, JM#43) |
| Md-LRWSRKMPCVS | (SEQ ID NO.: 46, JM#44) |
| IVRWSKKVP-NH2 | (SEQ ID NO.: 47, JM#106) |
| IVRWSKK-NH2 | (SEQ ID NO.: 48, JM#110) |
| ILRWSRKLP-NH2 | (SEQ ID NO.: 49, JM#114) |
| ILRWSRK-NH2 | (SEQ ID NO.: 50, JM#118) |
| ILRWSRK(Glu-Pal)LPCVS | (SEQ ID NO.: 54, JM#143) |
| ILRWSRKLPCK(Glu-Pal)S | (SEQ ID NO.: 56, JM#145) |
| IYRWSRKMPCLS | (SEQ ID NO.: 57, JM#146) |
| ILRWSRK(Glu-Pal)MPCLS | (SEQ ID NO.: 58, JM#148) |
| IVRWSKKVPSVS | (SEQ ID NO.: 60, JM#151) |
| IVRWSK(Pal)K-NH2 | (SEQ ID NO.: 61, JM#164) |
| IVRWSKK(Pal)-NH2 | (SEQ ID NO.: 62, JM#165) |
| IVRWSK(Pal)KVPCVS | (SEQ ID NO.: 65, JM#168) |
| d-ILRWSRK-NH2 | (SEQ ID NO.: 70, JM#173) |
| d-ILRWSRKLP-NH2 | (SEQ ID NO.: 71, JM#174) |
| d-ILRWSRK(Pal)LP-NH2 | (SEQ ID NO.: 72, JM#175) |
| d-LMRWSRK(Pal)MPCVS | (SEQ ID NO.: 73, JM#176) |
| Md-LRWSRK(Pal)LPCVS | (SEQ ID NO.: 74, JM#177) |
| Md-LRWSRK(Pal)LP-NH2 | (SEQ ID NO.: 76, JM#179) |
| Md-LRWSRK(Pal)-NH2 | (SEQ ID NO.: 78, JM#181) |
| ILRWSRK(Ste)LPCVS | (SEQ ID NO.: 79, JM#182) |
| ILRWSRK(Chl)LPCVS | (SEQ ID NO.: 81, JM#184) |
| Ac-ILRWSRKLPCVS | (SEQ ID NO.: 82, JM#185) |
| d-Ac-ILRWSRKLPCVS | (SEQ ID NO.: 83, JM#186) |
| Ac-MLRWSRKLPCVS | (SEQ ID NO.: 84, JM#187) |
| d-Ac-MLRWSRKLPCVS | (SEQ ID NO.: 85, JM#188) |
| VLRWSRKLPCVS | (SEQ ID NO.: 86, JM#189) |
| d-VLRWSRKLPCVS | (SEQ ID NO.: 87, JM#190) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197) |
| ILRWSRK(Glu-Ste)LPCVS | (SEQ ID NO.: 95, JM#198) |
| ILRWSRK(Glu-Myr)LPCVS | (SEQ ID NO.: 99, JM#204) |

- Group 6 consists of

| | |
|---|---|
| d-LMRWSRKK(Glu-Pal)-NH2 | (SEQ ID NO.: 92, JM#195) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197) |
| ILRWSRK(Glu-Ste)LPCVS | CVS (SEQ ID NO.: 95, JM#198) |

- Group 7 consists of

| | |
|---|---|
| Md-LRWSRKLPCVS | (SEQ ID NO.: 45, JM#43) |
| Md-LRWSRKMPCVS | (SEQ ID NO.: 46, JM#44) |
| ILRWSRK(Glu-Pal)LPCVS | (SEQ ID NO.: 54, JM#143) |
| ILRWSRK(Pal)LPCVS | (SEQ ID NO.: 55, JM#144) |
| ILRWSRKLPCK(Glu-Pal)S | (SEQ ID NO.: 56, JM#145) |
| ILRWSRK(Pal)MPCLS | (SEQ ID NO.: 59, JM#149) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| d-ILRWSRK-NH2 | (SEQ ID NO.: 70, JM#173) |
| d-ILRWSRKLP-NH2 | (SEQ ID NO.: 71, JM#174) |

- Group 8 consists of

| | |
|---|---|
| d-LLRWSRKMPCVS | (SEQ ID NO.: 31, JM#29) |
| ILRWSRK(Pal)L-NH2 | (SEQ ID NO.: 69, JM#172) |
| ILRWSRK(Glu-Pal)-NH2 | (SEQ ID NO.: 89, JM#192) |

- Group 9 consists of

| | |
|---|---|
| Ac-SLRWSRKMPCVS | (SEQ ID NO.: 27, JM#25) |
| d-MLRWSRKMPCVS | (SEQ ID NO.: 30, JM#28) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-LLRWSRK(Glu-Pal)MPCVS | (SEQ ID NO.: 52, JM#140) |
| d-LMRWSRK(Pal)MP-NH2 | (SEQ ID NO.: 75, JM#178) |

- Group 10 consists of

| | |
|---|---|
| ILRWSRK(Pal)LPCVS | (SEQ ID NO.: 55, JM#144) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRK(Pal)L-NH2 | (SEQ ID NO.: 69, JM#172) |
| d-LMRWSRK(Pal)-NH2 | (SEQ ID NO.: 77, JM#180) |
| ILRWSRKK(Glu-Pal)-NH2 | (SEQ ID NO.: 88, JM#191) |
| ILRWSRK(Glu-Pal)-NH2 | (SEQ ID NO.: 89, JM#192) |
| ILRWSRKLK(Glu-Pal)-NH2 | (SEQ ID NO.: 90, JM#193) |
| d-LMRWSRK(Glu-Pal)-NH2 | (SEQ ID NO.: 91, JM#194) |

wherein d- in front of an amino acid indicates a D-Amino acid, Pal indicates a palmitic acid on the preceding amino acid, Glu-Pal indicates a palmitic acid on the preceding amino acid with a glutamate linker, Dec indicates decanoic acid on the preceding amino acid, Glu-Dec indicates a decanoic acid on the preceding amino acid with a glutamate linker, Myr indicates myristic acid on the preceding amino acid, Glu-Myr indicates a myristic acid on the preceding amino acid with a glutamate linker, Ole indicates oleic acid on the preceding amino acid, Ste indicates stearic acid on the preceding amino acid, Glu-Ste indicates a stearic acid on the preceding amino acid with a glutamate linker, Chl indicates Cholesterol, and Ac indicates a substitution of an amino group by an acetyl group.

A second aspect of the invention is directed to a peptide consisting of one of the following amino acid sequences, selected from any of the groups 1 to 10, wherein
- Group 1 consists of

| | |
|---|---|
| ILRWSRKMPCVS | (SEQ ID NO.: 15, JM#13) |
| IMRWSRKMPCVS | (SEQ ID NO.: 19, JM#17) |
| ILRWSRKMPCLS | (SEQ ID NO.: 20, JM#18) |
| ILRWSRKMPCMS | (SEQ ID NO.: 22, JM#20) |
| ILRWSRKLPCVS | (SEQ ID NO.: 23, JM#21) |
| ILRWSRKFPCVS | (SEQ ID NO.: 24, JM#22) |
| ILRWSRK(Pal)LPCVS | (SEQ ID NO.: 55, JM#144) |
| ILRWSRK(Glu-Pal)-NH2 | (SEQ ID NO.: 89, JM#192) |
| d-LMRWSRK(Glu-Pal)-NH2 | (SEQ ID NO.: 91, JM#194) |

- Group 2 consists of

| | |
|---|---|
| ILRWSRKMPCFS | (SEQ ID NO.: 21, JM#19) |
| ILRWSRK(Pal)L-NH2 | (SEQ ID NO.: 69, JM#172) |
| d-LMRWSRK(Pal)MP-NH2 | (SEQ ID NO.: 75, JM#178) |
| d-LMRWSRK(Pal)-NH2 | (SEQ ID NO.: 77, JM#180) |
| ILRWSRK(Ole)LPCVS | (SEQ ID NO.: 80, JM#183) |

- Group 3 consists of

| | |
|---|---|
| d-LLRWSRK(Pal)MPCVS | (SEQ ID NO.: 53, JM#141) |
| IVRWSK(Pal)KVP-NH2 | (SEQ ID NO.: 63, JM#166) |
| IVRWSKK(Pal)VP-NH2 | (SEQ ID NO.: 64, JM#167) |
| IVRWSKK(Pal)VPCVS | (SEQ ID NO.: 66, JM#169) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRKK(Glu-Pal)-NH2 | (SEQ ID NO.: 88, JM#191) |
| ILRWSRKLK(Glu-Pal)-NH2 | (SEQ ID NO.: 90, JM#193) |
| ILRWSRKK(Glu-Ste)LPCVS | (SEQ ID NO.: 96, JM#199) |
| ILRWSRK(Glu-Dec)LPCVS | (SEQ ID NO.: 98, JM#203) |
| ILRWSRK(Glu-Ste)LPCVS | (SEQ ID NO.: 100, JM#205) |

- Group 4 consists of

| | |
|---|---|
| ILRWSRKVPCVS | (SEQ ID NO.: 10, JM#8) |
| IFRWSRKVPCVS | (SEQ ID NO.: 12, JM#10) |
| MLRWSRKMPCVS | (SEQ ID NO.: 29, JM#27) |
| MMRWSRKMPCVS | (SEQ ID NO.: 36, JM#34) |
| MLRWSRKLPCVS | (SEQ ID NO.: 41, JM#39) |
| ILRWSRKLPSVS | (SEQ ID NO.: 51, JM#122) |
| d-LLRWSRK(Glu-Pal)MPCVS | (SEQ ID NO.: 52, JM#140) |
| ILRWSRK(Pal)MPCLS | (SEQ ID NO.: 59, JM#149) |
| d-LMRWSRKK(Glu-Pal)-NH2 | (SEQ ID NO.: 92, JM#195) |
| ILRWSRK-AcLPCVS | (SEQ ID NO.: 97, JM#200) |

- Group 5 consisting of

| | |
|---|---|
| ILRWSKKVPCVS | (SEQ ID NO.: 3, JM#1) |
| IFRWSKKVPCVS | (SEQ ID NO.: 4, JM#2) |
| IVRWSRKVPCVS | (SEQ ID NO.: 5, JM#3) |
| IVRWSHKVPCVS | (SEQ ID NO.: 6, JM#4) |
| IVRWSKKLPCVS | (SEQ ID NO.: 7, JM#5) |
| IVRWSKKIPCVS | (SEQ ID NO.: 8, JM#6) |
| IVRWSKKFPCVS | (SEQ ID NO.: 9, JM#7) |
| ILRWSHKVPCVS | (SEQ ID NO.: 11, JM#9) |
| IFRWSHKVPCVS | (SEQ ID NO.: 13, JM#11) |
| IVRWSKKMPCVS | (SEQ ID NO.: 14, JM#12) |
| IVRWSKKVPCd-VS | (SEQ ID NO.: 16, JM#14) |
| ILRWSRKVPCd-VS | (SEQ ID NO.: 17, JM#15) |
| IIRWSRKMPCVS | (SEQ ID NO.: 18, JM#16) |
| ILRWSRKVPSVS | (SEQ ID NO.: 25, JM#23) |
| ILRWSRKMPSVS | (SEQ ID NO.: 26, JM#24) |
| Ac-SLRWSRKMPCVS | (SEQ ID NO.: 27, JM#25) |
| d-Ac-SLRWSRKMPCVS | (SEQ ID NO.: 28, JM#26) |
| d-MLRWSRKMPCVS | (SEQ ID NO.: 30, JM#28) |
| d-LLRWSRKMPCVS | (SEQ ID NO.: 31, JM#29) |
| d-FLRWSRKMPCVS | (SEQ ID NO.: 32, JM#30) |
| GLRWSRKMPCVS | (SEQ ID NO.: 33, JM#31) |
| Ac-SMRWSRKMPCVS | (SEQ ID NO.: 34, JM#32) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-FMRWSRKMPCVS | (SEQ ID NO.: 39, JM#37) |
| d-GMRWSRKMPCVS | (SEQ ID NO.: 40, JM#38) |
| d-MLRWSRKLPCVS | (SEQ ID NO.: 42, JM#40) |
| Id-LRWSRKLPCVS | (SEQ ID NO.: 43, JM#41) |
| Id-LRWSRKMPCVS | (SEQ ID NO.: 44, JM#42) |
| Md-LRWSRKLPCVS | (SEQ ID NO.: 45, JM#43) |
| Md-LRWSRKMPCVS | (SEQ ID NO.: 46, JM#44) |
| IVRWSKKVP-NH2 | (SEQ ID NO.: 47, JM#106) |
| IVRWSKK-NH2 | (SEQ ID NO.: 48, JM#110) |
| ILRWSRKLP-NH2 | (SEQ ID NO.: 49, JM#114) |
| ILRWSRK-NH2 | (SEQ ID NO.: 50, JM#118) |
| ILRWSRK(Glu-Pal)LPCVS | (SEQ ID NO.: 54, JM#143) |
| ILRWSRKLPCK(Glu-Pal)S | (SEQ ID NO.: 56, JM#145) |
| IYRWSRKMPCLS | (SEQ ID NO.: 57, JM#146) |
| ILRWSRK(Glu-Pal)MPCLS | (SEQ ID NO.: 58, JM#148) |
| IVRWSKKVPSVS | (SEQ ID NO.: 60, JM#151) |
| IVRWSK(Pal)K-NH2 | (SEQ ID NO.: 61, JM#164) |
| IVRWSKK(Pal)-NH2 | (SEQ ID NO.: 62, JM#165) |
| IVRWSK(Pal)KVPCVS | (SEQ ID NO.: 65, JM#168) |
| d-ILRWSRK-NH2 | (SEQ ID NO.: 70, JM#173) |
| d-ILRWSRKLP-NH2 | (SEQ ID NO.: 71, JM#174) |
| d-ILRWSRK(Pal)LP-NH2 | (SEQ ID NO.: 72, JM#175) |
| d-LMRWSRK(Pal)MPCVS | (SEQ ID NO.: 73, JM#176) |
| Md-LRWSRK(Pal)LPCVS | (SEQ ID NO.: 74, JM#177) |
| Md-LRWSRK(Pal)LP-NH2 | (SEQ ID NO.: 76, JM#179) |
| Md-LRWSRK(Pal)-NH2 | (SEQ ID NO.: 78, JM#181) |
| ILRWSRK(Ste)LPCVS | (SEQ ID NO.: 79, JM#182) |
| ILRWSRK(Chl)LPCVS | (SEQ ID NO.: 81, JM#184) |
| Ac-ILRWSRKLPCVS | (SEQ ID NO.: 82, JM#185) |
| d-Ac-ILRWSRKLPCVS | (SEQ ID NO.: 83, JM#186) |
| Ac-MLRWSRKLPCVS | (SEQ ID NO.: 84, JM#187) |
| d-Ac-MLRWSRKLPCVS | (SEQ ID NO.: 85, JM#188) |
| VLRWSRKLPCVS | (SEQ ID NO.: 86, JM#189) |
| d-VLRWSRKLPCVS | (SEQ ID NO.: 87, JM#190) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197) |
| ILRWSRK(Glu-Ste)LPCVS | (SEQ ID NO.: 95, JM#198) |
| ILRWSRK(Glu-Myr)LPCVS | (SEQ ID NO.: 99, JM#204) |

- Group 6 consists of

| | |
|---|---|
| d-LMRWSRKK(Glu-Pal)-NH2 | (SEQ ID NO.: 92, JM#195) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197) |
| ILRWSRK(Glu-Ste)LPCVS | (SEQ ID NO.: 95, JM#198) |

- Group 7 consists of

| | |
|---|---|
| Md-LRWSRKLPCVS | (SEQ ID NO.: 45, JM#43) |
| Md-LRWSRKMPCVS | (SEQ ID NO.: 46, JM#44) |
| ILRWSRK(Glu-Pal)LPCVS | (SEQ ID NO.: 54, JM#143) |
| ILRWSRK(Pal)LPCVS | (SEQ ID NO.: 55, JM#144) |
| ILRWSRKLPCK(Glu-Pal)S | (SEQ ID NO.: 56, JM#145) |
| ILRWSRK(Pal)MPCLS | (SEQ ID NO.: 59, JM#149) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| d-ILRWSRK-NH2 | (SEQ ID NO.: 70, JM#173) |
| d-ILRWSRKLP-NH2 | (SEQ ID NO.: 71, JM#174) |

- Group 8 consists of

| | |
|---|---|
| d-LLRWSRKMPCVS | (SEQ ID NO.: 31, JM#29) |
| ILRWSRK(Pal)L-NH2 | (SEQ ID NO.: 69, JM#172) |
| ILRWSRK(Glu-Pal)-NH2 | (SEQ ID NO.: 89, JM#192) |

- Group 9 consists of

| | |
|---|---|
| Ac-SLRWSRKMPCVS | (SEQ ID NO.: 27, JM#25) |
| d-MLRWSRKMPCVS | (SEQ ID NO.: 30, JM#28) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-LLRWSRK(Glu-Pal)MPCVS | (SEQ ID NO.: 52, JM#140) |
| d-LMRWSRK(Pal)MP-NH2 | (SEQ ID NO.: 75, JM#178) |

- Group 10 consists of

| | |
|---|---|
| ILRWSRK(Pal)LPCVS | (SEQ ID NO.: 55, JM#144) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRK(Pal)L-NH2 | (SEQ ID NO.: 69, JM#172) |
| d-LMRWSRK(Pal)-NH2 | (SEQ ID NO.: 77, JM#180) |
| ILRWSRKK(Glu-Pal)-NH2 | (SEQ ID NO.: 88, JM#191) |
| ILRWSRK(Glu-Pal)-NH2 | (SEQ ID NO.: 89, JM#192) |
| ILRWSRKLK(Glu-Pal)-NH2 | (SEQ ID NO.: 90, JM#193) |
| d-LMRWSRK(Glu-Pal)-NH2 | (SEQ ID NO.: 91, JM#194) |

wherein d- in front of an amino acid indicates a D-Amino acid, Pal indicates a palmitic acid on the preceding amino acid, Glu-Pal indicates a palmitic acid on the preceding amino acid with a glutamate linker, Dec indicates decanoic acid on the preceding amino acid, Glu-Dec indicates a decanoic acid on the preceding amino acid with a glutamate linker, Myr indicates myristic acid on the preceding amino acid, Glu-Myr indicates a myristic acid on the preceding amino acid with a glutamate linker, Ole indicates oleic acid on the preceding amino acid, Ste indicates stearic acid on the preceding amino acid, Glu-Ste indicates a stearic acid on the preceding amino acid with a glutamate linker, Chl indicates Cholesterol, Ac indicates a substitution of an amino group by an acetyl group, and wherein the peptide is C-terminally conjugated to a complexing agent.

A third aspect of the invention is directed to a peptide consisting of one of the following amino acid sequences, selected from any of the groups 1 to 10, wherein
- Group 1 consists of

| | |
|---|---|
| ILRWSRKMPCVS | (SEQ ID NO.: 15, JM#13) |
| IMRWSRKMPCVS | (SEQ ID NO.: 19, JM#17) |
| ILRWSRKMPCLS | (SEQ ID NO.: 20, JM#18) |
| ILRWSRKMPCMS | (SEQ ID NO.: 22, JM#20) |
| ILRWSRKLPCVS | (SEQ ID NO.: 23, JM#21) |
| ILRWSRKFPCVS | (SEQ ID NO.: 24, JM#22) |
| ILRWSRK(Pal)LPCVS | (SEQ ID NO.: 55, JM#144) |
| ILRWSRK(Glu-Pal)-NH2 | (SEQ ID NO.: 89, JM#192) |
| d-LMRWSRK(Glu-Pal)-NH2 | (SEQ ID NO.: 91, JM#194) |

- Group 2 consists of

| | |
|---|---|
| ILRWSRKMPCFS | (SEQ ID NO.: 21, JM#19) |
| ILRWSRK(Pal)L-NH2 | (SEQ ID NO.: 69, JM#172) |
| d-LMRWSRK(Pal)MP-NH2 | (SEQ ID NO.: 75, JM#178) |
| d-LMRWSRK(Pal)-NH2 | (SEQ ID NO.: 77, JM#180) |
| ILRWSRK(Ole)LPCVS | (SEQ ID NO.: 80, JM#183) |

- Group 3 consists of

| | |
|---|---|
| d-LLRWSRK(Pal)MPCVS | (SEQ ID NO.: 53, JM#141) |
| IVRWSK(Pal)KVP-NH2 | (SEQ ID NO.: 63, JM#166) |
| IVRWSKK(Pal)VP-NH2 | (SEQ ID NO.: 64, JM#167) |
| IVRWSKK(Pal)VPCVS | (SEQ ID NO.: 66, JM#169) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRKK(Glu-Pal)-NH2 | (SEQ ID NO.: 88, JM#191) |
| ILRWSRKLK(Glu-Pal)-NH2 | (SEQ ID NO.: 90, JM#193) |
| ILRWSRKK(Glu-Ste)LPCVS | (SEQ ID NO.: 96, JM#199) |
| ILRWSRK(Glu-Dec)LPCVS | (SEQ ID NO.: 98, JM#203) |
| ILRWSRK(Glu-Ste)LPCVS | (SEQ ID NO.: 100, JM#205) |

- Group 4 consists of

| | |
|---|---|
| ILRWSRKVPCVS | (SEQ ID NO.: 10, JM#8) |
| IFRWSRKVPCVS | (SEQ ID NO.: 12, JM#10) |
| MLRWSRKMPCVS | (SEQ ID NO.: 29, JM#27) |
| MMRWSRKMPCVS | (SEQ ID NO.: 36, JM#34) |
| MLRWSRKLPCVS | (SEQ ID NO.: 41, JM#39) |
| ILRWSRKLPSVS | (SEQ ID NO.: 51, JM#122) |
| d-LLRWSRK(Glu-Pal)MPCVS | (SEQ ID NO.: 52, JM#140) |
| ILRWSRK(Pal)MPCLS | (SEQ ID NO.: 59, JM#149) |
| d-LMRWSRKK(Glu-Pal)-NH2 | (SEQ ID NO.: 92, JM#195) |
| ILRWSRK-AcLPCVS | (SEQ ID NO.: 97, JM#200) |

Group 5 consisting of

| | |
|---|---|
| ILRWSKKVPCVS | (SEQ ID NO.: 3, JM#1) |
| IFRWSKKVPCVS | (SEQ ID NO.: 4, JM#2) |
| IVRWSRKVPCVS | (SEQ ID NO.: 5, JM#3) |
| IVRWSHKVPCVS | (SEQ ID NO.: 6, JM#4) |
| IVRWSKKLPCVS | (SEQ ID NO.: 7, JM#5) |
| IVRWSKKIPCVS | (SEQ ID NO.: 8, JM#6) |
| IVRWSKKFPCVS | (SEQ ID NO.: 9, JM#7) |
| ILRWSHKVPCVS | (SEQ ID NO.: 11, JM#9) |
| IFRWSHKVPCVS | (SEQ ID NO.: 13, JM#11) |
| IVRWSKKMPCVS | (SEQ ID NO.: 14, JM#12) |
| IVRWSKKVPCd-VS | (SEQ ID NO.: 16, JM#14) |
| ILRWSRKVPCd-VS | (SEQ ID NO.: 17, JM#15) |
| IIRWSRKMPCVS | (SEQ ID NO.: 18, JM#16) |
| ILRWSRKVPSVS | (SEQ ID NO.: 25, JM#23) |
| ILRWSRKMPSVS | (SEQ ID NO.: 26, JM#24) |
| Ac-SLRWSRKMPCVS | (SEQ ID NO.: 27, JM#25) |
| d-Ac-SLRWSRKMPCVS | (SEQ ID NO.: 28, JM#26) |
| d-MLRWSRKMPCVS | (SEQ ID NO.: 30, JM#28) |
| d-LLRWSRKMPCVS | (SEQ ID NO.: 31, JM#29) |
| d-FLRWSRKMPCVS | (SEQ ID NO.: 32, JM#30) |
| GLRWSRKMPCVS | (SEQ ID NO.: 33, JM#31) |
| Ac-SMRWSRKMPCVS | (SEQ ID NO.: 34, JM#32) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-FMRWSRKMPCVS | (SEQ ID NO.: 39, JM#37) |
| d-GMRWSRKMPCVS | (SEQ ID NO.: 40, JM#38) |
| d-MLRWSRKLPCVS | (SEQ ID NO.: 42, JM#40) |
| Id-LRWSRKLPCVS | (SEQ ID NO.: 43, JM#41) |
| Id-LRWSRKMPCVS | (SEQ ID NO.: 44, JM#42) |
| Md-LRWSRKLPCVS | (SEQ ID NO.: 45, JM#43) |
| Md-LRWSRKMPCVS | (SEQ ID NO.: 46, JM#44) |
| IVRWSKKVP-NH2 | (SEQ ID NO.: 47, JM#106) |
| IVRWSKK-NH2 | (SEQ ID NO.: 48, JM#110) |
| ILRWSRKLP-NH2 | (SEQ ID NO.: 49, JM#114) |
| ILRWSRK-NH2 | (SEQ ID NO.: 50, JM#118) |
| ILRWSRK(Glu-Pal)LPCVS | (SEQ ID NO.: 54, JM#143) |
| ILRWSRKLPCK(Glu-Pal)S | (SEQ ID NO.: 56, JM#145) |
| IYRWSRKMPCLS | (SEQ ID NO.: 57, JM#146) |
| ILRWSRK(Glu-Pal)MPCLS | (SEQ ID NO.: 58, JM#148) |
| IVRWSKKVPSVS | (SEQ ID NO.: 60, JM#151) |
| IVRWSK(Pal)K-NH2 | (SEQ ID NO.: 61, JM#164) |
| IVRWSKK(Pal)-NH2 | (SEQ ID NO.: 62, JM#165) |
| IVRWSK(Pal)KVPCVS | (SEQ ID NO.: 65, JM#168) |
| d-ILRWSRK-NH2 | (SEQ ID NO.: 70, JM#173) |
| d-ILRWSRKLP-NH2 | (SEQ ID NO.: 71, JM#174) |
| d-ILRWSRK(Pal)LP-NH2 | (SEQ ID NO.: 72, JM#175) |
| d-LMRWSRK(Pal)MPCVS | (SEQ ID NO.: 73, JM#176) |
| Md-LRWSRK(Pal)LPCVS | (SEQ ID NO.: 74, JM#177) |
| Md-LRWSRK(Pal)LP-NH2 | (SEQ ID NO.: 76, JM#179) |
| Md-LRWSRK(Pal)-NH2 | (SEQ ID NO.: 78, JM#181) |
| ILRWSRK(Ste)LPCVS | (SEQ ID NO.: 79, JM#182) |
| ILRWSRK(Chl)LPCVS | (SEQ ID NO.: 81, JM#184) |
| Ac-ILRWSRKLPCVS | (SEQ ID NO.: 82, JM#185) |
| d-Ac-ILRWSRKLPCVS | (SEQ ID NO.: 83, JM#186) |
| Ac-MLRWSRKLPCVS | (SEQ ID NO.: 84, JM#187) |
| d-Ac-MLRWSRKLPCVS | (SEQ ID NO.: 85, JM#188) |
| VLRWSRKLPCVS | (SEQ ID NO.: 86, JM#189) |
| d-VLRWSRKLPCVS | (SEQ ID NO.: 87, JM#190) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197) |
| ILRWSRK(Glu-Ste)LPCVS | (SEQ ID NO.: 95, JM#198) |
| ILRWSRK(Glu-Myr)LPCVS | (SEQ ID NO.: 99, JM#204) |

- Group 6 consists of

| | |
|---|---|
| d-LMRWSRKK(Glu-Pal)-NH2 | (SEQ ID NO.: 92, JM#195) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197) |
| ILRWSRK(Glu-Ste)LPCVS | (SEQ ID NO.: 95, JM#198) |

- Group 7 consists of

| | |
|---|---|
| Md-LRWSRKLPCVS | (SEQ ID NO.: 45, JM#43) |
| Md-LRWSRKMPCVS | (SEQ ID NO.: 46, JM#44) |
| ILRWSRK(Glu-Pal)LPCVS | (SEQ ID NO.: 54, JM#143) |
| ILRWSRK(Pal)LPCVS | (SEQ ID NO.: 55, JM#144) |
| ILRWSRKLPCK(Glu-Pal)S | (SEQ ID NO.: 56, JM#145) |
| ILRWSRK(Pal)MPCLS | (SEQ ID NO.: 59, JM#149) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| d-ILRWSRK-NH2 | (SEQ ID NO.: 70, JM#173) |
| d-ILRWSRKLP-NH2 | (SEQ ID NO.: 71, JM#174) |

- Group 8 consists of

| | |
|---|---|
| d-LLRWSRKMPCVS | (SEQ ID NO.: 31, JM#29) |
| ILRWSRK(Pal)L-NH2 | (SEQ ID NO.: 69, JM#172) |
| ILRWSRK(Glu-Pal)-NH2 | (SEQ ID NO.: 89, JM#192) |

- Group 9 consists of

| | |
|---|---|
| Ac-SLRWSRKMPCVS | (SEQ ID NO.: 27, JM#25) |
| d-MLRWSRKMPCVS | (SEQ ID NO.: 30, JM#28) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-LLRWSRK(Glu-Pal)MPCVS | (SEQ ID NO.: 52, JM#140) |
| d-LMRWSRK(Pal)MP-NH2 | (SEQ ID NO.: 75, JM#178) |

- Group 10 consists of

| | |
|---|---|
| ILRWSRK(Pal)LPCVS | (SEQ ID NO.: 55, JM#144) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRK(Pal)L-NH2 | (SEQ ID NO.: 69, JM#172) |
| d-LMRWSRK(Pal)-NH2 | (SEQ ID NO.: 77, JM#180) |
| ILRWSRKK(Glu-Pal)-NH2 | (SEQ ID NO.: 88, JM#191) |
| ILRWSRK(Glu-Pal)-NH2 | (SEQ ID NO.: 89, JM#192) |
| ILRWSRKLK(Glu-Pal)-NH2 | (SEQ ID NO.: 90, JM#193) |
| d-LMRWSRK(Glu-Pal)-NH2 | (SEQ ID NO.: 91, JM#194) |

wherein d- in front of an amino acid indicates a D-Amino acid, Pal indicates a palmitic acid on the preceding amino acid, Glu-Pal indicates a palmitic acid on the preceding amino acid with a glutamate linker, Dec indicates decanoic acid on the preceding amino acid, Glu-Dec indicates a decanoic acid on the preceding amino acid with a glutamate linker, Myr indicates myristic acid on the preceding amino acid, Glu-Myr indicates a myristic acid on the preceding amino acid with a glutamate linker, Ole indicates oleic acid on the preceding amino acid, Ste indicates stearic acid on the preceding amino acid, Glu-Ste indicates a stearic acid on the preceding amino acid with a glutamate linker, Chl indicates Cholesterol, and Ac indicates a substitution of an amino group by an acetyl group, and wherein the peptide is coupled to a polymer.

A fourth aspect of the invention is related to a peptide consisting of two identical monomeric peptides according to any of the preceding claims, wherein the monomeric peptides are linked to each other via a cysteine bridge which is formed between the monomeric peptides to form a dimeric peptide.

A fifth aspect of the invention is related to the inventive peptide or the inventive pharmaceutical composition for use in medicine.

A sixth aspect of the invention is related to a pharmaceutical composition comprising the inventive peptide together with at least one pharmaceutically acceptable carrier, mesoporous nanoparticles, cryoprotectant, lyoprotectant, excipient and/or diluent.

A seventh aspect of the invention is related to the use of the peptide or the pharmaceutical composition according to any of the preceding claims for the preparation of a formulation for oral administration, inhalation, intravenous administration, topical administration, intranasal, intraperitoneal administration, subcutaneous administration and/or any other injectable form.

An eighth aspect of the invention is related to the inventive peptide or the inventive pharmaceutical composition for use in the treatment of disorders of hematopoiesis, in the treatment of wounds, in the treatment of viral diseases, in particular infections with HIV-1, HIV-2, Cytomegalovirus, Herpes simplex virus (type 1 and 2), Varicella zoster virus, Hepatitis A and Hepatitis B virus, Influenza virus, Polio virus, Rhino virus, Rubella virus, Measles virus, Rabies virus, Rous sarcoma virus, Epstein-Barr Virus; in the treatment of infections caused by bacteria and fungi, in particular Pseudomonas, Candida, S. aureus, in the treatment of infectious processes, abnormal infectious processes, in the treatment of inflammation, in particular of periodontal disease, arthritis and inflammatory bowel disease, as well as dermatitis and asthma; in the treatment of growth disorders, in the treatment of neuronal diseases, disorders of the blood clotting cascade and hematopoiesis, vascular diseases, diseases of the immune system, for improving wound and bone healing, for use in the treatment of neurological diseases, in particular stroke, Parkinson's disease, Alzheimer's disease, multiple sclerosis, in the treatment of the Warts, Hypogammaglobulinemia, Immunodeficiency, and Myelokathexis syndrome (WHIM-syndrome) and rheumatoide arthritis; in the treatment of cancers, in particular cancers showing the CRCX receptor such as cancer of the liver, pancreas, prostate, breast cancer or other solid tumors, in the treatment of lack of mobilization, proliferation and migration of stem cells, T-cell activation as well as support of immunoblasts, such as CTL/PD-1, in the treatment of antifibrosis; in the treatment or prevention of scars; in the treatment of cardiologic disorders, and in the treatment of metabolic disorders, in particular diabetes.

A ninth aspect of the invention is related to a method of prophylaxis and/or treatment of cancer, viral diseases, metabolic disorders, neurologic disorders, diseases of the immune system, and disorders of the blood clotting cascade and hematopoiesis in a mammal, including a human.

An tenth aspect of the invention is related to a method for manufacturing the inventive peptide by solid phase synthesis.

### Brief description of the figures

Figure 1 shows two diagrams of X4-HIV-1 assays, wherein infections rates of cells are depending on concentrations of different peptide derivatives.
Figure 2 shows two diagrams of antibody competition assays, wherein the percentage of bound antibody is dependent on concentrations of truncated palmitoylated variants of peptide JM #21 (SEQ ID NO.: 23) (A) and of truncated palmitoylated variants of peptide JM #21 (SEQ ID NO.: 23) which are terminally modified (B).
Figure 3 shows a diagram of inhibition of CXCL12-induced Akt and Erk signaling, wherein the percentage of phosphorylated Akt and Erk is depending on concentrations of different peptide derivatives.
Figure 4 shows diagrams of stability of different peptides in whole human plasma.
Figure 5 shows a diagram of antibody competition assays, wherein the percentage of bound antibody is depending on concentrations of DOTA-coupled peptides.
Figure 6 shows two diagrams of X4-HIV-1 assays, wherein infections rates of cells are depending on concentrations of different peptides which are coupled to Polyethylene glycol (A) or poly(vinyl alcohol) and poly(vinyl pyrrilidone) (B).
Figure 7 shows two diagrams of antibody competition assays, wherein the percentage of bound antibody is dependent on concentrations of truncated palmitoylated variants of peptides which are coupled to Polyethylene glycol (A) or poly(vinyl alcohol) and poly(vinyl pyrrilidone) (B).
Figure 8 shows two diagrams of assays testing the activity of DSPE-coupled peptides, wherein (A) shows a diagram of X4-HIV-1 assays, and (B) shows a diagram of antibody competition assays.
Figure 9 shows diagrams illustrating the inhibition of CXCL12 induced Ca²⁺-signalling by A) JM21 (SEQ ID NO.: 23) and peptide variants thereof and B) WSC02 (SEQ ID NO.: 2) and peptide variants thereof.
Figure 10 shows diagrams illustrating the inhibition of CXCL12 induced T cell migration by A) peptide according to the invention JM21 (SEQ ID NO.: 23) compared to peptides of the state of the art EPIX4 (SEQ ID NO.: 1) and WSC02 (SEQ ID NO.: 2) and B) short peptides variants of JM 21 (SEQ ID NO.: 23) and WSC02 (SEQ ID NO.: 2) and C) fatty acid coupled peptide variants of JM21 (SEQ ID NO.: 23) and WSC02 (SEQ ID NO.: 2).
Figure 11 shows illustrations of the computational models developed for the design of novel peptide derivatives: A) peptide - protein model in explicit water and membrane environment, B) investigation of tentative binding sites, C) analysis of the energy contributions and D) molecular interactions in the binding site.

### Detailed description of the invention

A first aspect of the present invention is aspect of the invention is directed to a peptide consisting of one of the following amino acid sequences, selected from any of the groups 1 to 10, wherein
- Group 1 consists of

| | |
|---|---|
| ILRWSRKMPCVS | (SEQ ID NO.: 15, JM#13) |
| IMRWSRKMPCVS | (SEQ ID NO.: 19, JM#17) |
| ILRWSRKMPCLS | (SEQ ID NO.: 20, JM#18) |
| ILRWSRKMPCMS | (SEQ ID NO.: 22, JM#20) |
| ILRWSRKLPCVS | (SEQ ID NO.: 23, JM#21) |
| ILRWSRKFPCVS | (SEQ ID NO.: 24, JM#22) |
| ILRWSRK(Pal)LPCVS | (SEQ ID NO.: 55, JM#144) |
| ILRWSRK(Glu-Pal)-NH2 | (SEQ ID NO.: 89, JM#192) |
| d-LMRWSRK(Glu-Pal)-NH2 | (SEQ ID NO.: 91, JM#194) |

- Group 2 consists of

| | |
|---|---|
| ILRWSRKMPCFS | (SEQ ID NO.: 21, JM#19) |
| ILRWSRK(Pal)L-NH2 | (SEQ ID NO.: 69, JM#172) |
| d-LMRWSRK(Pal)MP-NH2 | (SEQ ID NO.: 75, JM#178) |
| d-LMRWSRK(Pal)-NH2 | (SEQ ID NO.: 77, JM#180) |
| ILRWSRK(Ole)LPCVS | (SEQ ID NO.: 80, JM#183) |

- Group 3 consists of

| | |
|---|---|
| d-LLRWSRK(Pal)MPCVS | (SEQ ID NO.: 53, JM#141) |
| IVRWSK(Pal)KVP-NH2 | (SEQ ID NO.: 63, JM#166) |
| IVRWSKK(Pal)VP-NH2 | (SEQ ID NO.: 64, JM#167) |
| IVRWSKK(Pal)VPCVS | (SEQ ID NO.: 66, JM#169) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRKK(Glu-Pal)-NH2 | (SEQ ID NO.: 88, JM#191) |
| ILRWSRKLK(Glu-Pal)-NH2 | (SEQ ID NO.: 90, JM#193) |
| ILRWSRKK(Glu-Ste)LPCVS | (SEQ ID NO.: 96, JM#199) |
| ILRWSRK(Glu-Dec)LPCVS | (SEQ ID NO.: 98, JM#203) |
| ILRWSRK(Glu-Ste)LPCVS | (SEQ ID NO.: 100, JM#205) |

- Group 4 consists of

| | |
|---|---|
| ILRWSRKVPCVS | (SEQ ID NO.: 10, JM#8) |
| IFRWSRKVPCVS | (SEQ ID NO.: 12, JM#10) |
| MLRWSRKMPCVS | (SEQ ID NO.: 29, JM#27) |
| MMRWSRKMPCVS | (SEQ ID NO.: 36, JM#34) |
| MLRWSRKLPCVS | (SEQ ID NO.: 41, JM#39) |
| ILRWSRKLPSVS | (SEQ ID NO.: 51, JM#122) |
| d-LLRWSRK(Glu-Pal)MPCVS | (SEQ ID NO.: 52, JM#140) |
| ILRWSRK(Pal)MPCLS | (SEQ ID NO.: 59, JM#149) |
| d-LMRWSRKK(Glu-Pal)-NH2 | (SEQ ID NO.: 92, JM#195) |
| ILRWSRK-AcLPCVS (SEQ | (SEQ ID NO.: 97, JM#200) |

- Group 5 consisting of

| | |
|---|---|
| ILRWSKKVPCVS | (SEQ ID NO.: 3, JM#1) |
| IFRWSKKVPCVS | (SEQ ID NO.: 4, JM#2) |
| IVRWSRKVPCVS | (SEQ ID NO.: 5, JM#3) |
| IVRWSHKVPCVS | (SEQ ID NO.: 6, JM#4) |
| IVRWSKKLPCVS | (SEQ ID NO.: 7, JM#5) |
| IVRWSKKIPCVS | (SEQ ID NO.: 8, JM#6) |
| IVRWSKKFPCVS | (SEQ ID NO.: 9, JM#7) |
| ILRWSHKVPCVS | (SEQ ID NO.: 11, JM#9) |
| IFRWSHKVPCVS | (SEQ ID NO.: 13, JM#11) |
| IVRWSKKMPCVS | (SEQ ID NO.: 14, JM#12) |
| IVRWSKKVPCd-VS | (SEQ ID NO.: 16, JM#14) |
| ILRWSRKVPCd-VS | (SEQ ID NO.: 17, JM#15) |
| IIRWSRKMPCVS | (SEQ ID NO.: 18, JM#16) |
| ILRWSRKVPSVS | (SEQ ID NO.: 25, JM#23) |
| ILRWSRKMPSVS | (SEQ ID NO.: 26, JM#24) |
| Ac-SLRWSRKMPCVS | (SEQ ID NO.: 27, JM#25) |
| d-Ac-SLRWSRKMPCVS | (SEQ ID NO.: 28, JM#26) |
| d-MLRWSRKMPCVS | (SEQ ID NO.: 30, JM#28) |
| d-LLRWSRKMPCVS | (SEQ ID NO.: 31, JM#29) |
| d-FLRWSRKMPCVS | (SEQ ID NO.: 32, JM#30) |
| GLRWSRKMPCVS | (SEQ ID NO.: 33, JM#31) |
| Ac-SMRWSRKMPCVS | (SEQ ID NO.: 34, JM#32) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-FMRWSRKMPCVS | (SEQ ID NO.: 39, JM#37) |
| d-GMRWSRKMPCVS | (SEQ ID NO.: 40, JM#38) |
| d-MLRWSRKLPCVS | (SEQ ID NO.: 42, JM#40) |
| Id-LRWSRKLPCVS | (SEQ ID NO.: 43, JM#41) |
| Id-LRWSRKMPCVS | (SEQ ID NO.: 44, JM#42) |
| Md-LRWSRKLPCVS | (SEQ ID NO.: 45, JM#43) |
| Md-LRWSRKMPCVS | (SEQ ID NO.: 46, JM#44) |
| IVRWSKKVP-NH2 | (SEQ ID NO.: 47, JM#106) |
| IVRWSKK-NH2 | (SEQ ID NO.: 48, JM#110) |
| ILRWSRKLP-NH2 | (SEQ ID NO.: 49, JM#114) |
| ILRWSRK-NH2 | (SEQ ID NO.: 50, JM#118) |
| ILRWSRK(Glu-Pal)LPCVS | (SEQ ID NO.: 54, JM#143) |
| ILRWSRKLPCK(Glu-Pal)S | (SEQ ID NO.: 56, JM#145) |
| IYRWSRKMPCLS | (SEQ ID NO.: 57, JM#146) |
| ILRWSRK(Glu-Pal)MPCLS | (SEQ ID NO.: 58, JM#148) |
| IVRWSKKVPSVS | (SEQ ID NO.: 60, JM#151) |
| IVRWSK(Pal)K-NH2 | (SEQ ID NO.: 61, JM#164) |
| IVRWSKK(Pal)-NH2 | (SEQ ID NO.: 62, JM#165) |
| IVRWSK(Pal)KVPCVS | (SEQ ID NO.: 65, JM#168) |
| d-ILRWSRK-NH2 | (SEQ ID NO.: 70, JM#173) |
| d-ILRWSRKLP-NH2 | (SEQ ID NO.: 71, JM#174) |
| d-ILRWSRK(Pal)LP-NH2 | (SEQ ID NO.: 72, JM#175) |
| d-LMRWSRK(Pal)MPCVS | (SEQ ID NO.: 73, JM#176) |
| Md-LRWSRK(Pal)LPCVS | (SEQ ID NO.: 74, JM#177) |
| Md-LRWSRK(Pal)LP-NH2 | (SEQ ID NO.: 76, JM#179) |
| Md-LRWSRK(Pal)-NH2 | (SEQ ID NO.: 78, JM#181) |
| I LRWSRK(Ste)LPCVS | (SEQ ID NO.: 79, JM#182) |
| ILRWSRK(Chl)LPCVS | (SEQ ID NO.: 81, JM#184) |
| Ac-ILRWSRKLPCVS | (SEQ ID NO.: 82, JM#185) |
| d-Ac-ILRWSRKLPCVS | (SEQ ID NO.: 83, JM#186) |
| Ac-MLRWSRKLPCVS | (SEQ ID NO.: 84, JM#187) |
| d-Ac-MLRWSRKLPCVS | (SEQ ID NO.: 85, JM#188) |
| VLRWSRKLPCVS | (SEQ ID NO.: 86, JM#189) |
| d-VLRWSRKLPCVS | (SEQ ID NO.: 87, JM#190) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197 |
| ILRWSRK(Glu-Ste)LPCVS | (SEQ ID NO.: 95, JM#198) |
| ILRWSRK(Glu-Myr)LPCVS | (SEQ ID NO.: 99, JM#204) |

- Group 6 consists of

| | |
|---|---|
| d-LMRWSRKK(Glu-Pal)-NH2 | (SEQ ID NO.: 92, JM#195) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197) |
| ILRWSRK(Glu-Ste)LPCVS | (SEQ ID NO.: 95, JM#198) |

- Group 7 consists of

| | |
|---|---|
| Md-LRWSRKLPCVS | (SEQ ID NO.: 45, JM#43) |
| Md-LRWSRKMPCVS | (SEQ ID NO.: 46, JM#44) |
| ILRWSRK(Glu-Pal)LPCVS | (SEQ ID NO.: 54, JM#143) |
| ILRWSRK(Pal)LPCVS | (SEQ ID NO.: 55, JM#144) |
| ILRWSRKLPCK(Glu-Pal)S | (SEQ ID NO.: 56, JM#145) |
| ILRWSRK(Pal)MPCLS | (SEQ ID NO.: 59, JM#149) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| d-ILRWSRK-NH2 | (SEQ ID NO.: 70, JM#173) |
| d-ILRWSRKLP-NH2 | (SEQ ID NO.: 71, JM#174) |

- Group 8 consists of

| | |
|---|---|
| d-LLRWSRKMPCVS | (SEQ ID NO.: 31, JM#29) |
| ILRWSRK(Pal)L-NH2 | (SEQ ID NO.: 69, JM#172) |
| ILRWSRK(Glu-Pal)-NH2 | (SEQ ID NO.: 89, JM#192) |

- Group 9 consists of

| | |
|---|---|
| Ac-SLRWSRKMPCVS | (SEQ ID NO.: 27, JM#25) |
| d-MLRWSRKMPCVS | (SEQ ID NO.: 30, JM#28) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-LLRWSRK(Glu-Pal)MPCVS | (SEQ ID NO.: 52, JM#140) |
| d-LMRWSRK(Pal)MP-NH2 | (SEQ ID NO.: 75, JM#178) |

- Group 10 consists of

| | |
|---|---|
| ILRWSRK(Pal)LPCVS | (SEQ ID NO.: 55, JM#144) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRK(Pal)L-NH2 | (SEQ ID NO.: 69, JM#172) |
| d-LMRWSRK(Pal)-NH2 | (SEQ ID NO.: 77, JM#180) |
| ILRWSRKK(Glu-Pal)-NH2 | (SEQ ID NO.: 88, JM#191) |
| ILRWSRK(Glu-Pal)-NH2 | (SEQ ID NO.: 89, JM#192) |
| ILRWSRKLK(Glu-Pal)-NH2 | (SEQ ID NO.: 90, JM#193) |
| d-LMRWSRK(Glu-Pal)-NH2 | (SEQ ID NO.: 91, JM#194) |

wherein d- in front of an amino acid indicates a D-Amino acid, Pal indicates a palmitic acid on the preceding amino acid, Glu-Pal indicates a palmitic acid on the preceding amino acid with a glutamate linker, Dec indicates decanoic acid on the preceding amino acid, Glu-Dec indicates a decanoic acid on the preceding amino acid with a glutamate linker, Myr indicates myristic acid on the preceding amino acid, Glu-Myr indicates a myristic acid on the preceding amino acid with a glutamate linker, Ole indicates oleic acid on the preceding amino acid, Ste indicates stearic acid on the preceding amino acid, Glu-Ste indicates a stearic acid on the preceding amino acid with a glutamate linker, Chl indicates Cholesterol, and Ac indicates a substitution of an amino group by an acetyl group.

The inventive peptide derivatives are about 100 times more effective in binding to CXCR4 than previously known peptides. Furthermore, the invention provides peptides with a significantly higher plasma stability than peptides of the state of the art. Consequently, the invention provides peptides with a high therapeutic potential compared to drugs of the state of the art, because smaller doses will be sufficient in order to provide the desired effect.

The term derivative means all length fragments of the peptide EPI-X4 (SEQ ID NO.: 1) including truncations at the N and C terminus, the peptide of the invention containing amino acid residue substitutions including D-amino acid residues and modified amino acid residues as well as peptides containing disulfide bonds and extension at the N and C terminus. Here, the terms peptides, peptide derivatives and derivatives are used synonymously. The term peptides does also extent to cyclized peptides, if the inventive peptides can be provided in cyclized form.

Apart from the modifications mentioned above, the peptides are suitable to be coupled to proteins. Proteins to be coupled to the peptides are, for example, antibodies or HSA.

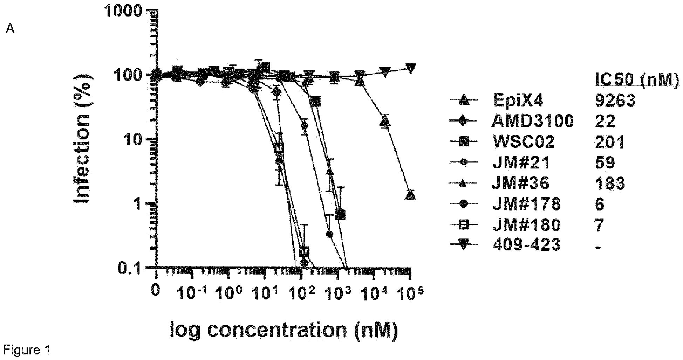

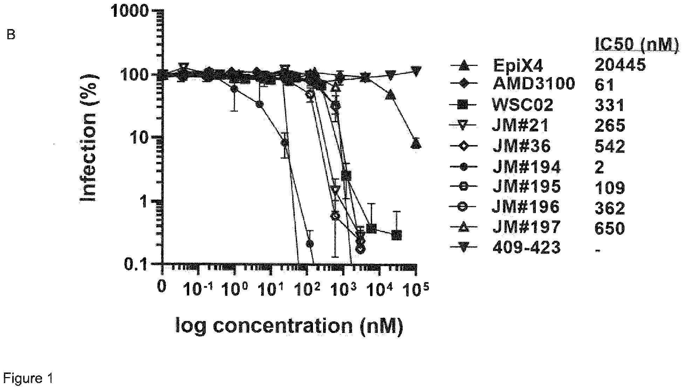

The activity of the peptides was estimated by different assays. First, the activity was tested by an HIV-1 inhibition assay (Fig. 1). The potency of inhibition of CXCR4-tropic X4-HIV-1 indirectly reflects binding affinity of derivatives to CXCR4, as X4-HIV-1 uses CXCR4 together with CD4 for entry into the cell. For cell entry the HIV-1 glycoprotein gp120 needs to bind to the receptors what subsequently leads to cells fusion. Is CXCR4 blocked by a receptor-ligand, HIV-1 entry is blocked and infection is inhibited.

All tested EPI-X4 derivatives dose dependently and specifically inhibited infection of reporter cells by X4-HIV-1. Peptides EPI-X4 (SEQ ID NO.: 1) and WSC02 (SEQ ID NO.: 2) (EP 3 007 717 B1) were used as reference peptides. In Fig. 1, results of two assay are shown by way of diagrams wherein the percentage of infected cells depends on the log concentration of different tested peptides (Figures 1A and 1B). Using mean values estimated over several tests, JM#21 (SEQ ID NO.: 23) lead to an almost 100fold more effective inhibition (IC₅₀ = 98 nM) than EPI-X4 (SEQ ID NO.: 1, IC₅₀ = 8630 nM) and WSC02 (SEQ ID NO.: 2, IC₅₀ = 310 nM). Peptide derivatives coupled to fatty acids, eg. palmitic acid (SEQ ID NO.: 75, JM#178; SEQ ID NO.: 77, JM#180; SEQ ID NO.: 91, JM#194; SEQ ID NO.: 92, JM#195; SEQ ID NO.: 93, JM#196; SEQ ID NO.: 94, JM#197) strongly increased anti-HIV-1 activity. Potency of JM#21 (SEQ ID NO.: 23) coupled to palmitic acid was increased by more than 60-fold (IC₅₀ of JM#143 (SEQ ID NO.: 54) = 5 nM, not shown) compared to WSC02 (SEQ ID NO.: 2) and C-terminal truncations and amidation of those derivatives even further increased anti-HIV-1 activity (JM#192 = 2nM, not shown, JM#194 = 2 nM). Experiments were done in triplicates. The error bars refer to the standard deviation.

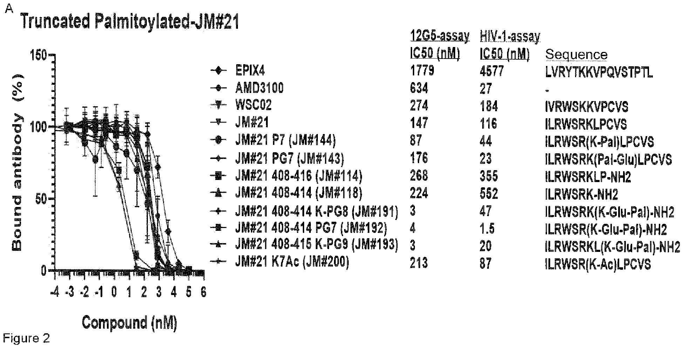

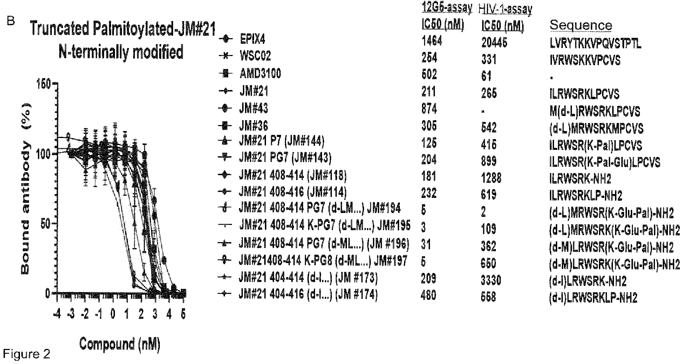

Second, the activity was tested by an antibody competition assay (Fig. 2). Values determined by the competition assay represent the potency of a compound to compete with an antibody that specifically binds to the binding pocket (ECL2) of CXCR4. Those values most probable correlate with CXCR4 binding affinity of the compounds to CXCR4. In Fig. 2 (A and B), results of two assays are shown by way of diagrams wherein the percentage of bound antibody depends on the concentration of different peptides. To the right of the diagrams, an assignment of the curves to the tested peptides is explained. To the right of the legend a table shows the values of the IC50 in the completion assay (12G5-assay), in a HIV-1-assay and the individual sequences of the tested peptides. All tested EPI-X4 derivatives compete with binding of the antibody. JM#21 (SEQ ID NO.: 23) led to a more effective antibody competition (lower IC₅₀s) than WSC02 (SEQ ID NO.: 2) (IC₅₀ = 350 nM) and AMD3100 (IC₅₀ = 690 nM). Taking JM#21 (SEQ ID NO.: 23) as a lead for further development, we designed derivatives that bind with a similar affinity to CXCR4 than JM#21 (SEQ ID NO.: 23), however have molecular weight below 1000 Da (eg. JM#118). Strikingly, coupling of fatty acids to those shorter derivatives strongly increase binding to CXCR4. Compared to EPI-X4 (SEQ ID NO.: 1) (IC₅₀ ∼2500 nM) some of the derivatives bind more than 2000-fold more effectively to the receptor (eg. IC₅₀ of: JM#167 (SEQ ID NO.: 64) = 2 nM; JM#178 (SEQ ID NO.: 75) = 2 nM; JM#191 (SEQ ID NO.: 88) = 1 nM) what is about 300-fold more effective than WSC02 (SEQ ID NO.: 2). Three independent experiments were carried out for each peptide in each assay. The error bars refer to the standard deviation.

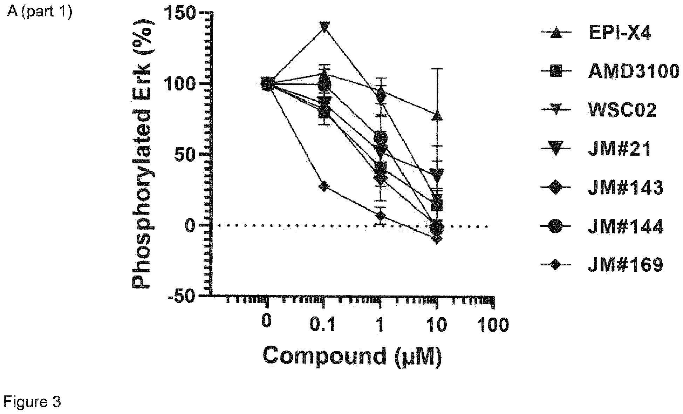

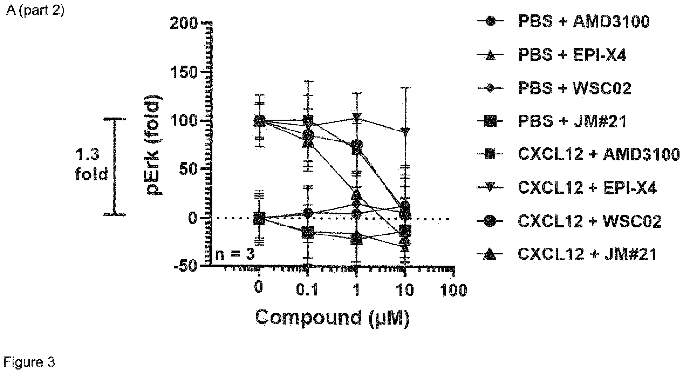

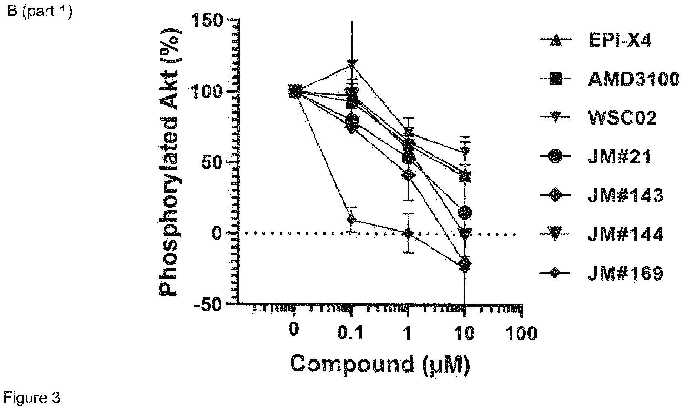

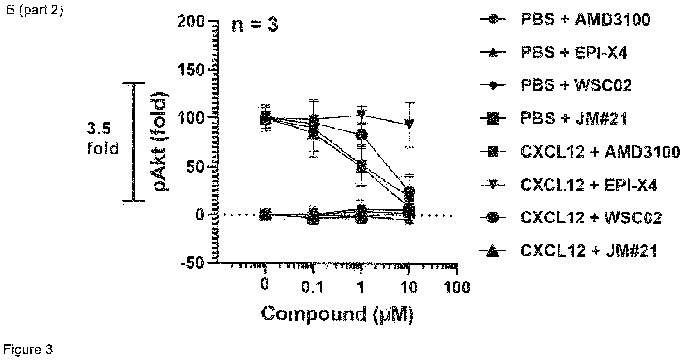

Third, the activity of the claimed EPI-X4 derivatives were tested by the effect on CXCL12/CXCR4-mediated ERK (Extracellular-signal Regulated Kinases) and AKT (a serine/threonine-protein kinase) signaling (Fig. 3). In that assay CXCR4 expressing cells are stimulated with the CXCR4 chemokine-ligand CXCL12, which subsequently leads to phosphorylation of ERK and AKT. Preincubation of cells with CXCR4-antagonists blocks those pathways. In Fig. 3, the results of two assays are shown by way of diagrams wherein the percentage of phosphorylated ERK (Fig. 3A) and of phosphorylated AKT (Fig. 3B) depends on the concentration of different peptides. All tested EPI-X4 derivatives blocked CXCL12 induced signaling in a dose dependent manner. JM#21 (SEQ ID NO.: 23) blocked signaling more effectively than WSC02. 10 µM WSC02 (SEQ ID NO.: 2) reduced AKT signaling by about 50 %, JM#21 (SEQ ID NO.: 23) led to an about 85 % inhibition with the same concentration. At a concentration of 1 µM JM#21 blocked CXCL12-induced AKT signaling by about 50% what is about 10-fold as effective as WSC02 (SEQ ID NO.: 2). Also ERK signaling was effectively blocked by JM#21 (SEQ ID NO.: 23) (60% reduction at a concentration of 1 µM), whereas WSC02 (SEQ ID NO.: 2) only reduced Erk phosphorylation by 20% at the same concentration. JM#21 (SEQ ID NO.: 23) was also more effective than AMD3100 (reduction of AKT phosphorylation at 10 µM by about 60 %) and EPI-X4 (SEQ ID NO.: 1) (reduction at 10 µM by about 40 %). Also JM#18 (SEQ ID NO.: 20) had a very strong antagonistic effect in this assay. JM#18 (SEQ ID NO.: 20) blocked CXCL12-induced AKT and ERK signaling by about 40 % at a concentration of 0.1 µM and by almost 70 % at a concentration of 1 µM (not shown). Interestingly, palmitic acid coupling led to an increased antagonistic effect. At a concentration of 10 µM all tested palmitic acid coupled derivatives blocked CXCL 12 induced AKT and ERK signaling by 100 %. JM#143 (SEQ ID NO.: 54), a fatty acid coupled derivative of JM#21 (SEQ ID NO.: 23), blocked AKT and ERK phosphorylation at a concentration of 1 µM by almost 70 % and even at a concentration of 0.1 µM signaling was reduced by 20 - 25 %. Strikingly, pamitic acid coupled WSC02 (JM#169, (SEQ ID NO.: 66) had a very strong antagonistic activity and reduced AKT signaling by 85 % and ERK signaling by over 70% at a concentration of already 0.1 µM, at a concentration of 1 µM AKT signaling was completely and ERK signaling almost completely blocked. Two or three independent experiments were carried out in triplicates for each peptide in each assay. The error bars refer to the standard deviation.

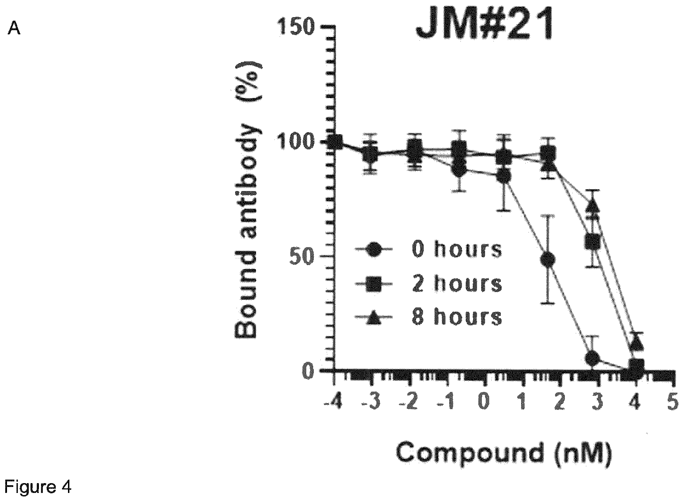

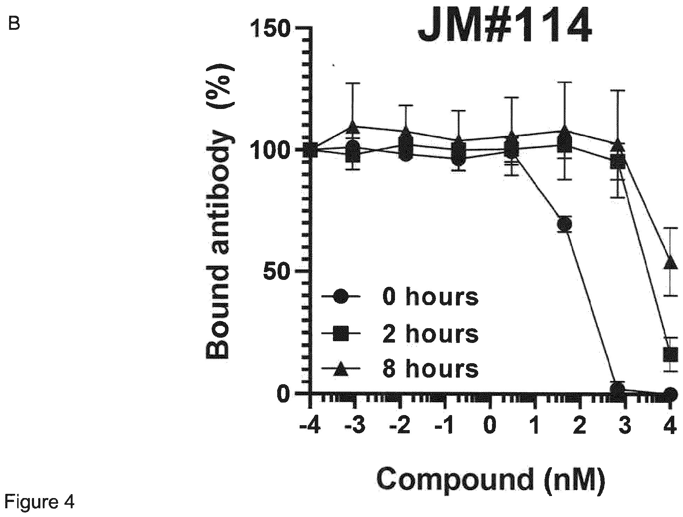

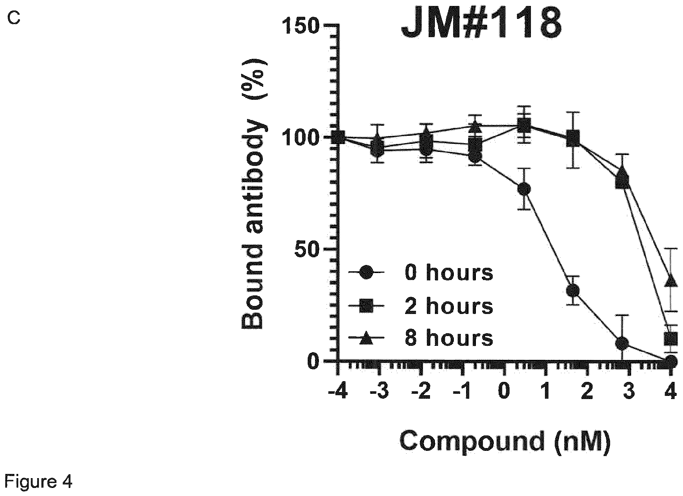

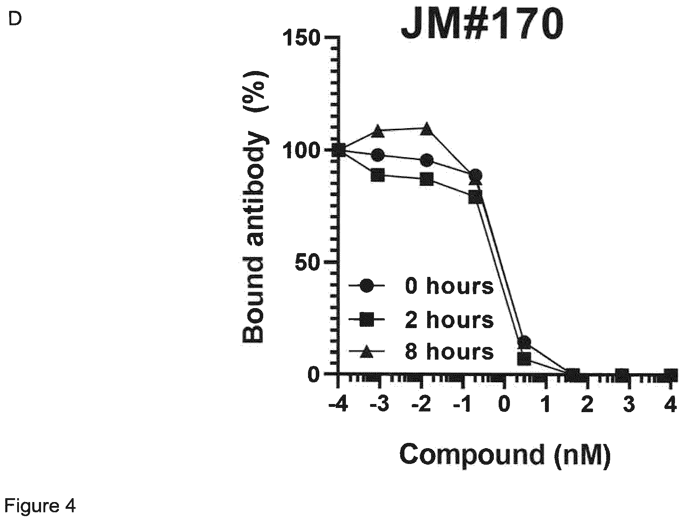

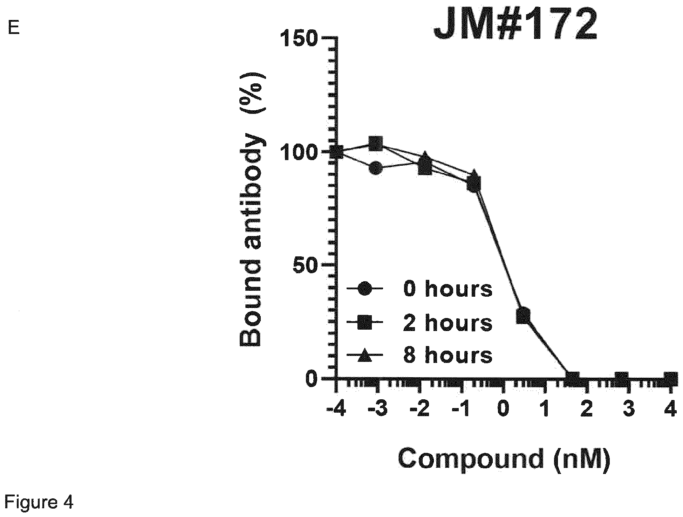

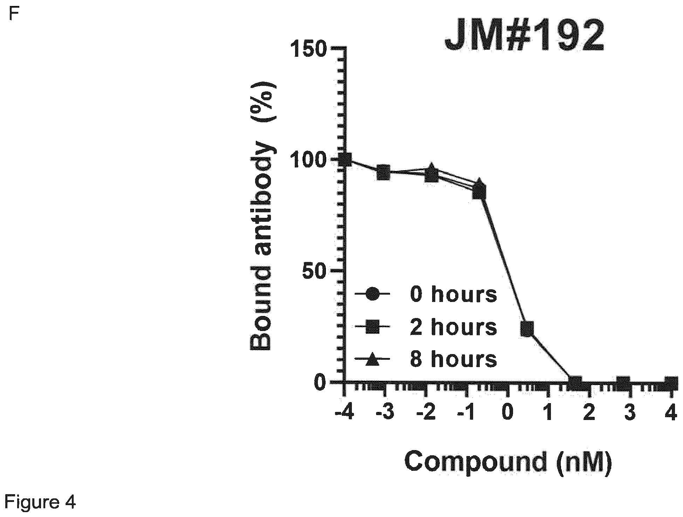

Fourth, the stability of peptides in human plasma and whole human blood was tested (Fig. 4). The functional stability of EPI-X4 (SEQ ID NO.: 1) derivatives was screened using a CXCR4 antibody-based screening approach. Peptides were diluted in full human plasma or blood (> 99 %) and the functional activity was then measured after either 2 hours or 8 hours and compared to a sample that was not incubated in plasma or blood.

In the diagrams of Fig. 4, the stability of the peptides is shown by way of the percentage of bound antibody depending on the molar concentration of the peptides. The functional stability of both WSC02 (SEQ ID NO.: 2) and JM#21 (SEQ ID NO.: 23) was very low compared to EPI-X4 (SEQ ID NO.: 1) (t ½ = 17 min) (Fig. 4A). After 2 hours both optimized peptides were completely inactive. Rapid degradation of those variants was also confirmed using mass spectrometry. Also, the truncated variants of both (JM#114 (SEQ ID NO.: 49), Fig. 4B, and JM#118 (SEQ ID NO.: 50), Fig. 4C) were rapidly inactivated in plasma. Using mass spectrometry, we showed that enzymatic degradation of EPI-X4 (SEQ ID NO.: 1) derivatives was exclusively detected at the N-terminus. EPI-X4 (SEQ ID NO.: 1) variants that were modified at the N-terminus (JM#25 (SEQ ID NO.: 27) - JM#44 (SEQ ID NO.: 46)) had a strongly increased functional plasma stability compared to WSC02 (SEQ ID NO.: 2). Whereas the anti-CXCR4 activity was not or not strongly affected (IC50 HIV-1 inhibition assay for: JM#28 (SEQ ID NO.: 30) = 246 nM; JM#36 (SEQ ID NO.: 38) = 206 nM; JM#43 (SEQ ID NO.: 45) = 810 nM) activity was still remained after 2 and even 8 hours of plasma incubation (Remaining activity of: JM#28 (SEQ ID NO.: 30) = 96 % after 2 hours and 77 % after 8 hours; JM#36 (SEQ ID NO.: 38) = 85 % after 2 hours and 81 % after 8 hours; JM#43 (SEQ ID NO.: 45) = 100 % after 2 and 8 hours).

We further aimed to increase plasma stability (and also bioavailability) and therefore designed EPI-X4 (SEQ ID NO.: 1) derivatives that are coupled to fatty acids (e.g. palmitic acid). Most of the fatty acid coupled derivatives had a strongly increased plasma stability as shown e.g. for fatty acid coupled JM#21 (JM#143 (SEQ ID NO.: 54) - JM#145 (SEQ ID NO.: 56)) that did not lose activity at all after 8 hours of plasma incubation. Strikingly, the same is true also for most truncated and fatty acid coupled versions of JM#21 (SEQ ID NO.: 23), WSC02 (SEQ ID NO.: 2) or similar (e.g. JM#170 (SEQ ID NO.: 67), Fig. 4D - JM#172 (SEQ ID NO.: 69), Fig. 4E) and JM#191 (SEQ ID NO.: 88) - JM#193 (SEQ ID NO.: 90) (Fig. 4F for JM#192)). The same is true for those variants which were functional stable in the stability assay if combined with the N-terminal modifications (e.g. JM#194 (SEQ ID NO.: 91) - JM#197 (SEQ ID NO.: 94)).

An alternative approach of stabilization was the PEGylation of JM#21 (SEQ ID NO.: 23). PEGylation only marginally or not at all affected anti-CXCR4 activity of the variants (IC50 in HIV-1 inhibition assay for: SC024 (20 kDa) = 118 nM, SC029 (telechelic peptide conjugate, 20 kDa) = 98 nM, SC033 (5 kDa) = 716 nM) however strongly increased functional plasma stability (Remaining activity after 8 hours of plasma incubation for: SC024 = 30 %, SC029 = 38 %, SC033 = 72%). In Fig. 4 A - C, three individual experiments were carried out. The error bars refer to the standard deviation. Fig. 4 D - F are based on one representative experiment.

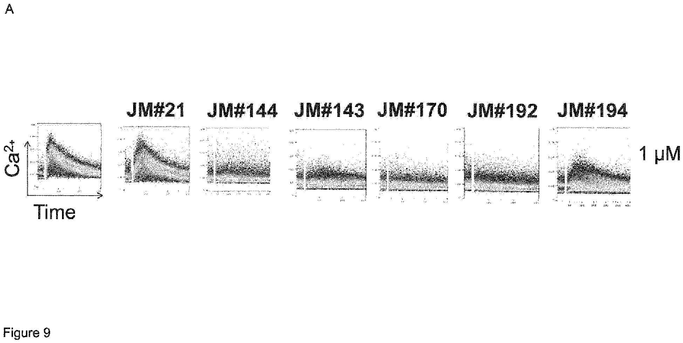

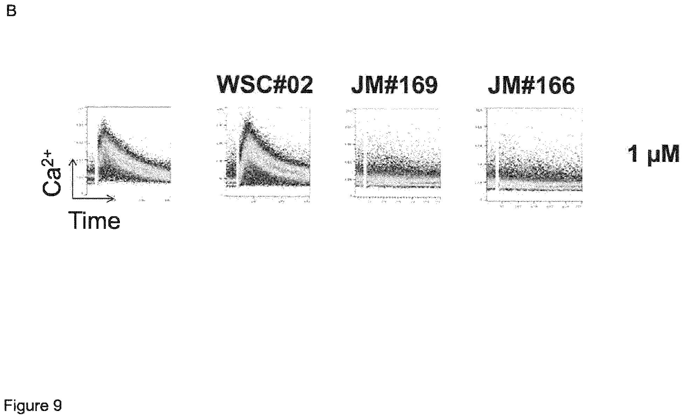

Fifth, it was shown that the peptide derivates inhibit calcium-signalling. CXCL12 stimulation of CXCR4 expressing B-cells leads to a strong calcium-release. In the presence of CXCR4 antagonists this response is reduced. We saw a reduction of cytokine induced calcium-signaling for 1 µM of JM#21 (SEQ ID NO.: 23) that was much stronger than the reduction seen for WSC02 (SEQ ID NO.: 2) at the same concentration. Fatty acid coupled JM#21 (SEQ ID NO.: 23) variants (JM#143 (SEQ ID NO.: 54), JM#144 (SEQ ID NO.: 55), JM#170 (SEQ ID NO.: 67), JM#192 (SEQ ID NO.: 89), JM#194 (SEQ ID NO.: 91)) (Fig. 9A) as well as fatty acid coupled WSC02 (SEQ ID NO.: 2) variants (Fig. 9B) almost completely blocked calcium-signaling at a concentration of 1 µM. For each peptide, Fig. 9 shows the result of one representative experiment.

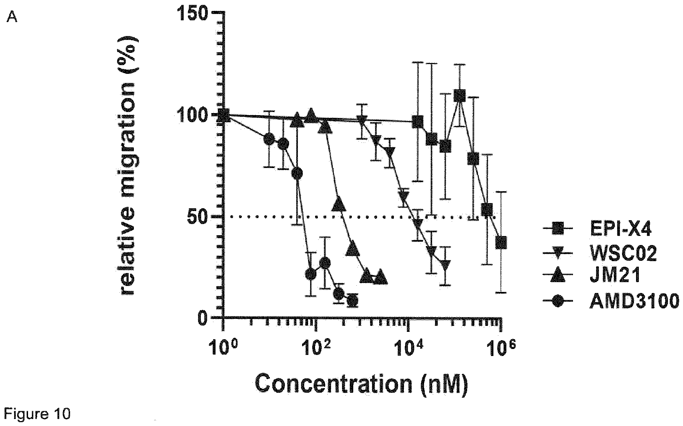

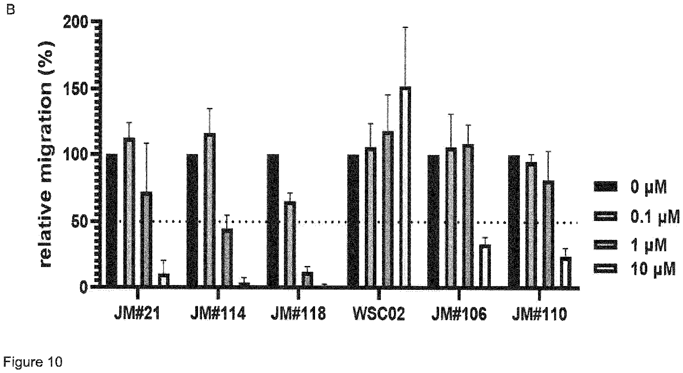

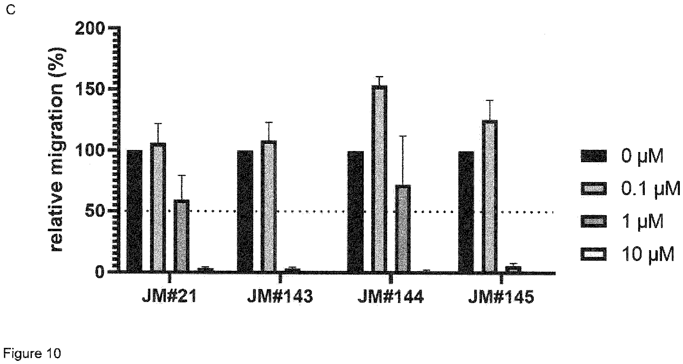

Sixth, all tested EPI-X4 derivatives inhibited CXCL12 induced migration of T-cells. JM#21 (SEQ ID NO.: 23) was more effective than WSC02 (SEQ ID NO.: 2) and EPI-X4 (SEQ ID NO.: 1) (Fig. 10A). Truncated versions of JM#21 (SEQ ID NO.: 23) (JM#114 (SEQ ID NO.: 49), JM#118 (SEQ ID NO.: 50)) were even more effective than the full-length peptide (Fig. 10B). This effect could also be shown truncated versions of WSC02 (SEQ ID NO.: 2) (JM#106 (SEQ ID NO.: 47), JM#110 (SEQ ID NO.: 48)). Tested fatty acid coupled variants of JM#21 (SEQ ID NO.: 23) (especially JM#143 (SEQ ID NO.: 54)) had a strongly increased antagonistic effect in CXCL12 induced cell migration (Fig. 10C). For each peptide, three independent experiments were performed. The error bars refer to the standard deviation.

A toxicity test in Zebrafish showed that all tested derivatives were not toxic for Zebrafish Embryos.

The peptides of group 1 are characterized by an inhibitory activity characterized by a half maximal inhibitory concentration (IC₅₀) of below 5 nM, as measured in an X4-HIV-1 inhibition assay (in order to estimate the capability of blocking X4-HIV-1 infection). The X4-HIV inhibition assay is designed to measure the activity of the inventive peptides by their efficiency of blocking the infection of tissue culture cells by CXCR4-tropic HIV-1 variants.

The peptides of group 2 are characterized by an inhibitory activity characterized by an IC₅₀ between 5 and 10 nM, as measured in an HIV inhibition assay.

The peptides of group 3 are characterized by an inhibitory activity characterized by an IC₅₀ between 10 and 50 nM, as measured in an HIV inhibition assay.

The peptides of group 4 are characterized by an inhibitory activity characterized by an IC₅₀ between 50 and 150 nM, as measured in an HIV inhibition assay.

The peptides of group 5 are characterized by an inhibitory activity characterized by an IC₅₀ of above 150 nM, as measured in an HIV inhibition assay.

The peptides of group 6 are characterized by an IC₅₀ below 25 nM, as measured in an antibody competition assay. These peptides had an IC₅₀ of above 150 nM, as measured in an HIV inhibition assay.

The peptides of group 7 are characterized by a relative activity of 100%, as measured after 8 hours of plasma incubation. In this test, the peptides were incubated in human plasma for a certain time period. The relative activity is estimated by measuring the maintenance of the capability of blocking X4-HIV-1 infection over the certain time period.

The peptides of group 8 are characterized by a relative activity of 100% after 2 hours of plasma incubation, but less (75 - 99%) after 8 hours of plasma incubation.

The peptides of group 9 are characterized by a relative activity of 70 - 99% after 2 hours of plasma incubation.

The peptides of group 10 are characterized by both an IC₅₀ below 50 nM and a relative activity of 100% after 8 hours of plasma incubation. With other words, these peptides were shown to maintain a high activity over a relatively long period of time.

Substitution of the N-terminal amino group, introduction of D-Amino acids as well as certain amino acid substitutions at the N-terminus of the peptide have been shown to inhibit protease activity. These kinds of modification are advantageous because they increase the plasma stability of the peptides, as indicated by a half-life of up to 29 hours.

The coupling of fatty acids, i.e. of palmitic acid, decanoic acid, myristic acid, oleic acid, and stearic acid has been shown to provide higher activity of the peptides. Furthermore, the coupling of fatty acids could prolong the circulation of the peptides at a certain level of concentration *in vivo.*

Cholesterol has been shown to increase the relative stability of the peptides in human plasma as well as the biological availability.

A second aspect of the invention is directed to a peptide consisting of one of the following amino acid sequences, selected from any of the groups 1 to 10, wherein
- Group 1 consists of

| | |
|---|---|
| ILRWSRKMPCVS | (SEQ ID NO.: 15, JM#13) |
| IMRWSRKMPCVS | (SEQ ID NO.: 19, JM#17) |
| ILRWSRKMPCLS | (SEQ ID NO.: 20, JM#18) |
| ILRWSRKMPCMS | (SEQ ID NO.: 22, JM#20) |
| ILRWSRKLPCVS | (SEQ ID NO.: 23, JM#21) |
| ILRWSRKFPCVS | (SEQ ID NO.: 24, JM#22) |
| ILRWSRK(Pal)LPCVS | (SEQ ID NO.: 55, JM#144) |
| ILRWSRK(Glu-Pal)-NH2 | (SEQ ID NO.: 89, JM#192) |
| d-LMRWSRK(Glu-Pal)-NH2 | (SEQ ID NO.: 91, JM#194) |

- Group 2 consists of

| | |
|---|---|
| ILRWSRKMPCFS | (SEQ ID NO.: 21, JM#19) |
| ILRWSRK(Pal)L-NH2 | (SEQ ID NO.: 69, JM#172) |
| d-LMRWSRK(Pal)MP-NH2 | (SEQ ID NO.: 75, JM#178) |
| d-LMRWSRK(Pal)-NH2 | (SEQ ID NO.: 77, JM#180) |
| ILRWSRK(Ole)LPCVS | (SEQ ID NO.: 80, JM#183) |

- Group 3 consists of

| | |
|---|---|
| d-LLRWSRK(Pal)MPCVS | (SEQ ID NO.: 53, JM#141) |
| IVRWSK(Pal)KVP-NH2 | (SEQ ID NO.: 63, JM#166) |
| IVRWSKK(Pal)VP-NH2 | (SEQ ID NO.: 64, JM#167) |
| IVRWSKK(Pal)VPCVS | (SEQ ID NO.: 66, JM#169) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRKK(Glu-Pal)-NH2 | (SEQ ID NO.: 88, JM#191) |
| ILRWSRKLK(Glu-Pal)-NH2 | (SEQ ID NO.: 90, JM#193) |
| ILRWSRKK(Glu-Ste)LPCVS | (SEQ ID NO.: 96, JM#199) |
| ILRWSRK(Glu-Dec)LPCVS | (SEQ ID NO.: 98, JM#203) |
| ILRWSRK(Glu-Ste)LPCVS | (SEQ ID NO.: 100, JM#205) |

- Group 4 consists of

| | |
|---|---|
| ILRWSRKVPCVS | (SEQ ID NO.: 10, JM#8) |
| IFRWSRKVPCVS | (SEQ ID NO.: 12, JM#10) |
| MLRWSRKMPCVS | (SEQ ID NO.: 29, JM#27) |
| MMRWSRKMPCVS | (SEQ ID NO.: 36, JM#34) |
| MLRWSRKLPCVS | (SEQ ID NO.: 41, JM#39) |
| ILRWSRKLPSVS | (SEQ ID NO.: 51, JM#122) |
| d-LLRWSRK(Glu-Pal)MPCVS | (SEQ ID NO.: 52, JM#140) |
| ILRWSRK(Pal)MPCLS | (SEQ ID NO.: 59, JM#149) |
| d-LMRWSRKK(Glu-Pal)-NH2 | (SEQ ID NO.: 92, JM#195) |
| ILRWSRK-AcLPCVS | (SEQ ID NO.: 97, JM#200) |

- Group 5 consisting of

| | |
|---|---|
| ILRWSKKVPCVS | (SEQ ID NO.: 3, JM#1) |
| IFRWSKKVPCVS | (SEQ ID NO.: 4, JM#2) |
| IVRWSRKVPCVS | (SEQ ID NO.: 5, JM#3) |
| IVRWSHKVPCVS | (SEQ ID NO.: 6, JM#4) |
| IVRWSKKLPCVS | (SEQ ID NO.: 7, JM#5) |
| IVRWSKKIPCVS | (SEQ ID NO.: 8, JM#6) |
| IVRWSKKFPCVS | (SEQ ID NO.: 9, JM#7) |
| ILRWSHKVPCVS | (SEQ ID NO.: 11, JM#9) |
| IFRWSHKVPCVS | (SEQ ID NO.: 13, JM#11) |
| IVRWSKKMPCVS | (SEQ ID NO.: 14, JM#12) |
| IVRWSKKVPCd-VS | (SEQ ID NO.: 16, JM#14) |
| ILRWSRKVPCd-VS | (SEQ ID NO.: 17, JM#15) |
| IIRWSRKMPCVS | (SEQ ID NO.: 18, JM#16) |
| ILRWSRKVPSVS | (SEQ ID NO.: 25, JM#23) |
| ILRWSRKMPSVS | (SEQ ID NO.: 26, JM#24) |
| Ac-SLRWSRKMPCVS | (SEQ ID NO.: 27, JM#25) |
| d-Ac-SLRWSRKMPCVS | (SEQ ID NO.: 28, JM#26) |
| d-MLRWSRKMPCVS | (SEQ ID NO.: 30, JM#28) |
| d-LLRWSRKMPCVS | (SEQ ID NO.: 31, JM#29) |
| d-FLRWSRKMPCVS | (SEQ ID NO.: 32, JM#30) |
| GLRWSRKMPCVS | (SEQ ID NO.: 33, JM#31) |
| Ac-SMRWSRKMPCVS | (SEQ ID NO.: 34, JM#32) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-FMRWSRKMPCVS | (SEQ ID NO.: 39, JM#37) |
| d-GMRWSRKMPCVS | (SEQ ID NO.: 40, JM#38) |
| d-MLRWSRKLPCVS | (SEQ ID NO.: 42, JM#40) |
| Id-LRWSRKLPCVS | (SEQ ID NO.: 43, JM#41) |
| Id-LRWSRKMPCVS | (SEQ ID NO.: 44, JM#42) |
| Md-LRWSRKLPCVS | (SEQ ID NO.: 45, JM#43) |
| Md-LRWSRKMPCVS | (SEQ ID NO.: 46, JM#44) |
| IVRWSKKVP-NH2 | (SEQ ID NO.: 47, JM#106) |
| IVRWSKK-NH2 | (SEQ ID NO.: 48, JM#110) |
| ILRWSRKLP-NH2 | (SEQ ID NO.: 49, JM#114) |
| ILRWSRK-NH2 | (SEQ ID NO.: 50, JM#118) |
| ILRWSRK(Glu-Pal)LPCVS | (SEQ ID NO.: 54, JM#143) |
| ILRWSRKLPCK(Glu-Pal)S | (SEQ ID NO.: 56, JM#145) |
| IYRWSRKMPCLS | (SEQ ID NO.: 57, JM#146) |
| ILRWSRK(Glu-Pal)MPCLS | (SEQ ID NO.: 58, JM#148) |
| IVRWSKKVPSVS | (SEQ ID NO.: 60, JM#151) |
| IVRWSK(Pal)K-NH2 | (SEQ ID NO.: 61, JM#164) |
| IVRWSKK(Pal)-NH2 | (SEQ ID NO.: 62, JM#165) |
| IVRWSK(Pal)KVPCVS | (SEQ ID NO.: 65, JM#168) |
| d-ILRWSRK-NH2 | (SEQ ID NO.: 70, JM#173) |
| d-ILRWSRKLP-NH2 | (SEQ ID NO.: 71, JM#174) |
| d-ILRWSRK(Pal)LP-NH2 | (SEQ ID NO.: 72, JM#175) |
| d-LMRWSRK(Pal)MPCVS | (SEQ ID NO.: 73, JM#176) |
| Md-LRWSRK(Pal)LPCVS | (SEQ ID NO.: 74, JM#177) |
| Md-LRWSRK(Pal)LP-NH2 | (SEQ ID NO.: 76, JM#179) |
| Md-LRWSRK(Pal)-NH2 | (SEQ ID NO.: 78, JM#181) |
| ILRWSRK(Ste)LPCVS | (SEQ ID NO.: 79, JM#182) |
| ILRWSRK(Chl)LPCVS | (SEQ ID NO.: 81, JM#184) |
| Ac-ILRWSRKLPCVS | (SEQ ID NO.: 82, JM#185) |
| d-Ac-ILRWSRKLPCVS | (SEQ ID NO.: 83, JM#186) |
| Ac-MLRWSRKLPCVS | (SEQ ID NO.: 84, JM#187) |
| d-Ac-MLRWSRKLPCVS | (SEQ ID NO.: 85, JM#188) |
| VLRWSRKLPCVS | (SEQ ID NO.: 86, JM#189) |
| d-VLRWSRKLPCVS | (SEQ ID NO.: 87, JM#190) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197) |
| ILRWSRK(Glu-Ste)LPCVS | (SEQ ID NO.: 95, JM#198) |
| ILRWSRK(Glu-Myr)LPCVS | (SEQ ID NO.: 99, JM#204) |

- Group 6 consists of

| | |
|---|---|
| d-LMRWSRKK(Glu-Pal)-NH2 | (SEQ ID NO.: 92, JM#195) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197) |
| ILRWSRK(Glu-Ste)LPCVS | (SEQ ID NO.: 95, JM#198) |

- Group 7 consists of

| | |
|---|---|
| Md-LRWSRKLPCVS | (SEQ ID NO.: 45, JM#43) |
| Md-LRWSRKMPCVS | (SEQ ID NO.: 46, JM#44) |
| ILRWSRK(Glu-Pal)LPCVS | (SEQ ID NO.: 54, JM#143) |
| ILRWSRK(Pal)LPCVS | (SEQ ID NO.: 55, JM#144) |
| ILRWSRKLPCK(Glu-Pal)S | (SEQ ID NO.: 56, JM#145) |
| ILRWSRK(Pal)MPCLS | (SEQ ID NO.: 59, JM#149) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| d-ILRWSRK-NH2 | (SEQ ID NO.: 70, JM#173) |
| d-ILRWSRKLP-NH2 | (SEQ ID NO.: 71, JM#174) |

- Group 8 consists of

| | |
|---|---|
| d-LLRWSRKMPCVS | (SEQ ID NO.: 31, JM#29) |
| ILRWSRK(Pal)L-NH2 | (SEQ ID NO.: 69, JM#172) |
| ILRWSRK(Glu-Pal)-NH2 | (SEQ ID NO.: 89, JM#192) |

- Group 9 consists of

| | |
|---|---|
| Ac-SLRWSRKMPCVS | (SEQ ID NO.: 27, JM#25) |
| d-MLRWSRKMPCVS | (SEQ ID NO.: 30, JM#28) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-LLRWSRK(Glu-Pal)MPCVS | (SEQ ID NO.: 52, JM#140) |
| d-LMRWSRK(Pal)MP-NH2 | (SEQ ID NO.: 75, JM#178) |

- Group 10 consists of

| | |
|---|---|
| ILRWSRK(Pal)LPCVS | (SEQ ID NO.: 55, JM#144) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRK(Pal)L-NH2 | (SEQ ID NO.: 69, JM#172) |
| d-LMRWSRK(Pal)-NH2 | (SEQ ID NO.: 77, JM#180) |
| ILRWSRKK(Glu-Pal)-NH2 | (SEQ ID NO.: 88, JM#191) |
| ILRWSRK(Glu-Pal)-NH2 | (SEQ ID NO.: 89, JM#192) |
| ILRWSRKLK(Glu-Pal)-NH2 | (SEQ ID NO.: 90, JM#193) |
| d-LMRWSRK(Glu-Pal)-NH2 | (SEQ ID NO.: 91, JM#194) |

wherein d- in front of an amino acid indicates a D-Amino acid, Pal indicates a palmitic acid on the preceding amino acid, Glu-Pal indicates a palmitic acid on the preceding amino acid with a glutamate linker, Dec indicates decanoic acid on the preceding amino acid, Glu-Dec indicates a decanoic acid on the preceding amino acid with a glutamate linker, Myr indicates myristic acid on the preceding amino acid, Glu-Myr indicates a myristic acid on the preceding amino acid with a glutamate linker, Ole indicates oleic acid on the preceding amino acid, Ste indicates stearic acid on the preceding amino acid, Glu-Ste indicates a stearic acid on the preceding amino acid with a glutamate linker, Chl indicates Cholesterol, and Ac indicates a substitution of an amino group by an acetyl group,
and wherein the peptide is C-terminally conjugated to a complexing agent.

In a particularly preferred embodiment, the complexing agent is the chelator DOTA. With other words, the peptide is C-terminally conjugated to the chelator DOTA. DOTA (also known as tetraxetan) is an organic compound with the formula (CH₂CH₂NCH₂CO₂H)₄. The molecule consists of a central 12-membered tetraaza (i.e., containing four nitrogen atoms) ring. The acronym DOTA (for dodecane tetraacetic acid) is shorthand for both the tetracarboxylic acid and its various conjugate bases. DOTA-conjugated peptides are suitable for labelling with radioactive nucleotides, e.g. ⁶⁸Ga and ¹⁷⁷Lu. Consequently, these peptides are useful in applications in diagnostic and therapeutic approaches. The inventive EPI-X4 (SEQ ID NO.: 1) derivates, which are specific for the CXCR4, can be used for blending diagnostic and therapeutic with the same molecule (radiotheranostics). Radiotheranostics based on these peptides is offering new imaging testes and therapeutic options to patients suffering from CXCR4-expressing malignancies.

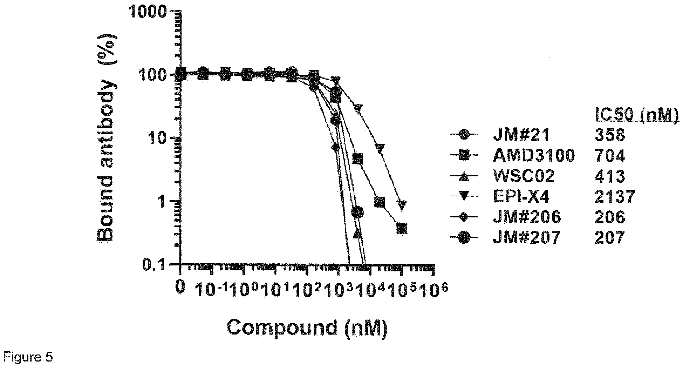

Fig. 5 shows a diagram of an antibody competition assay (based on one representative experiment per peptide), wherein the percentage of bound antibody is depending on the molar concentration of the shown peptides. It is demonstrated that the DOTA-conjugated peptides JM#206 (SEQ ID NO.: 101) (JM#21 (SEQ ID NO.: 23) with DOTA) and JM#207 (SEQ ID NO.: 102) (JM#122 (SEQ ID NO.: 51) with DOTA) displaced the antibody as efficiently as the non-conjugated JM#21 (SEQ ID NO.: 23). Similarly, both DOTA-conjugated peptides suppressed HIV-1 infection with similar potency as the non-conjugated peptide.

A third aspect of the invention is directed to a peptide consisting of one of the following amino acid sequences, selected from any of the groups 1 to 10, wherein
- Group 1 consists of

| | |
|---|---|
| ILRWSRKMPCVS | (SEQ ID NO.: 15, JM#13) |
| IMRWSRKMPCVS | (SEQ ID NO.: 19, JM#17) |
| ILRWSRKMPCLS | (SEQ ID NO.: 20, JM#18) |
| ILRWSRKMPCMS | (SEQ ID NO.: 22, JM#20) |
| ILRWSRKLPCVS | (SEQ ID NO.: 23, JM#21) |
| ILRWSRKFPCVS | (SEQ ID NO.: 24, JM#22) |
| ILRWSRK(Pal)LPCVS | (SEQ ID NO.: 55, JM#144) |
| ILRWSRK(Glu-Pal)-NH2 | (SEQ ID NO.: 89, JM#192) |
| d-LMRWSRK(Glu-Pal)-NH2 | (SEQ ID NO.: 91, JM#194) |

- Group 2 consists of

| | |
|---|---|
| ILRWSRKMPCFS | (SEQ ID NO.: 21, JM#19) |
| ILRWSRK(Pal)L-NH2 | (SEQ ID NO.: 69, JM#172) |
| d-LMRWSRK(Pal)MP-NH2 | (SEQ ID NO.: 75, JM#178) |
| d-LMRWSRK(Pal)-NH2 | (SEQ ID NO.: 77, JM#180) |
| ILRWSRK(Ole)LPCVS | (SEQ ID NO.: 80, JM#183) |

- Group 3 consists of

| | |
|---|---|
| d-LLRWSRK(Pal)MPCVS | (SEQ ID NO.: 53, JM#141) |
| IVRWSK(Pal)KVP-NH2 | (SEQ ID NO.: 63, JM#166) |
| IVRWSKK(Pal)VP-NH2 | (SEQ ID NO.: 64, JM#167) |
| IVRWSKK(Pal)VPCVS | (SEQ ID NO.: 66, JM#169) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRKK(Glu-Pal)-NH2 | (SEQ ID NO.: 88, JM#191) |
| ILRWSRKLK(Glu-Pal)-NH2 | (SEQ ID NO.: 90, JM#193) |
| ILRWSRKK(Glu-Ste)LPCVS | (SEQ ID NO.: 96, JM#199) |
| ILRWSRK(Glu-Dec)LPCVS | (SEQ ID NO.: 98, JM#203) |
| ILRWSRK(Glu-Ste)LPCVS | (SEQ ID NO.: 100, JM#205) |

- Group 4 consists of

| | |
|---|---|
| ILRWSRKVPCVS | (SEQ ID NO.: 10, JM#8) |
| IFRWSRKVPCVS | (SEQ ID NO.: 12, JM#10) |
| MLRWSRKMPCVS | (SEQ ID NO.: 29, JM#27) |
| MMRWSRKMPCVS | (SEQ ID NO.: 36, JM#34) |
| MLRWSRKLPCVS | (SEQ ID NO.: 41, JM#39) |
| ILRWSRKLPSVS | (SEQ ID NO.: 51, JM#122) |
| d-LLRWSRK(Glu-Pal)MPCVS | (SEQ ID NO.: 52, JM#140) |
| ILRWSRK(Pal)MPCLS | (SEQ ID NO.: 59, JM#149) |
| d-LMRWSRKK(Glu-Pal)-NH2 | (SEQ ID NO.: 92, JM#195) |
| ILRWSRK-AcLPCVS | (SEQ ID NO.: 97, JM#200) |

- Group 5 consisting of

| | |
|---|---|
| ILRWSKKVPCVS | (SEQ ID NO.: 3, JM#1) |
| IFRWSKKVPCVS | (SEQ ID NO.: 4, JM#2) |
| IVRWSRKVPCVS | (SEQ ID NO.: 5, JM#3) |
| IVRWSHKVPCVS | (SEQ ID NO.: 6, JM#4) |
| IVRWSKKLPCVS | (SEQ ID NO.: 7, JM#5) |
| IVRWSKKIPCVS | (SEQ ID NO.: 8, JM#6) |
| IVRWSKKFPCVS | (SEQ ID NO.: 9, JM#7) |
| ILRWSHKVPCVS | (SEQ ID NO.: 11, JM#9) |
| IFRWSHKVPCVS | (SEQ ID NO.: 13, JM#11) |
| IVRWSKKMPCVS | (SEQ ID NO.: 14, JM#12) |
| IVRWSKKVPCd-VS | (SEQ ID NO.: 16, JM#14) |
| ILRWSRKVPCd-VS | (SEQ ID NO.: 17, JM#15) |
| IIRWSRKMPCVS | (SEQ ID NO.: 18, JM#16) |
| ILRWSRKVPSVS | (SEQ ID NO.: 25, JM#23) |
| ILRWSRKMPSVS | (SEQ ID NO.: 26, JM#24) |
| Ac-SLRWSRKMPCVS | (SEQ ID NO.: 27, JM#25) |
| d-Ac-SLRWSRKMPCVS | (SEQ ID NO.: 28, JM#26) |
| d-MLRWSRKMPCVS | (SEQ ID NO.: 30, JM#28) |
| d-LLRWSRKMPCVS | (SEQ ID NO.: 31, JM#29) |
| d-FLRWSRKMPCVS | (SEQ ID NO.: 32, JM#30) |
| GLRWSRKMPCVS | (SEQ ID NO.: 33, JM#31) |
| Ac-SMRWSRKMPCVS | (SEQ ID NO.: 34, JM#32) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-FMRWSRKMPCVS | (SEQ ID NO.: 39, JM#37) |
| d-GMRWSRKMPCVS | (SEQ ID NO.: 40, JM#38) |
| d-MLRWSRKLPCVS | (SEQ ID NO.: 42, JM#40) |
| Id-LRWSRKLPCVS | (SEQ ID NO.: 43, JM#41) |
| Id-LRWSRKMPCVS | (SEQ ID NO.: 44, JM#42) |
| Md-LRWSRKLPCVS | (SEQ ID NO.: 45, JM#43) |
| Md-LRWSRKMPCVS | (SEQ ID NO.: 46, JM#44) |
| IVRWSKKVP-NH2 | (SEQ ID NO.: 47, JM#106) |
| IVRWSKK-NH2 | (SEQ ID NO.: 48, JM#110) |
| ILRWSRKLP-NH2 | (SEQ ID NO.: 49, JM#114) |
| ILRWSRK-NH2 | (SEQ ID NO.: 50, JM#118) |
| ILRWSRK(Glu-Pal)LPCVS | (SEQ ID NO.: 54, JM#143) |
| ILRWSRKLPCK(Glu-Pal)S | (SEQ ID NO.: 56, JM#145) |
| IYRWSRKMPCLS | (SEQ ID NO.: 57, JM#146) |
| ILRWSRK(Glu-Pal)MPCLS | (SEQ ID NO.: 58, JM#148) |
| IVRWSKKVPSVS | (SEQ ID NO.: 60, JM#151) |
| IVRWSK(Pal)K-NH2 | (SEQ ID NO.: 61, JM#164) |
| IVRWSKK(Pal)-NH2 | (SEQ ID NO.: 62, JM#165) |
| IVRWSK(Pal)KVPCVS | (SEQ ID NO.: 65, JM#168) |
| d-ILRWSRK-NH2 | (SEQ ID NO.: 70, JM#173) |
| d-ILRWSRKLP-NH2 | (SEQ ID NO.: 71, JM#174) |
| d-ILRWSRK(Pal)LP-NH2 | (SEQ ID NO.: 72, JM#175) |
| d-LMRWSRK(Pal)MPCVS | (SEQ ID NO.: 73, JM#176) |
| Md-LRWSRK(Pal)LPCVS | (SEQ ID NO.: 74, JM#177) |
| Md-LRWSRK(Pal)LP-NH2 | (SEQ ID NO.: 76, JM#179) |
| Md-LRWSRK(Pal)-NH2 | (SEQ ID NO.: 78, JM#181) |
| ILRWSRK(Ste)LPCVS | (SEQ ID NO.: 79, JM#182) |
| ILRWSRK(Chl)LPCVS | (SEQ ID NO.: 81, JM#184) |
| Ac-ILRWSRKLPCVS | (SEQ ID NO.: 82, JM#185) |
| d-Ac-ILRWSRKLPCVS | (SEQ ID NO.: 83, JM#186) |
| Ac-MLRWSRKLPCVS | (SEQ ID NO.: 84, JM#187) |
| d-Ac-MLRWSRKLPCVS | (SEQ ID NO.: 85, JM#188) |
| VLRWSRKLPCVS | (SEQ ID NO.: 86, JM#189) |
| d-VLRWSRKLPCVS | (SEQ ID NO.: 87, JM#190) |
| d-MLRWSRK(Glu-Pal)-NH2 | (SEQ ID NO.: 93, JM#196) |
| d-MLRWSRKK(Glu-Pat)-NH2 | (SEQ ID NO.: 94, JM#197) |
| ILRWSRK(Glu-Ste)LPCVS | (SEQ ID NO.: 95, JM#198) |
| ILRWSRK(Glu-Myr)LPCVS | (SEQ ID NO.: 99, JM#204) |

- Group 6 consists of

| | |
|---|---|
| d-LMRWSRKK(Glu-Pal)-NH2 | (SEQ ID NO.: 92, JM#195) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197) |
| ILRWSRK(Glu-Ste)LPCVS | (SEQ ID NO.: 95, JM#198) |

- Group 7 consists of

| | |
|---|---|
| Md-LRWSRKLPCVS | (SEQ ID NO.: 45, JM#43) |
| Md-LRWSRKMPCVS | (SEQ ID NO.: 46, JM#44) |
| ILRWSRK(Glu-Pal)LPCVS | (SEQ ID NO.: 54, JM#143) |
| ILRWSRK(Pal)LPCVS | (SEQ ID NO.: 55, JM#144) |
| ILRWSRKLPCK(Glu-Pal)S | (SEQ ID NO.: 56, JM#145) |
| ILRWSRK(Pal)MPCLS | (SEQ ID NO.: 59, JM#149) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| d-ILRWSRK-NH2 | (SEQ ID NO.: 70, JM#173) |
| d-ILRWSRKLP-NH2 | (SEQ ID NO.: 71, JM#174) |

- Group 8 consists of

| | |
|---|---|
| d-LLRWSRKMPCVS | (SEQ ID NO.: 31, JM#29) |
| ILRWSRK(Pal)L-NH2 | (SEQ ID NO.: 69, JM#172) |
| ILRWSRK(Glu-Pal)-NH2 | (SEQ ID NO.: 89, JM#192) |

- Group 9 consists of

| | |
|---|---|
| Ac-SLRWSRKMPCVS | (SEQ ID NO.: 27, JM#25) |
| d-MLRWSRKMPCVS | (SEQ ID NO.: 30, JM#28) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-LLRWSRK(Glu-Pal)MPCVS | (SEQ ID NO.: 52, JM#140) |
| d-LMRWSRK(Pal)MP-NH2 | (SEQ ID NO.: 75, JM#178) |

- Group 10 consists of

| | |
|---|---|
| ILRWSRK(Pal)LPCVS | (SEQ ID NO.: 55, JM#144) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRK(Pal)L-NH2 | (SEQ ID NO.: 69, JM#172) |
| d-LMRWSRK(Pal)-NH2 | (SEQ ID NO.: 77, JM#180) |
| ILRWSRKK(Glu-Pal)-NH2 | (SEQ ID NO.: 88, JM#191) |
| ILRWSRK(Glu-Pal)-NH2 | (SEQ ID NO.: 89, JM#192) |
| ILRWSRKLK(Glu-Pal)-NH2 | (SEQ ID NO.: 90, JM#193) |
| d-LMRWSRK(Glu-Pal)-NH2 | (SEQ ID NO.: 91, JM#194) |

wherein d- in front of an amino acid indicates a D-Amino acid, (Pal) indicates a palmitoylation of the preceding amino acid, (Glu-Pal) indicates a palmitoylation of the preceding amino acid with a glutamate linker, Dec indicates decanoic acid, (Glu-Dec) indicates a decanoic acid on the preceding amino acid with a glutamate linker, Myr indicates myristic acid, (Glu-Myr) indicates a myristic acid on the preceding amino acid with a glutamate linker, Ole indicates oleic acid, Ste indicates stearic acid, (Glu-Ste) indicates a stearic acid on the preceding amino acid with a glutamate linker, Chl indicates Cholesterol, Ac indicates a substitution of an amino group by an acetyl group,
wherein the peptide is coupled to a polymer.

Polymer-coupled peptides have been shown to have a higher relative stability in human plasma as well as a longer biological availability. Polymers change the physical and chemical properties of the coupled peptide, e.g. hydrophilic properties and, consequently, its size, which inhibits the renal excretion of the peptide. Furthermore, the coupled polymers envelope the peptides, protecting them advantageously from degradation by protease and antibody activity. The peptide activity is enhanced by the coupled polymers.

In a preferred embodiment, the polymer-coupled peptide is coupled to a further peptide. The dimerization of the polymer-coupled peptides is further enhancing their activity.

A preferred polymer is polyethylene glycol (PEG). With other words, the peptide is preferably coupled to PEG.

An also preferred polymer is a poly(vinyl alcohol) (PVA). With other words, the peptide is preferably coupled to a poly(vinyl alcohol). PVA provides an alternative to PEG in case a patient has developed anti-PEG antibodies.

An also preferred polymer is a poly(vinyl pyrrolidone) (PVP). With other words, the peptide is preferably coupled to a poly(vinyl pyrrolidone). PVA provides a further alternative to PEG in case a patient has developed anti-PEG antibodies.

The coupled polymers have a suitable molecular weight, for example between 5 and 20 kDa. Other molecular weights are possible if necessary, depending on the application. In inhibiting HIV-1 infection, the 20kDa variant was more active than the 5 kDa variant, while in antibody competition they showed similar activity.

In a preferred embodiment, the coupled polymers may be coupled to two copies of identical monomeric peptides. Coupling of two different monomeric peptides is also possible. It is preferred if the peptide copies are coupled on one end of the polymer. It is also possible that the peptide copies are coupled to different ends of the polymer (telechelic peptide conjugates). It has been shown that polymers with peptide copies on one end (SC066) have a higher activity than polymers with peptide copies are coupled to different ends of the polymer (SC029).

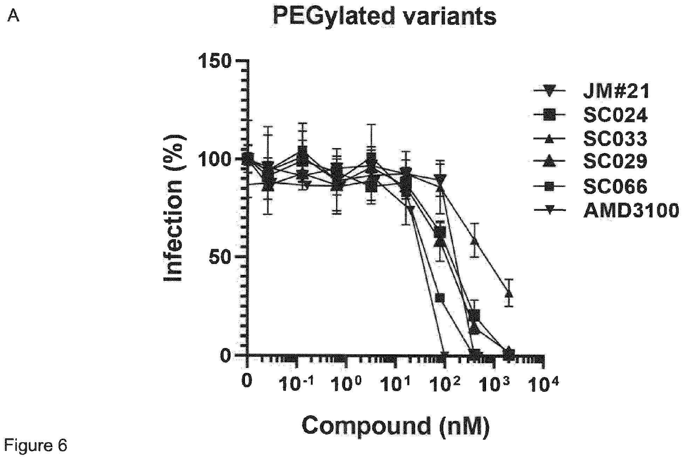

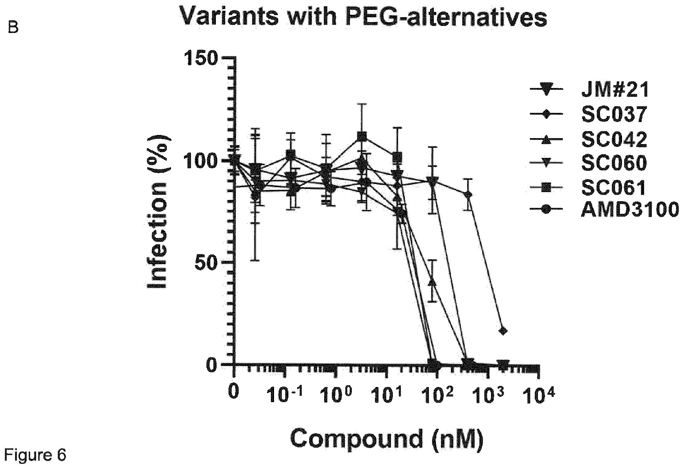

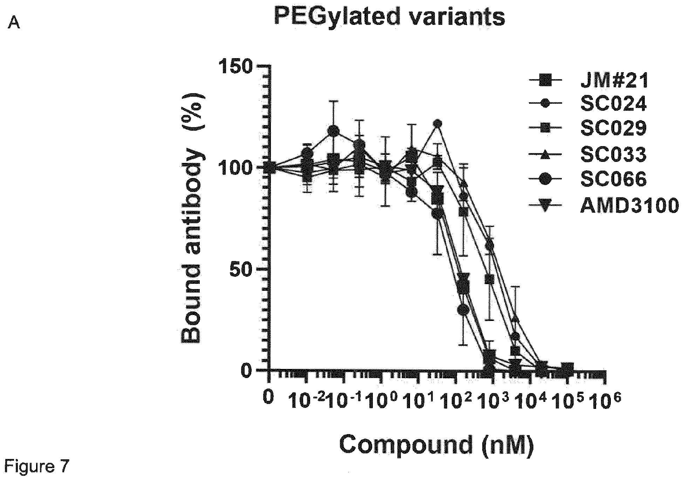

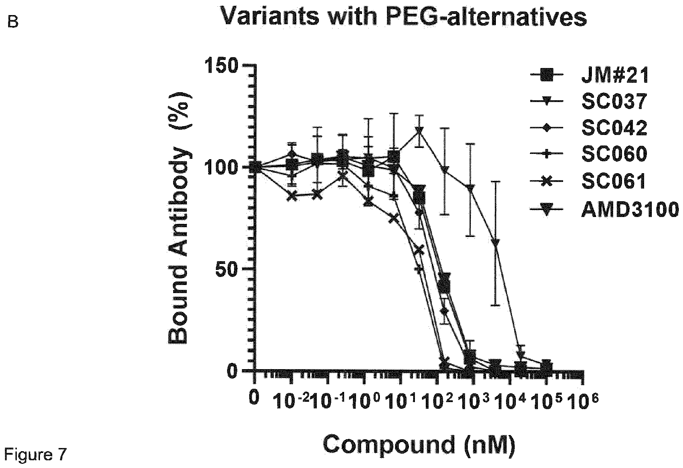

The effect of the polymer-coupled variants is illustrated in Fig. 6 and Fig. 7. The polymers were coupled to peptide JM#21 (SEQ ID NO.: 23). The PEG-coupled peptides SC024 (average molecular mass 20 kDa), SC033 (average molecular mass 5 kDa), SC029 (average molecular mass 20 kDa), and SC066 (average molecular mass 20 kDa) show an activity estimated by blocking HIV1 infection which is comparable to the state of the art CXCR4-antagonist AMD3100 and JM#21 (Fig. 6A). The same is true for the antibody competition assay wherein the PEG-coupled derivatives bound with a similar affinity to CXCR4 as JM#21 and AMD3100 (Fig. 7A). It has been shown that polymers with two peptide copies on one end (SC066) have a higher activity than polymers with peptide copies are coupled to different ends of the polymer (SC029). In the antibody competition assay, the derivative SC066 (two peptide copies on one end of PEG) showed an even higher activity than JM#21 and AMD3100. For each peptide and assay, three independent experiments (Fig. 6) or two independent experiments (Fig. 7) were carried out. The error bars refer to the standard deviation.

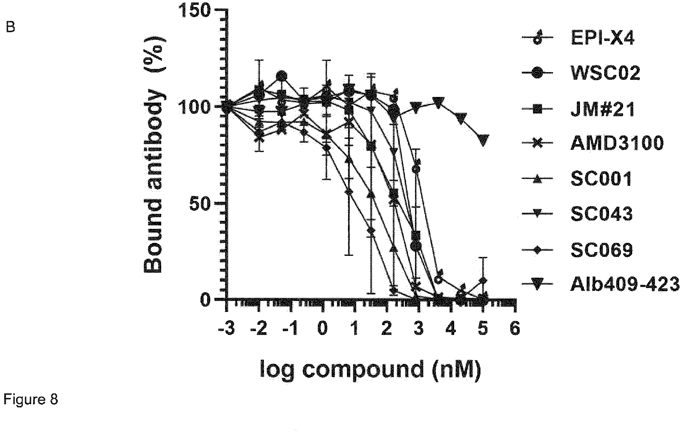

The activities of the PVP-coupled peptides SC037 (average molecular mass 20 kDa) and SC060 (two peptides on one end, average molecular mass 20 kDa) as well as of the PVA-coupled peptides SC042 (average molecular mass 20 kDa) and SC061 (two peptides on one end, average molecular mass 20 kDa) are shown in Figs. 8B and 9B. All derivatives had a comparable activity as JM#21 and AMD3100, wherein the derivatives SC060 and SC061, harboring two peptide copies on one end of PVP and PVA, respectively, showed an even higher activity. For each peptide, three independent experiments were carried out. The error bars refer to the standard deviation.

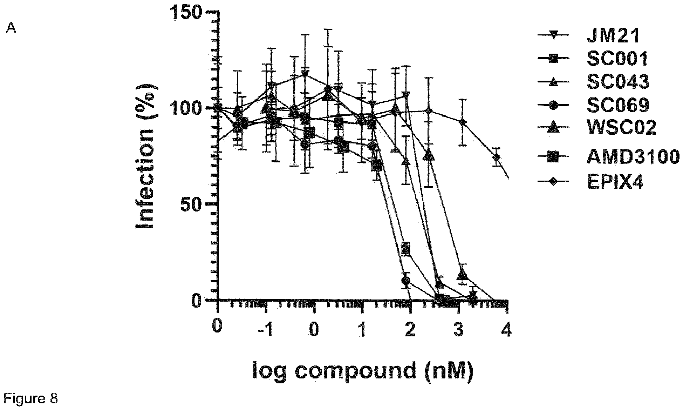

In a further preferred embodiment, the PEG-coupled peptide is coupled to 1,2-distearoyl-sn-glycero-3-phosphoethanolamine (DSPE). DSPE is a phospholipid which has been shown to increase the relative stability of the peptides in human plasma as well as the biological availability. DSPE is coupled to the peptide via PEG, so it has the formula 1,2-distearoyl-sn-glycero-3-phosphoethanolamine-N-[amino(polyethylene glycol)-2000]. It is also preferred if the DSPE is coupled to a further peptide (via PEG). In Fig. 8, it can be seen that DSPE-coupled derivative SC001 (one peptide copy) and SC069 (two peptide copies on one end) have a higher activity as cholesterol-coupled SC043, JM#21 (SEQ ID NO.: 23), EPI-X4 (SEQ ID NO.: 1), WSC02 (SEQ ID NO.: 2), AMD3100 and Albumin fragment Alb409-423, as shown by an HIV-inhibition assay (Fig. 8A) and an antibody competition assay (Fig. 8B). The derivative with two peptide copies on one end (SC069) was shown to have a higher activity than the derivative with one peptide copy (SC001).

A fourth aspect of the invention is related to a peptide consisting of two identical monomeric peptides according to any of the preceding claims, wherein the monomeric peptides are linked to each other via a cysteine bridge which is formed between the monomeric peptides to form a dimeric peptide. Dimeric peptides consisting of two different monomeric peptides are also possible, though dimeric peptides consisting of two identical monomeric peptides are more effective. The dimeric peptides show a higher activity compared to a double amount of the respective monomeric peptides (both versions are disclosed already in EP3007717).

A fifth aspect of the invention is related to the inventive peptide or the inventive pharmaceutical composition for use in medicine.

A sixth aspect of the invention is related to a pharmaceutical composition comprising the inventive peptide together with at least one pharmaceutically acceptable carrier, mesoporous nanoparticles, cryoprotectant, lyoprotectant, excipient and/or diluent. The pharmaceutical composition may further comprise binders, disintegrates, glidents, lubricants, coloring agents, sweetening agents, flavoring agents, preservatives, and/or the like. Ingredients are selected for their use in specific applications. Mesoporous nanoparticles, for example, are advantageous for a sustained release of the peptides.

A seventh aspect of the invention is related to the use of the peptide or the pharmaceutical composition according to any of the preceding claims for the preparation of a formulation for oral administration, inhalation, intravenous administration, topical administration, intranasal, intraperitoneal administration, subcutaneous administration and/or any other injectable form. The pharmaceutical composition may be administered, for example, in the form of liquid formulations including solutions, suspensions and emulsions, and in the form of pills, tablets, film tablets, coated tablets, capsules, liposomal formulations, micro- and nano-formulations, and powders.

In a preferred embodiment, the pharmaceutical composition is prepared as a lyophilized formulation of a buffered liquid formulation.

An eighth aspect of the invention is related to the inventive peptide or the inventive pharmaceutical composition for use in the treatment of disorders of hematopoiesis, in particular for support of the mobilization, proliferation and migration of stem cells; in the treatment of wounds, in particular wounds caused by burning; in the treatment of viral diseases, in particular infections with HIV-1, HIV-2, Cytomegalovirus, Herpes simplex virus (type 1 and 2), Varicella zoster virus, Hepatitis A and Hepatitis B virus, Influenza virus, Polio virus, Rhino virus, Rubella virus, Measles virus, Rabies virus, Rous sarcoma virus, Epstein-Barr Virus; in the treatment of infections caused by bacteria and fungi, in particular Pseudomonas, Candida, S. aureus, in the treatment of infectious processes, abnormal infectious processes; in the treatment of inflammation, in particular of periodontal disease; in the treatment of growth disorders; in the treatment of neuronal diseases, disorders of the blood clotting cascade and hematopoiesis, vascular diseases, diseases of the immune system, for improving wound and bone healing, for use in the treatment of neurological diseases, in particular stroke, Parkinson's disease, Alzheimer's disease, multiple sclerosis; in the treatment of the Warts, Hypogammaglobulinemia, Immunodeficiency, and Myelokathexis syndrome (WHIM-syndrome) and rheumatoid arthritis; in the treatment of cancers, in particular cancers showing the CXCR4 receptor, preferably cancer of the liver, pancreas, prostate, breast cancer or other solid tumors; in the treatment of lack of mobilization, proliferation and migration of stem cells, T-cell activation as well as support of immunoblasts, preferably of cytotoxic T lymphocytes with programmed cell death receptor 1 (CTL/PD-1); in the treatment of antifibrosis; in the treatment or prevention of scars; in the treatment of cardiologic disorders, in particular heart insufficiency; in the treatment of metabolic disorders, in particular diabetes.

Experimental data support the efficiency of the claimed peptide derivates against the diseases mentioned above. At the example of JM#21 (SEQ ID NO.: 23) it could be shown that the peptides counteracts growth and migration in vitro and in vivo of Acute Myeloid Leukemia (AML) cells as well as primary patient material and Waldenström's Macroglobulinemia (WM) cells harboring different WHIM-like CXCR4 mutations. This was accompanied by intrinsic changes suppressing oncogenic MAP kinase signaling of AML and WM cells. In relation to AMS cells, JM#21 (SEQ ID NO.: 23) efficiently blocked the CXCR4 12G5 epitope of AML cells in a dose dependent manner, inhibited migration of AML cells along a CXCL12 gradient, reduced CXCL12-induced ERK phosphorylation of AML cells, and reduced engraftment potential of primary CXCR4 AML patient samples in NSG mice whereas it has no inhibiting effect on the engraftment potential of CD34+ normal cell. In relation to WM cells, JM#21 (SEQ ID NO.: 23) blocked the CXCR4 12G5 epitope of WM cells in presence or absence of different CXCR4 mutations in a dose dependent manner, impaired migration of WM cells with or without S338X mutation along a CXCL 12 gradient, and reduced CXCL12-induced ERK phosphorylation of CXCR4 mutant WM cells in a dose-dependent manner.

A ninth aspect of the invention is related to a method of prophylaxis and/or treatment of cancer, viral diseases, metabolic disorders, neurologic disorders, diseases of the immune system, and disorders of the blood clotting cascade and hematopoiesis in a mammal, including a human. The terms "prophylaxis" and "treatment" comprise administering to the mammal a pharmaceutically effective amount of the peptide or the pharmaceutical composition according to any of the preceding claims, or salts or hydrates thereof effective to treat the above mentioned conditions.

An tenth aspect of the invention is related to a method for manufacturing the inventive peptide by solid phase synthesis. If this is not possible, e.g. for peptides coupled to polymers, other methods are selected for manufacture of those derivates. In a preferred embodiment, monomeric peptides are provided and coupled under oxidative reaction conditions which are capable to oxidize SH bonds to yield -S-S-bonds.

### Experimental procedures

**HIV-1 inhibition assay.** Viral stocks of CXCR4-tropic NL4-3 were generated by transient transfection of 293T cells with proviral DNA as described (Münch et al., 2007). The next day the transfection mixture was removed and fresh medium containing 2.5 % FCS was added. 2 days after transfection the supernatant was harvested and cell debris were removed by centrifugation. Aliquots were stored at -80°C. For infection of TZM-bl cells in presence of inhibitors, cells were seeded at a density of 1 x 10^5 cells/ml in 70 µl DMEM containing 2.5 % FCS. Compounds were diluted in PBS and 10 µl were added. After 15 minutes of incubation cells were inoculated with 20 µl of diluted virus. Infection rates were determined three days later using Gal-Screen system (Applied Biosystems).

**Antibody competition assay.** Competition of compounds with antibody binding was performed on SupT1 cells. For that cells were washed in PBS containing 1 % FCS and 50,000 cells were then seeded per well in a 96 V-well plate. Buffer was removed and plates were precooled at 4°C.

Compounds were diluted in PBS and antibody (clone 12G5, APC labelled) was diluted in PBS containing 1 % FCS. The antibody was used at a concentration closed to its determined Kd. 15 µl compounds were then added to the cells and 15 µl antibody immediately afterwards. Plates were incubated at 4°C in the dark for 2 hours. Afterwards cells were washed twice with PBS containing 1 % FCS and fixed with 2 % PFA. Antibody binding was analyzed by flow cytometry (FACS CytoFLEX; Beckman Coulter®).

**Stability assay.** Whole blood was collected from healthy donors in EDTA tubes and directly used or subsequently centrifuged for 15 min at 2,500 x g to obtain plasma. Plasma from 6 donors was pooled and stored in aliquots at - 80°C. Compounds were 200-fold diluted in human plasma or whole human blood to reach final concentrations of 20 µM. The t = 0 sample was immediately taken and stored at -80°C. Plasma/compound or blood/compound mixture was then transferred to 37°C and shook at 350 rpm. At given time points samples were taken and stored at -80°C°. For measuring the functional activity of the plasma/peptide samples, the mixtures were thawed and diluted in ice cold PBS. 12G5-APC antibody competition was then performed as described before. For blood/peptide functional stability, samples were thawed and centrifuged at 14,000 rpm to remove cells and debris. The supernatant was then diluted in PBS and 12G5-antibody competition assay was performed. After the 2 hours incubation, the cells were washed and 50 µl of 1-step-Fix/Lyse solution (Thermo Fisher #00-5333-54) was added for 15 minutes at room temperature. Afterwards cells were washed again and analyzed for bound antibody.

**ERK/AKT signaling assay.** CXCL12 induced ERK and AKT phosphorylation was determined in SupT1 cells. For this, 100,000 cells were seeded per well in a 96-V well plate in 100 µl medium supplemented with 1% FCS. Cells were incubated for 2 hours at 37°C before 5 µl of compounds were added. After 15 min incubation at 37°C cells were stimulated by adding 5 µl CXCL12 diluted in PBS to reach a final concentration of 100 ng/ml. Cells were further incubated for 2 min before the reaction was stopped by adding 20 µl of 10% PFA. Cells were fixed for 15 min at 4°C before PFA was removed and cell permeabilized by adding 100 µl ice cold methanol. After 15 min at 4°C the methanol was removed, cells were washed and 30 µl primary antibody was added (phospho-p44/42 MAPK (Erk1) (Tyr204)/ (Erk2) (Tyr187) (D1H6G) mouse mAb #5726; phosphor-Akt (Ser473) (193H12) rabbit mAb #4058 Cell Signaling) for 1 hour at 4°C. After the antibody was removed and cells were washed secondary antibody was added for 30 min. Cells were washed afterwards and subsequently analyzed by flow cytometry.

**Migration assay.** Migration Assays were performed using 96-well transwell assay plates (Corning Incorporated, Kennebunk, ME, USA) with 5 µm polycarbonate filters. First, the lower chambers were filled with 235 µl assay buffer (RPMI supplemented with 0.1 % BSA) with or without 100 ng/ml CXCL12 and serial dilutions of the CXCR4-inhibiting compounds (in assay buffer). Next, 75 µL (0.5x105 cells) of Jurkat cells (in assay buffer), together with/without the compounds, were added into the upper chambers. After 4 h at 37°C (5 % CO2), 100 µL of the lower chambers were transferred to a fresh 96 V-well plate and analyzed with Cell-Titer-Glo® assay (Promega, Madison, Wl, USA). The percentage of migrated cells was calculated as described by Balabanian et al. (2005). In order to obtain the relative migration in %, the percentage of migrated cells were normalized to the CXCL12-only control.

### Ca++ signaling

For calcium measurement, 1×10 6 BCR-ABL-transformed murine bone marrow cells were incubated with 5 µg/mL of Indo-1 (Molecular Probes) and 0.5 µg/mL of pluronic F-127 (Molecular Probes) in Iscove's medium supplemented with 1% FCS (Pan Biotech) at 37°C for 45 min. Cells were then washed by centrifugation and the cell pellets were resuspended in Iscove's medium with 1% FCS and treated with the EPI-X4 derivatives (1 µM or 0.5 µM) for 10 minutes at room temperature. Cells were pre-warmed for 5 minutes at 37°C. Calcium flux was assessed by FACS measurement at BD LSR Fortessa. After 30 seconds of baseline recording, CXCR4-dependent calcium signaling was determined by stimulating with 100 ng/ml of mouse SDF-1a (PeproTech).

### Molecular modeling

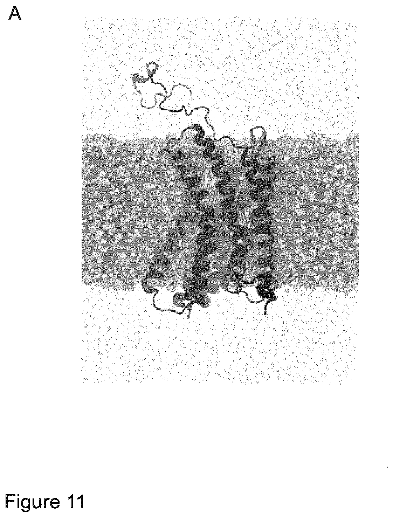

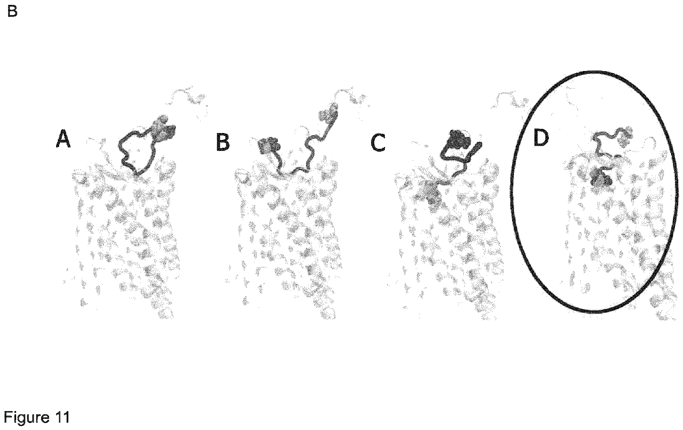

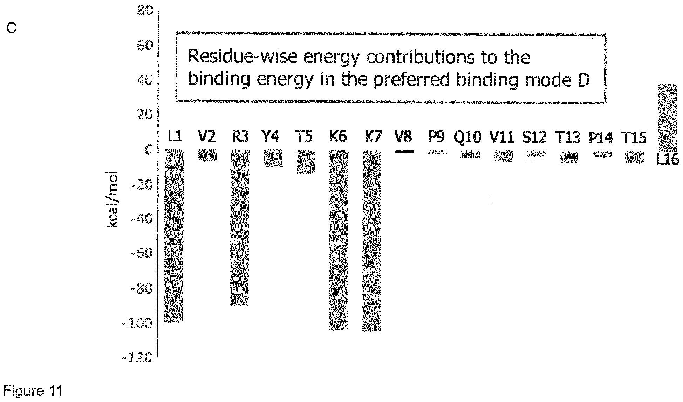

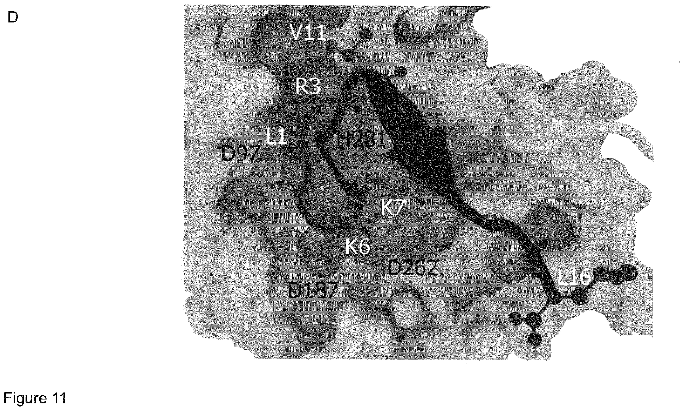

The first step for the design of enhanced EPI-X4 (SEQ ID NO.: 1) derivatives was to determine how the peptide binds to CXCR4. With this knowledge, we were able to improve ligand efficiency by designing shorter peptides that are potentially more active than EPI-X4 (SEQ ID NO.: 1). Accordingly, our computational approach comprised the following steps:
a. Building a CXCR4 model based on the reported crystal structure (2.50 Å, PDB code: 3ODU). This model also includes the highly flexible N-terminus region (available in the literature from NMR studies, PDB code 2K04).
b. Docking calculations and homology modeling for the initial exploration of EPI-X4 binding sites in CXCR4.
c. For each binding site, we built CXCR4 - EPI-X4 models in explicit solvent and lipid membrane (an example is shown in Fig. 11 A). The models consisted of 257 POPC lipids, approximately 40000 TIP3P water molecules, 50 mM KCI and the CXCR4 - EPI-X4 complex.
d. We performed atomistic molecular dynamics simulations (MD) of each model to analyze factors like ligand flexibility, interaction interface area, solvent accessible surface and hydrogen bonding interactions in the different binding modes. The analysis of all these parameters indicated that D is the preferred binding motif. In D, the N-terminus of EPI-X4 is inserted in CXCR4 while the C-terminus of the peptide is solvent-exposed (Fig. 11 B).
e. Based on the MD simulations, we performed an energetic analysis of the electrostatic and van der Waals contributions to the interaction energy in each binding motif as well as the contribution to the interaction energy of individual residues of EPI-X4 (Fig. 11 C).
f. We also performed extensive coarse-grained (CG) MD simulations to investigate the self-assembly of the CXCR4 - EPI-X4 complex from the unbound state, using non-equilibrium dynamics. With these CG simulations, we further established D as the most favored mode, as predicted by the atomistic MDs (Fig. 11D).

With the information of how EPI-X4 (SEQ ID NO.: 1) binds to CXCR4 and the individual contribution of each residue of the peptide to the binding, we designed shortened peptide derivatives with neutral C-terminus that we predict to be more efficient than EPI-X4 (SEQ ID NO.: 1). A set of peptides was thus identified and their experimental activity assessed.

**Toxicity in Zebrafish.** In order to test for toxic effects in zebrafish, fish embryos without chorion (24 hours post fertilization) were exposed to the compounds for 24 hours and then assayed in a stereomicroscope. Each assay was done for 3 x 10 embryos (in 100 µl) per concentration in duplicates (total n = 60). As negative control peptide solvent at the highest concentration was used. As a positive control for acute toxicity pleurocidin antimicrobial peptide NRC-03 (GRRKRKWLRRIGKGVKIIGGAALDHL-NH2) at a concentration of 6 µM was used.

**Peptide synthesis. Materials** 1,2-distearoyl-sn-glycero-3-phosphoethanolamine-N-[maleimide(polyethylene glycol) 2000 Da] (ammonium salt) (cat. No. PG2-DSML-2k) and cholesterol - poly (ethylene glycol) maleimide 5000 Da (PG2-CSML-5k) were purchased from Nanocs Inc. (New York, USA). Methoxy poly (ethylene glycol) 20 kDa maleimide (cat. No. PJK-231) and di-maleimide poly (ethylene glycol) 20 kDa (cat. No. PSB-305) were purchased from Creative PEGWorks (North Carolina, USA). All other chemicals were purchased from commercial vendors (Sigma-Aldrich, Acros, TCl). Deuterated solvents were supplied from Euriso-Top. Dichloromethane (CH2Cl2), acetonitrile (MeCN), tetrahydrofuran (THF) and toluene (PhMe) were acquired through a MBraun SPS-800 solvent purification system. Ultrapure water was dispensed from MilliQ Direct 8 (Millipore) [18.2 MΩ • cm].

**¹H-NMR Spectroscopy** Nuclear magnetic resonance (NMR) spectra were recorded on a Varian Mercury 400 MHz spectrometer, running at 400 MHz. Chemical shifts (δ) are reported in ppm relative to the residual solvent

**Sodium Dodecyl Sulfate** - **Polyacrylamide Gel Electrophoresis (SDS PAGE)** SDS-PAGE analysis was conducted with a NuPAGE® bis-tris 4-12 % precast gel (Invitrogen) in an electrophoresis tank. Samples were prepared with NuPAGE® LDS sample buffer (4×). Samples volumes loaded were typically 10 µL. The running buffer was NuPAGE® MOPS SDS running buffer (20×). The voltage applied for electrophoresis was 150 V and the run time was 1 hour. The SDS PAGE gel was first stained with Coomassie blue stain for 30 minutes, then washed with distilled water for 1 hour.

**Preparative Reverse-Phased High-Performance Liquid Chromatography (Prep RP-HPLC)** RP-HPLC was conducted using C₁₈DiscoveryBIO Wide Pore column (10 µm, 150 × 10 mm) with 5% acetonitrile containing 0.01% trifluoracetic acid (TFA) as solvent A and 100% acetonitrile containing 0.01% TFA as solvent B. All solvents used were of HPLC grade. The gradient used was 30 to 60% solvent B in 25 minutes. Flow rate of 2 mL/min and UV detection at 280 nm was used for analysis.

**Azeotropic distillation of PEG compounds** Typically, to a flame-dried 50 mL schlenk flask fitted with a rubber septum and magnetic stir bar was added poly (ethylene glycol) (PEG) (0.05 - 0.1 g). Anhydrous toluene (5 mL) was injected into the flask using a clean glass syringe and needle. The flask was gently warmed to dissolve the PEG in toluene. The stoppered side arm of the schlenk flask was connected to a vacuum oil pump fitted with an ice trap. Toluene was observed to slowly foam upon slow opening of the side arm stopper to vacuum. The flask was gently swirled to avoid spluttering of the mixture. Moisture formed on the outer walls of the flask was wiped until all the solvent was removed from the flask. The flask was allowed to remain in vacuum for further 30 min at room temperature.

### Reagent Synthesis

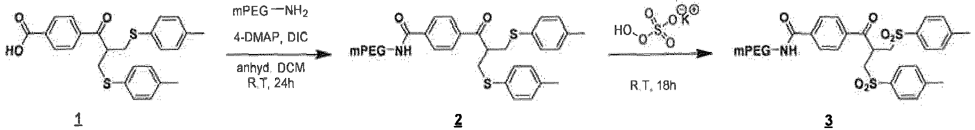

**Synthesis of 4-(3-(p-tolylthio)-2-((p-tolylthio)methyl)propanoyl)benzoic acid.** 4-(3-(p-Tolylthio)-2-((p-tolylthio)methyl)propanoyl)benzoic acid **1** was prepared as described previously in literature¹ (1.6 g, 72.7%). **¹H-NMR** (CDCl₃): 2.38 (s, 6H), 3.16-3.31 (m, 4H), 3.85 (q, 1H), 7.15 (d, 4H), 7.18 (d, 4H), 7.64 (d, 2H), 8.07 (d, 2H)

**Synthesis of *bis*-sulfide PEG 20 kDa.** Toluene-dried methoxy poly (ethylene glycol) amine (mPEG-NH₂, 20 000 g/mol, 100 mg, 1 equiv, 5.1 µmol) and 4-dimethylaminopyridine (0.06 mg, 0.1 equiv., 0.5 µmol) were dissolved in anhydrous dichloromethane (3 mL) under argon atmosphere. A mixture of 4-(3-(p-tolylthio)-2-((p-tolylthio)methyl)propanoyl)benzoic acid 1 (8.73 mg, 4 equiv, 20 µmol) and N,N'-diisopropylcarbodiimide (3.12 µL, 4 equiv, 200 µmol) in anhydrous dichloromethane (2 mL) was added drop-wise to the initial PEG solution under argon atmosphere. The reaction mixture was left to stir at room temperature for 24 h. After this time, dichloromethane was removed from the filtrate by roto-evaporation, and the viscous crude product residue was re-dissolved in acetone with gentle warming. The flask was then placed in a dry ice bath to precipitate the product, which was isolated by centrifugation dried *in vacuo* to afford PEG *bis*-sulfide **2** as a white solid product (0.101 g, 98.2%). ¹H NMR: (CDCl₃, 400 MHz) δ 2.49 (s, 6H), 3.38 (s, 3H), 3.44-3.84 (m, PEG + 4H), 4.34 CHCO (qn, 1H), 7.36, 7.69 (q, 4H), 7.64, 7.81 (q, 4H).

**Sulfide oxidation and synthesis of bis-sulfone PEG 20 kDa.** *Bis*-sufide PEG 20 kDa **2** (50 mg, 1 equiv., 2.5 µmol) and potassium peroxymonosulfate Oxone® (3.08 mg, 4 equiv., 10 µmol) were dissolved in an aqueous solution of 50 % methanol (3 mL). The reaction mixture was stirred over-night at room temperature. After this time, volatiles were removed by rotary evaporation and purification achieved by acetone/dry-ice precipitation as described previously. The solid obtained was dried in desiccator to afford *bis*-sulfone PEG **3** as a white, fluffy solid (24 mg, 48 %). **¹H-NMR** (CDCl₃): 2.38 (s, 6H), 3.16-3.31 (m, 4H), 3.85 (q, 1H), 7.15 (d, 4H), 7.18 (d, 4H), 7.64 (d, 2H), 8.07 (d, 2H);

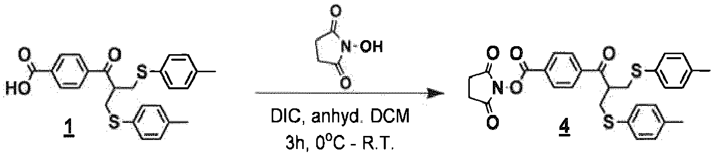

**Synthesis of NHS-activated *bis*-sulfide 4.** Under an argon atmosphere, a mixture of 4-(2,2-bis[(p-tolylsulfonyl)methyl]acetyl) benzoic acid **1** (0.5 g, 1 equiv., 1.15 mmol), N-hydroxysuccinimide (0.139 g, 1.05 equiv., 1.21 mmol) and anhydrous dichloromethane (5 mL) were cooled using an ice bath. Neat 1,3-diisopropylcarbodiimide (188 µL, 1.05 equiv., 1.21 mmol) was then added dropwise. A further 20 µL of DIC was added after 1.5 h. After 3 h, the reaction mixture was passed through a non-absorbent cotton wool filter. The homogeneous filtrate was diluted with dichloromethane, washed twice with water, and dried over magnesium sulphate. Gravity filtration followed by removal of volatiles under vacuum gave the desired active NHS ester **4** as a solid product (0.39 g, 78% yield). ¹H-NMR (CDCl₃): 2.35 (s, 6H), 2.94 (s, 4), 3.16-3.25 (dd, 4H), 3.80 (q, 1H), 7.05 (d, 4H), 7.10 (d, 4H), 7.60 (d, 2H), 8.05 (d, 2H);

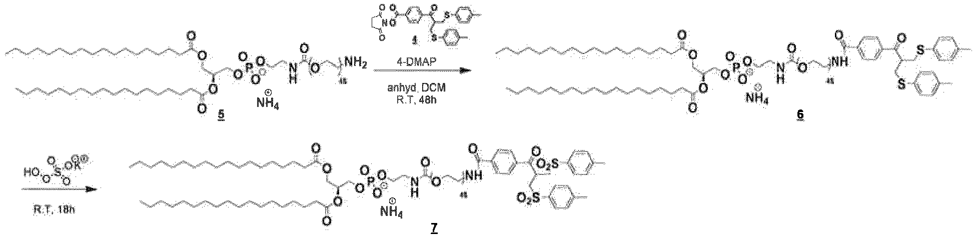

**Synthesis of DSPE-PEG-*bis*-sulfide 2 kDa.** Toluene-dried 1,2-distearoyl-sn-glycero-3-phosphoethanolamine-N-[amino(polyethylene glycol) 2kDa] (ammonium salt) **5** (DSPE-PEG-NH₂, 2 000 g/mol, 50 mg, 1 equiv, 25 µmol), NHS activated *bis*-sulfide **4** (53.4 mg, 4 equiv., 100 µmol), and 4-dimethylaminopyridine (0.3 mg, 0.1 equiv, 2.5 µmol) were dissolved in anhydrous dichloromethane (5 mL) under argon atmosphere. The reaction mixture was left to stir at room temperature for 48 h. After this time, dichloromethane was removed from the filtrate by rotary evaporation, and the viscous crude product residue was re-dissolved in acetone with gentle warming. The flask was then placed in a dry ice bath to precipitate the product, which was isolated by centrifugation dried *in vacuo* to afford DSPE-PEG bis-sulfide **6** as a white solid (26.5 mg, 42.4%). ¹H NMR: (CDCl₃, 400 MHz) δ 2.35 (s, 6H), 3.39-3.84 (m, PEG), 4.27 (br, 1H), 7.05 (d, 4H), 7.10 (d, 4H), 7.60 (d, 2H), 8.05 (d, 2H)

**Sulfide oxidation to bis-sulfone DSPE-PEG 2 kDa.** *Bis*-sulfide DSPE-PEG 2 kDa **5** (26.5 mg, 1 equiv., 8.28 µmol) and potassium peroxymonosulfate Oxone® (10.2 mg, 4 equiv., 33.1 µmol) were dissolved in an aqueous solution of 50 % methanol (3 mL). The reaction mixture was stirred over-night at room temperature. After this time, volatiles were removed by rotary evaporation and purification achieved by acetone/dry-ice precipitation as described previously. The solid obtained was dried in desiccator to afford the *bis-*sulfone DSPE PEG **6** as a white solid (16.3 mg, 56.2 %). ¹H-NMR (CDCl₃): 2.49 (s, 6H), 3.38-3.80 (m, PEG), 4.27 (m, 2H), 7.36 (d, 4H), 7.63 (d, 2H), 7.70 (d, 4H), 7.80 (d, 2H);

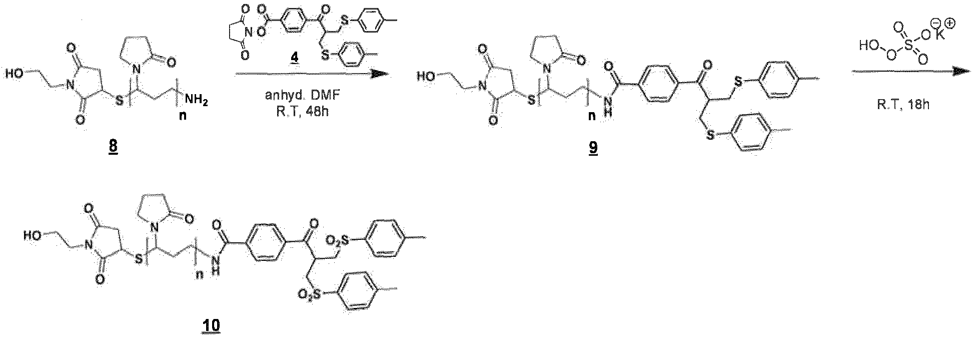

**Synthesis of bis-sulfide PVP 20 kDa.** Amine-terminated polyvinylpyrrolidone (PVP-NH₂, 23 800 g/mol, 100 mg, 1 equiv, 4.2 µmol) and NHS-activated *bis*-sulfide (8.97 mg, 4 equiv, 16.8 µmol) were dissolved in anhydrous dimethylformamide (DMF, 2 mL) under argon atmosphere. The reaction mixture was stirred at room temperature for 48 h. After this time, the product was precipitated in ethyl ether and isolated by centrifugation. The obtained white precipitate was diluted in MQ water and lyophilized to afford *bis*-sulfide PVP as a white solid (66.5 mg, 66.5%). ¹H NMR: (CDCl₃, 400 MHz) 1.72 (br, 2H, PVP), 2.06 (br, 2H, PVP),), 2.39 (br, 2H, PVP), 3.20 (br, 2H, PVP), 3.73 (br, 1H, PVP), 7.04 (d, 4H), 7.08 (d, 4H), 7.5 - 7.6 (br, 4H)

**Sulfide oxidation to bis-sulfone PVP 20 kDa.** *Bis*-sulfide PVP 20 kDa 9 (66.5 mg, 1 equiv., 2.74 µmol) and potassium peroxymonosulfate Oxone® (5.07 mg, 4 equiv., 11 µmol) were dissolved in an aqueous solution of 50 % methanol (3 mL). The reaction mixture was stirred over-night at room temperature. After this time, volatiles were removed by rotary evaporation and purification achieved by DMF/ethyl ether precipitation as described previously. The solid obtained was dried in desiccator to afford the *bis-*sulfone PVP as a white solid (31.85 mg, 47.7 %). ¹H NMR: (CDCl₃, 400 MHz) 1.72 (br, 2H, PVP), 2.06 (br, 2H, PVP),), 2.39 (br, 2H, PVP + 6H from *bis*-sulfone), 3.20 (br, 2H, PVP), 3.73 (br, 1H, PVP), 7.36 (d, 4H), 7.63 (d, 2H), 7.70 (d, 4H), 7.81 (d, 2H)

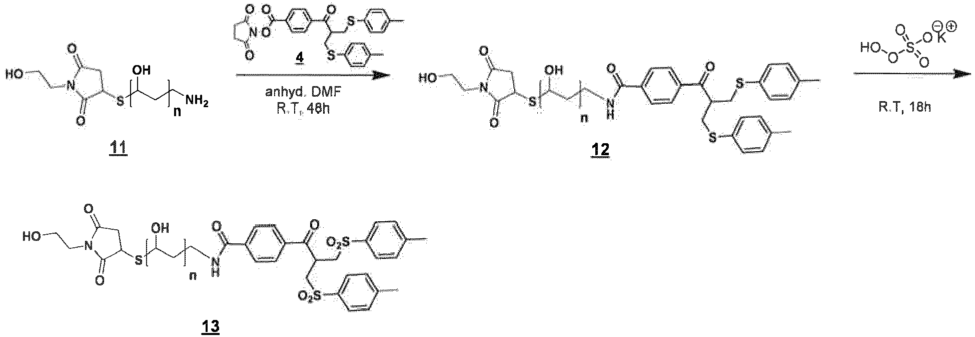

**Synthesis of bis-sulfide PVA 20 kDa.** Amine-terminated poly (vinyl alcohol) (PVA-NH₂, 19 800 g/mol, 100 mg, 1 equiv, 4.2 µmol) was dissolved in DMSO (1 mL) and heated to 60 °C until complete dissolution. The solution was allowed to cool to room temperature and NHS-activated *bis*-sulfide (8.97 mg, 4 equiv, 16.8 µmol) in DMSO (1 mL) was added under argon atmosphere. The reaction mixture was stirred at room temperature for 48 h. After this time, the product was precipitated in heptane and isolated by centrifugation. The obtained white precipitate was diluted in MQ water and lyophilized to afford PVA *bis*-sulfide as a white solid (78.2 mg, 91.7%). ¹H NMR: (DMSO-d₆, 400 MHz) δ 1.36 (br, 2H, PVA), 3.81 (br, 1H, PVA), 7.04 - 7.10 (br, 8H), 7.5 - 7.6 (br, 4H)

**Sulfide oxidation to bis-sulfone PVA 20 kDa.** *Bis*-sulfide PVA 20 kDa (78.2 mg, 1 equiv., 3.85 µmol) and potassium peroxymonosulfate Oxone® (4.74 mg, 4 equiv., 15.4 µmol) were dissolved in an aqueous solution of 50 % methanol (3 mL). The reaction mixture was stirred over-night at room temperature. After this time, volatiles were removed by rotary evaporation and purification achieved by DMSO/heptane precipitation as described previously. The solid obtained was dried in desiccator to afford the *bis-*sulfone PVA as a white solid (54.6 mg, 69.5 %). ¹H NMR: (DMSO-d₆, 400 MHz) δ 1.36 (br, 2H, PVA), 3.81 (br, 1H, PVA), 7.34 (d, 4H), 7.62 (d, 2H), 7.70 (d, 4H), 7.80 (d, 2H)

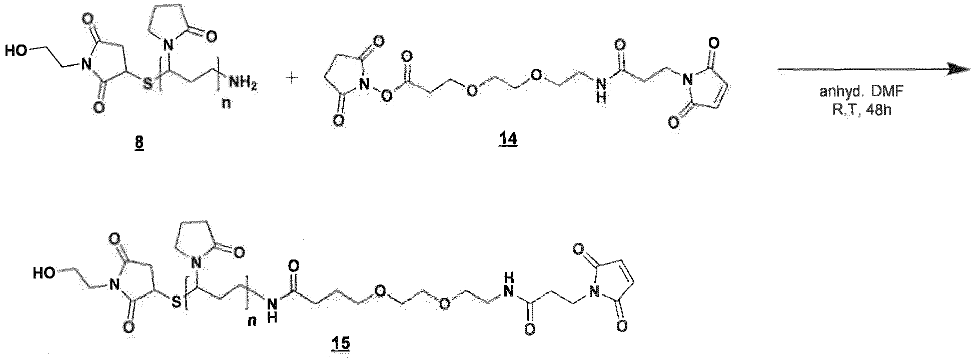

**Synthesis of maleimide PVP 20 kDa.** Amine-terminated polyvinylpyrrolidone (PVP-NH₂, 23 800 g/mol, 50 mg, 1 equiv, 2.1 µmol) and maleimide-PEG₂-succinimidyl ester (3.57 mg, 4 equiv, .8 µmol) were dissolved in anhydrous dimethylformamide (DMF, 2 mL) under argon atmosphere. The reaction mixture was stirred at room temperature for 48 h. After this time, the product was precipitated in ethyl ether and isolated by centrifugation. The obtained white precipitate was diluted in MQ water and lyophilized to afford PVP maleimide as a white solid (23.6 mg, 47.2%). ¹H NMR: (CDCl₃, 400 MHz) 1.72 (br, 2H, PVP), 1.99 (br, 2H, PVP),), 2.38 (br, 2H, PVP), 3.20 (br, 2H, PVP), 3.73 (br, 1H, PVP), 8.02 (s, 2H).

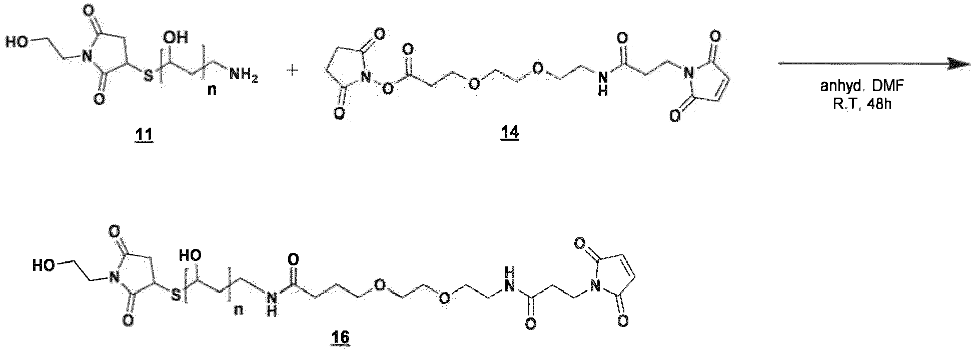

**Synthesis of maleimide PVA 20 kDa.** Amine-terminated polyvinylalcohol (PVÁ-NH₂, 19 800 g/mol, 100 mg, 1 equiv, 5.05 µmol) was dissolved in anhydrous DMSO (2 mL) and heated to 60 °C. After complete dissolution, maleimide-PEG₂-succinimidyl ester (8.59 mg, 4 equiv, .20.2 µmol) was added and the mixture was stirred at room temperature for 48 h. After this time, the product was precipitated in heptane and isolated by centrifugation. The obtained white precipitate was diluted in MQ water and lyophilized to afford PVA maleimide as a white solid (88.7 mg, 86.8%). ¹H NMR: (DMSO-d₆, 400 MHz) δ 1.36 (br, 2H, PVA), 3.81 (br, 1H, PVA), 6.99 (s, 2H, maleimide), 8.0 (br, 2H, -NH)

### Peptide Bioconjugation

**General procedure for maleimide mono-conjugations.** Native peptide (1 mg, 1 equiv., 0.714 µmol) was dissolved in 0.5 mL phosphate buffer saline, pH 7.4 (10 mM phosphate, 150 mM sodium chloride). To the peptide solution was added one equivalent of the respective maleimide conjugation reagent dissolved in 0.5 mL of PBS buffer, with a final peptide concentration of 1 mg/mL. The mixture was incubated for 4 h at room temperature. After this time, the bioconjugate was isolated from the native peptide by preparative RP-HPLC or gel filtration. The collected fractions were analysed by UV-Vis spectroscopy at 280 nm to determine the presence of peptide and combined respectively. After freeze-drying, the peptide conjugate was typically obtained as a solid.

**Table 1. Quantities and purification methods for maleimide mono-conjugation reactions**

| **Peptide Conjugate** | **Conjugation Reagent** | **Mass of Reagent (mg)** | **Purification method** |
|---|---|---|---|
| DSPE-peptide | DSPE-PEG₂₀₀₀-maleimide | 2.15 | Prep RP-HPLC |
| Cholesterol-peptide | Cholesterol-PEG₅₀₀₀-maleimide | 3.57 | LH20 gel filtration |
| mPEG 5 kDa-peptide | mPEG₅₀₀₀-maleimide | 3.57 | |
| mPEG 20 kDa-peptide | mPEG₂₀₀₀₀-maleimide | 14.8 | |
| PVP 20 kDa-peptide | PVP₂₀₀₀₀-maleimide | 17.4 | |
| PVA 20 kDa-peptide | PVA₂₀₀₀₀-maleimide | 14.3 | |

**General procedure for maleimide di-conjugation.** Native peptide (10 mg, 1 equiv., 7.14 µmol) was dissolved in 5 mL phosphate buffer saline, pH 7.4 (10 mM phosphate, 150 mM sodium chloride). To the peptide solution was added di-maleimide PEG 20 kDa (81.4 mg, 0.5 equiv., 3.57 µmol) dissolved in 5 mL of PBS buffer. The mixture was incubated for 4 h at room temperature. After this time, the bioconjugate was isolated from the native peptide by LH20 gel filtration with ACN / MQ water as the eluent. The collected fractions were analysed by UV-Vis spectroscopy at 280 nm to determine the presence of peptide and combined respectively. After freeze-drying, the peptide conjugate was obtained as a solid.

**General procedure for *bis*-sulfone conjugations.** To one equivalent of the respective *bis*-alkylating reagent was added excess native peptide 10 equiv.in 1 mL of 50 mM sodium phosphate buffer, pH 7.8 with 20 mM EDTA. For PVA conjugations, the reagents were first dissolved in 100 µL of DMSO and heated to 60 °C to allow for dissolution. The mixture was incubated for 48 hours at room temperature. After this time, the bioconjugate was isolated from the native peptide by gel filtration. The collected fractions were analysed by UV-Vis spectroscopy at 280 nm to determine the presence of peptide and combined respectively. After freeze-drying, the peptide conjugate was typically obtained as a solid. Peptide content was characterized by UV absorbance, SDS-PAGE and/or RP-HPLC.

**Table 2 Quantities and final peptide concentrations for bis-sulfone conjugations**

| **Peptide Conjugate** | **Conjugation Reagent** | **Reagent (mg)** | **Peptide (mg)** | **(Peptide]_{FINAL} mg/mL** |
|---|---|---|---|---|
| DSPE-*bis* (peptide) | DSPE-PEG₂₀₀₀-*bis-*sulfone | 5 | 21.8 | 10.9 |
| mPEG 20 kDa-*bis* (peptide) | mPEG₂₀₀₀₀-*bis-*sulfone | 10 | 6.87 | 3.4 |
| PVP 20 kDa-*bis* (peptide) | PVP₂₀₀₀₀-*bis*-sulfone | 8.68 | 5 | 2 |
| PVA 20 kDa-*bis* | PVA₂₀₀₀₀-*bis*-sulfone | 7.25 | 5 | 2 |
| (peptide) | | | | |

### References

Hendrix CW, Flexner C, MacFarland RT, Giandomenico C, Fuchs EJ, Redpath E, Bridger G, Henson GW, 2000, Antimicrob Agents Chemother 44: 1667-1673.
Munch J, Rajan D, Schindler M, Specht A, Rücker E, Novembre, FJ, Nerrienet, E, Müller-Trutwin, MC, Peeters, M, Hahn, BH and Kirchhoff, F, 2007, J Virol 81: 13852 - 13864.
Balabanian K, Levoye A, Klemm L, Lagane B, Hermine O, Harriague J, Baleux F, Arenzana-Seisdedos F, Bachelerie F, 2008 J Clin Invest 118: 1074 -1084.

## Claims

1. A peptide consisting of one of the following amino acid sequences, selected from any of the groups 1 to 10, wherein
- Group 1 consists of
| | |
|---|---|
| ILRWSRKMPCVS | (SEQ ID NO.: 15, JM#13) |
| IMRWSRKMPCVS | (SEQ ID NO.: 19, JM#17) |
| ILRWSRKMPCLS | (SEQ ID NO.: 20, JM#18) |
| ILRWSRKMPCMS | (SEQ ID NO.: 22, JM#20) |
| ILRWSRKLPCVS | (SEQ ID NO.: 23, JM#21) |
| ILRWSRKFPCVS | (SEQ ID NO.: 24, JM#22) |
| ILRWSRK(Pal)LPCVS | (SEQ ID NO.: 55, JM#144) |
| ILRWSRK(Glu-Pal)-NH2 | (SEQ ID NO.: 89, JM#192) |
| d-LMRWSRK(Glu-Pal)-NH2 | (SEQ ID NO.: 91, JM#194) |
- Group 2 consists of
| | |
|---|---|
| ILRWSRKMPCFS | (SEQ ID NO.: 21, JM#19) |
| ILRWSRK(Pal)L-NH2 | (SEQ ID NO.: 69, JM#172) |
| d-LMRWSRK(Pal)MP-NH2 | (SEQ ID NO.: 75, JM#178) |
| d-LMRWSRK(Pal)-NH2 | (SEQ ID NO.: 77, JM#180) |
| ILRWSRK(Ole)LPCVS | (SEQ ID NO.: 80, JM#183) |
- Group 3 consists of
| | |
|---|---|
| d-LLRWSRK(Pal)MPCVS | (SEQ ID NO.: 53, JM#141) |
| IVRWSK(Pal)KVP-NH2 | (SEQ ID NO.: 63, JM#166) |
| IVRWSKK(Pal)VP-NH2 | (SEQ ID NO.: 64, JM#167) |
| IVRWSKK(Pal)VPCVS | (SEQ ID NO.: 66, JM#169) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRKK(Glu-Pal)-NH2 | (SEQ ID NO.: 88, JM#191) |
| ILRWSRKLK(Glu-Pal)-NH2 | (SEQ ID NO.: 90, JM#193) |
| ILRWSRKK(Glu-Ste)LPCVS | (SEQ ID NO.: 96, JM#199) |
| ILRWSRK(Glu-Dec)LPCVS | (SEQ ID NO.: 98, JM#203) |
| ILRWSRK(Glu-Ste)LPCVS | (SEQ ID NO.: 100, JM#205) |
- Group 4 consists of
| | |
|---|---|
| ILRWSRKVPCVS | (SEQ ID NO.: 10, JM#8) |
| IFRWSRKVPCVS | (SEQ ID NO.: 12, JM#10) |
| MLRWSRKMPCVS | (SEQ ID NO.: 29, JM#27) |
| MMRWSRKMPCVS | (SEQ ID NO.: 36, JM#34) |
| MLRWSRKLPCVS | (SEQ ID NO.: 41, JM#39) |
| ILRWSRKLPSVS | (SEQ ID NO.: 51, JM#122) |
| d-LLRWSRK(Glu-Pal)MPCVS | (SEQ ID NO.: 52, JM#140) |
| ILRWSRK(Pal)MPCLS | (SEQ ID NO.: 59, JM#149) |
| d-LMRWSRKK(Glu-Pal)-NH2 | (SEQ ID NO.: 92, JM#195) |
| ILRWSRK-AcLPCVS | (SEQ ID NO.: 97, JM#200) |
- Group 5 consisting of
| | |
|---|---|
| ILRWSKKVPCVS | (SEQ ID NO.: 3, JM#1) |
| IFRWSKKVPCVS | (SEQ ID NO.: 4, JM#2) |
| IVRWSRKVPCVS | (SEQ ID NO.: 5, JM#3) |
| IVRWSHKVPCVS | (SEQ ID NO.: 6, JM#4) |
| IVRWSKKLPCVS | (SEQ ID NO.: 7, JM#5) |
| IVRWSKKIPCVS | (SEQ ID NO.: 8, JM#6) |
| IVRWSKKFPCVS | (SEQ ID NO.: 9, JM#7) |
| ILRWSHKVPCVS | (SEQ ID NO.: 11, JM#9) |
| IFRWSHKVPCVS | (SEQ ID NO.: 13, JM#11) |
| IVRWSKKMPCVS | (SEQ ID NO.: 14, JM#12) |
| IVRWSKKVPCd-VS | (SEQ ID NO.: 16, JM#14) |
| ILRWSRKVPCd-VS | (SEQ ID NO.: 17, JM#15) |
| IIRWSRKMPCVS | (SEQ ID NO.: 18, JM#16) |
| ILRWSRKVPSVS | (SEQ ID NO.: 25, JM#23) |
| ILRWSRKMPSVS | (SEQ ID NO.: 26, JM#24) |
| Ac-SLRWSRKMPCVS | (SEQ ID NO.: 27, JM#25) |
| d-Ac-SLRWSRKMPCVS | (SEQ ID NO.: 28, JM#26) |
| d-MLRWSRKMPCVS | (SEQ ID NO.: 30, JM#28) |
| d-LLRWSRKMPCVS | (SEQ ID NO.: 31, JM#29) |
| d-FLRWSRKMPCVS | (SEQ ID NO.: 32, JM#30) |
| GLRWSRKMPCVS | (SEQ ID NO.: 33, JM#31) |
| Ac-SMRWSRKMPCVS | (SEQ ID NO.: 34, JM#32) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-FMRWSRKMPCVS | (SEQ ID NO.: 39, JM#37) |
| d-GMRWSRKMPCVS | (SEQ ID NO.: 40, JM#38) |
| d-MLRWSRKLPCVS | (SEQ ID NO.: 42, JM#40) |
| Id-LRWSRKLPCVS | (SEQ ID NO.: 43, JM#41) |
| Id-LRWSRKMPCVS | (SEQ ID NO.: 44, JM#42) |
| Md-LRWSRKLPCVS | (SEQ ID NO.: 45, JM#43) |
| Md-LRWSRKMPCVS | (SEQ ID NO.: 46, JM#44) |
| IVRWSKKVP-NH2 | (SEQ ID NO.: 47, JM#106) |
| IVRWSKK-NH2 | (SEQ ID NO.: 48, JM#110) |
| ILRWSRKLP-NH2 | (SEQ ID NO.: 49, JM#114) |
| ILRWSRK-NH2 | (SEQ ID NO.: 50, JM#118) |
| ILRWSRK(Glu-Pal)LPCVS | (SEQ ID NO.: 54, JM#143) |
| ILRWSRKLPCK(Glu-Pal)S | (SEQ ID NO.: 56, JM#145) |
| IYRWSRKMPCLS | (SEQ ID NO.: 57, JM#146) |
| ILRWSRK(Glu-Pal)MPCLS | (SEQ ID NO.: 58, JM#148) |
| IVRWSKKVPSVS | (SEQ ID NO.: 60, JM#151) |
| IVRWSK(Pal)K-NH2 | (SEQ ID NO.: 61, JM#164) |
| IVRWSKK(Pal)-NH2 | (SEQ ID NO.: 62, JM#165) |
| IVRWSK(Pal)KVPCVS | (SEQ ID NO.: 65, JM#168) |
| d-ILRWSRK-NH2 | (SEQ ID NO.: 70, JM#173) |
| d-ILRWSRKLP-NH2 | (SEQ ID NO.: 71, JM#174) |
| d-ILRWSRK(Pal)LP-NH2 | (SEQ ID NO.: 72, JM#175) |
| d-LMRWSRK(Pal)MPCVS | (SEQ ID NO.: 73, JM#176) |
| Md-LRWSRK(Pal)LPCVS | (SEQ ID NO.: 74, JM#177) |
| Md-LRWSRK(Pal)LP-NH2 | (SEQ ID NO.: 76, JM#179) |
| Md-LRWSRK(Pal)-NH2 | (SEQ ID NO.: 78, JM#181) |
| ILRWSRK(Ste)LPCVS | (SEQ ID NO.: 79, JM#182) |
| ILRWSRK(Chl)LPCVS | (SEQ ID NO.: 81, JM#184) |
| Ac-ILRWSRKLPCVS | (SEQ ID NO.: 82, JM#185) |
| d-Ac-ILRWSRKLPCVS | (SEQ ID NO.: 83, JM#186) |
| Ac-MLRWSRKLPCVS | (SEQ ID NO.: 84, JM#187) |
| d-Ac-MLRWSRKLPCVS | (SEQ ID NO.: 85, JM#188) |
| VLRWSRKLPCVS | (SEQ ID NO.: 86, JM#189) |
| d-VLRWSRKLPCVS | (SEQ ID NO.: 87, JM#190) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197) |
| ILRWSRK(Glu-Ste)LPCVS | (SEQ ID NO.: 95, JM#198) |
| ILRWSRK(Glu-Myr)LPCVS | (SEQ ID NO.: 99, JM#204) |
- Group 6 consists of
| | |
|---|---|
| d-LMRWSRKK(Glu-Pal)-NH2 | (SEQ ID NO.: 92, JM#195) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197) |
| ILRWSRK(Glu-Ste)LPCVS | (SEQ ID NO.: 95, JM#198) |
- Group 7 consists of
| | |
|---|---|
| Md-LRWSRKLPCVS | (SEQ ID NO.: 45, JM#43) |
| Md-LRWSRKMPCVS | (SEQ ID NO.: 46, JM#44) |
| ILRWSRK(Glu-Pal)LPCVS | (SEQ ID NO.: 54, JM#143) |
| ILRWSRK(Pal)LPCVS | (SEQ ID NO.: 55, JM#144) |
| ILRWSRKLPCK(Glu-Pal)S | (SEQ ID NO.: 56, JM#145) |
| ILRWSRK(Pal)MPCLS | (SEQ ID NO.: 59, JM#149) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| d-ILRWSRK-NH2 | (SEQ ID NO.: 70, JM#173) |
| d-ILRWSRKLP-NH2 | (SEQ ID NO.: 71, JM#174) |
- Group 8 consists of
| | |
|---|---|
| d-LLRWSRKMPCVS | (SEQ ID NO.: 31, JM#29) |
| ILRWSRK(Pal)L-NH2 | (SEQ ID NO.: 69, JM#172) |
| ILRWSRK(Glu-Pal)-NH2 | (SEQ ID NO.: 89, JM#192) |
- Group 9 consists of
| | |
|---|---|
| Ac-SLRWSRKMPCVS | (SEQ ID NO.: 27, JM#25) |
| d-MLRWSRKMPCVS | (SEQ ID NO.: 30, JM#28) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-LLRWSRK(Glu-Pal)MPCVS | (SEQ ID NO.: 52, JM#140) |
| d-LMRWSRK(Pal)MP-NH2 | (SEQ ID NO.: 75, JM#178) |
- Group 10 consists of
| | |
|---|---|
| ILRWSRK(Pal)LPCVS | (SEQ ID NO.: 55, JM#144) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRK(Pal)L-NH2 | (SEQ ID NO.: 69, JM#172) |
| d-LMRWSRK(Pal)-NH2 | (SEQ ID NO.: 77, JM#180) |
| ILRWSRKK(Glu-Pal)-NH2 | (SEQ ID NO.: 88, JM#191) |
| ILRWSRK(Glu-Pal)-NH2 | (SEQ ID NO.: 89, JM#192) |
| ILRWSRKLK(Glu-Pal)-NH2 | (SEQ ID NO.: 90, JM#193) |
| d-LMRWSRK(Glu-Pal)-NH2 | (SEQ ID NO.: 91, JM#194) |
wherein d- in front of an amino acid indicates a D-Amino acid, Pal indicates a palmitic acid on the preceding amino acid, Glu-Pal indicates a palmitic acid on the preceding amino acid with a glutamate linker, Dec indicates decanoic acid on the preceding amino acid, Glu-Dec indicates a decanoic acid on the preceding amino acid with a glutamate linker, Myr indicates myristic acid on the preceding amino acid, Glu-Myr indicates a myristic acid on the preceding amino acid with a glutamate linker, Ole indicates oleic acid on the preceding amino acid, Ste indicates stearic acid on the preceding amino acid, Glu-Ste indicates a stearic acid on the preceding amino acid with a glutamate linker, Chi indicates Cholesterol, and Ac indicates a substitution of an amino group by an acetyl group

2. A peptide consisting of one of the following amino acid sequences, selected from any of the groups 1 to 10, wherein
- Group 1 consists of
| | |
|---|---|
| ILRWSRKMPCVS | (SEQ ID NO.: 15, JM#13) |
| IMRWSRKMPCVS | (SEQ ID NO.: 19, JM#17) |
| ILRWSRKMPCLS | (SEQ ID NO.: 20, JM#18) |
| ILRWSRKMPCMS | (SEQ ID NO.: 22, JM#20) |
| ILRWSRKLPCVS | (SEQ ID NO.: 23, JM#21) |
| ILRWSRKFPCVS | (SEQ ID NO.: 24, JM#22) |
| ILRWSRK(Pal)LPCVS | (SEQ ID NO.: 55, JM#144) |
| ILRWSRK(Glu-Pal)-NH2 | (SEQ ID NO.: 89, JM#192) |
| d-LMRWSRK(Glu-Pal)-NH2 | (SEQ ID NO.: 91, JM#194) |
- Group 2 consists of
| | |
|---|---|
| ILRWSRKMPCFS | (SEQ ID NO.: 21, JM#19) |
| ILRWSRK(Pal)L-NH2 | (SEQ ID NO.: 69, JM#172) |
| d-LMRWSRK(Pal)MP-NH2 | (SEQ ID NO.: 75, JM#178) |
| d-LMRWSRK(Pal)-NH2 | (SEQ ID NO.: 77, JM#180) |
| ILRWSRK(Ole)LPCVS | (SEQ ID NO.: 80, JM#183) |
- Group 3 consists of
| | |
|---|---|
| d-LLRWSRK(Pal)MPCVS | (SEQ ID NO.: 53, JM#141) |
| IVRWSK(Pal)KVP-NH2 | (SEQ ID NO.: 63, JM#166) |
| IVRWSKK(Pal)VP-NH2 | (SEQ ID NO.: 64, JM#167) |
| IVRWSKK(Pal)VPCVS | (SEQ ID NO.: 66, JM#169) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRKK(Glu-Pal)-NH2 | (SEQ ID NO.: 88, JM#191) |
| ILRWSRKLK(Glu-Pal)-NH2 | (SEQ ID NO.: 90, JM#193) |
| ILRWSRKK(Glu-Ste)LPCVS | (SEQ ID NO.: 96, JM#199) |
| ILRWSRK(Glu-Dec)LPCVS | (SEQ ID NO.: 98, JM#203) |
| ILRWSRK(Glu-Ste)LPCVS | (SEQ ID NO.: 100, JM#205) |
- Group 4 consists of
| | |
|---|---|
| ILRWSRKVPCVS | (SEQ ID NO.: 10, JM#8) |
| IFRWSRKVPCVS | (SEQ ID NO.: 12, JM#10) |
| MLRWSRKMPCVS | (SEQ ID NO.: 29, JM#27) |
| MMRWSRKMPCVS | (SEQ ID NO.: 36, JM#34) |
| MLRWSRKLPCVS | (SEQ ID NO.: 41, JM#39) |
| ILRWSRKLPSVS | (SEQ ID NO.: 51, JM#122) |
| d-LLRWSRK(Glu-Pal)MPCVS | (SEQ ID NO.: 52, JM#140) |
| ILRWSRK(Pal)MPCLS | (SEQ ID NO.: 59, JM#149) |
| d-LMRWSRKK(Glu-Pal)-NH2 | (SEQ ID NO.: 92, JM#195) |
| ILRWSRK-AcLPCVS | (SEQ ID NO.: 97, JM#200) |
- Group 5 consisting of
| | |
|---|---|
| ILRWSKKVPCVS | (SEQ ID NO.: 3, JM#1) |
| IFRWSKKVPCVS | (SEQ ID NO.: 4, JM#2) |
| IVRWSRKVPCVS | (SEQ ID NO.: 5, JM#3) |
| IVRWSHKVPCVS | (SEQ ID NO.: 6, JM#4) |
| IVRWSKKLPCVS | (SEQ ID NO.: 7, JM#5) |
| IVRWSKKIPCVS | (SEQ ID NO.: 8, JM#6) |
| IVRWSKKFPCVS | (SEQ ID NO.: 9, JM#7) |
| ILRWSHKVPCVS | (SEQ ID NO.: 11, JM#9) |
| IFRWSHKVPCVS | (SEQ ID NO.: 13, JM#11) |
| IVRWSKKMPCVS | (SEQ ID NO.: 14, JM#12) |
| IVRWSKKVPCd-VS | (SEQ ID NO.: 16, JM#14) |
| ILRWSRKVPCd-VS | (SEQ ID NO.: 17, JM#15) |
| IIRWSRKMPCVS | (SEQ ID NO.: 18, JM#16) |
| ILRWSRKVPSVS | (SEQ ID NO.: 25, JM#23) |
| ILRWSRKMPSVS | (SEQ ID NO.: 26, JM#24) |
| Ac-SLRWSRKMPCVS | (SEQ ID NO.: 27, JM#25) |
| d-Ac-SLRWSRKMPCVS | (SEQ ID NO.: 28, JM#26) |
| d-MLRWSRKMPCVS | (SEQ ID NO.: 30, JM#28) |
| d-LLRWSRKMPCVS | (SEQ ID NO.: 31, JM#29) |
| d-FLRWSRKMPCVS | (SEQ ID NO.: 32, JM#30) |
| GLRWSRKMPCVS | (SEQ ID NO.: 33, JM#31) |
| Ac-SMRWSRKMPCVS | (SEQ ID NO.: 34, JM#32) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-FMRWSRKMPCVS | (SEQ ID NO.: 39, JM#37) |
| d-GMRWSRKMPCVS | (SEQ ID NO.: 40, JM#38) |
| d-MLRWSRKLPCVS | (SEQ ID NO.: 42, JM#40) |
| Id-LRWSRKLPCVS | (SEQ ID NO.: 43, JM#41) |
| Id-LRWSRKMPCVS | (SEQ ID NO.: 44, JM#42) |
| Md-LRWSRKLPCVS | (SEQ ID NO.: 45, JM#43) |
| Md-LRWSRKMPCVS | (SEQ ID NO.: 46, JM#44) |
| IVRWSKKVP-NH2 | (SEQ ID NO.: 47, JM#106) |
| IVRWSKK-NH2 | (SEQ ID NO.: 48, JM#110) |
| ILRWSRKLP-NH2 | (SEQ ID NO.: 49, JM#114) |
| ILRWSRK-NH2 | (SEQ ID NO.: 50, JM#118) |
| ILRWSRK(Glu-Pal)LPCVS | (SEQ ID NO.: 54, JM#143) |
| ILRWSRKLPCK(Glu-Pal)S | (SEQ ID NO.: 56, JM#145) |
| IYRWSRKMPCLS | (SEQ ID NO.: 57, JM#146) |
| ILRWSRK(Glu-Pal)MPCLS | (SEQ ID NO.: 58, JM#148) |
| IVRWSKKVPSVS | (SEQ ID NO.: 60, JM#151) |
| IVRWSK(Pal)K-NH2 | (SEQ ID NO.: 61, JM#164) |
| IVRWSKK(Pal)-NH2 | (SEQ ID NO.: 62, JM#165) |
| IVRWSK(Pal)KVPCVS | (SEQ ID NO.: 65, JM#168) |
| d-ILRWSRK-NH2 | (SEQ ID NO.: 70, JM#173) |
| d-ILRWSRKLP-NH2 | (SEQ ID NO.: 71, JM#174) |
| d-ILRWSRK(Pal)LP-NH2 | (SEQ ID NO.: 72, JM#175) |
| d-LMRWSRK(Pal)MPCVS | (SEQ ID NO.: 73, JM#176) |
| Md-LRWSRK(Pal)LPCVS | (SEQ ID NO.: 74, JM#177) |
| Md-LRWSRK(Pal)LP-NH2 | (SEQ ID NO.: 76, JM#179) |
| Md-LRWSRK(Pal)-NH2 | (SEQ ID NO.: 78, JM#181) |
| ILRWSRK(Ste)LPCVS | (SEQ ID NO.: 79, JM#182) |
| ILRWSRK(Chl)LPCVS | (SEQ ID NO.: 81, JM#184) |
| Ac-ILRWSRKLPCVS | (SEQ ID NO.: 82, JM#185) |
| d-Ac-ILRWSRKLPCVS | (SEQ ID NO.: 83, JM#186) |
| Ac-MLRWSRKLPCVS | (SEQ ID NO.: 84, JM#187) |
| d-Ac-MLRWSRKLPCVS | (SEQ ID NO.: 85, JM#188) |
| VLRWSRKLPCVS | (SEQ ID NO.: 86, JM#189) |
| d-VLRWSRKLPCVS | (SEQ ID NO.: 87, JM#190) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197) |
| ILRWSRK(Glu-Ste)LPCVS | (SEQ ID NO.: 95, JM#198) |
| ILRWSRK(Glu-Myr)LPCVS | (SEQ ID NO.: 99, JM#204) |
| - Group 6 consists of | |
| d-LMRWSRKK(Glu-Pal)-NH2 | (SEQ ID NO.: 92, JM#195) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197) |
| ILRWSRK(Glu-Ste)LPCVS | (SEQ ID NO.: 95, JM#198) |
- Group 7 consists of
| | |
|---|---|
| Md-LRWSRKLPCVS | (SEQ ID NO.: 45, JM#43) |
| Md-LRWSRKMPCVS | (SEQ ID NO.: 46, JM#44) |
| ILRWSRK(Glu-Pal)LPCVS | (SEQ ID NO.: 54, JM#143) |
| ILRWSRK(Pal)LPCVS | (SEQ ID NO.: 55, JM#144) |
| ILRWSRKLPCK(Glu-Pal)S | (SEQ ID NO.: 56, JM#145) |
| ILRWSRK(Pal)MPCLS | (SEQ ID NO.: 59, JM#149) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| d-ILRWSRK-NH2 | (SEQ ID NO.: 70, JM#173) |
| d-ILRWSRKLP-NH2 | (SEQ ID NO.: 71, JM#174) |
- Group 8 consists of
| | |
|---|---|
| d-LLRWSRKMPCVS | (SEQ ID NO.: 31, JM#29) |
| ILRWSRK(Pal)L-NH2 | (SEQ ID NO.: 69, JM#172) |
| ILRWSRK(Glu-Pal)-NH2 | (SEQ ID NO.: 89, JM#192) |
- Group 9 consists of
| | |
|---|---|
| Ac-SLRWSRKMPCVS | (SEQ ID NO.: 27, JM#25) |
| d-MLRWSRKMPCVS | (SEQ ID NO.: 30, JM#28) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-LLRWSRK(Glu-Pal)MPCVS | (SEQ ID NO.: 52, JM#140) |
| d-LMRWSRK(Pal)MP-NH2 | (SEQ ID NO.: 75, JM#178) |
- Group 10 consists of
| | |
|---|---|
| ILRWSRK(Pal)LPCVS | (SEQ ID NO.: 55, JM#144) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRK(Pal)L-NH2 | (SEQ ID NO.: 69, JM#172) |
| d-LMRWSRK(Pal)-NH2 | (SEQ ID NO.: 77, JM#180) |
| ILRWSRKK(Glu-Pal)-NH2 | (SEQ ID NO.: 88, JM#191) |
| ILRWSRK(Glu-Pal)-NH2 | (SEQ ID NO.: 89, JM#192) |
| ILRWSRKLK(Glu-Pal)-NH2 | (SEQ ID NO.: 90, JM#193) |
| d-LMRWSRK(Glu-Pal)-NH2 | (SEQ ID NO.: 91, JM#194) |
wherein d- in front of an amino acid indicates a D-Amino acid, Pal indicates a palmitic acid on the preceding amino acid, Glu-Pal indicates a palmitic acid on the preceding amino acid with a glutamate linker, Dec indicates decanoic acid on the preceding amino acid, Glu-Dec indicates a decanoic acid on the preceding amino acid with a glutamate linker, Myr indicates myristic acid on the preceding amino acid, Glu-Myr indicates a myristic acid on the preceding amino acid with a glutamate linker, Ole indicates oleic acid on the preceding amino acid, Ste indicates stearic acid on the preceding amino acid, Glu-Ste indicates a stearic acid on the preceding amino acid with a glutamate linker, Chl indicates Cholesterol, and Ac indicates a substitution of an amino group by an acetyl group,
and wherein the peptide is C-terminally conjugated to a complexing agent.

3. A peptide consisting of one of the following amino acid sequences, selected from any of the groups 1 to 10, wherein
- Group 1 consists of
| | |
|---|---|
| ILRWSRKMPCVS | (SEQ ID NO.: 15, JM#13) |
| IMRWSRKMPCVS | (SEQ ID NO.: 19, JM#17) |
| ILRWSRKMPCLS | (SEQ ID NO.: 20, JM#18) |
| ILRWSRKMPCMS | (SEQ ID NO.: 22, JM#20) |
| ILRWSRKLPCVS | (SEQ ID NO.: 23, JM#21) |
| ILRWSRKFPCVS | (SEQ ID NO.: 24, JM#22) |
| ILRWSRK(Pal)LPCVS | (SEQ ID NO.: 55, JM#144) |
| ILRWSRK(Glu-Pal)-NH2 | (SEQ ID NO.: 89, JM#192) |
| d-LMRWSRK(Glu-Pal)-NH2 | (SEQ ID NO.: 91, JM#194) |
- Group 2 consists of
| | |
|---|---|
| ILRWSRKMPCFS | (SEQ ID NO.: 21, JM#19) |
| ILRWSRK(Pal)L-NH2 | (SEQ ID NO.: 69, JM#172) |
| d-LMRWSRK(Pal)MP-NH2 | (SEQ ID NO.: 75, JM#178) |
| d-LMRWSRK(Pal)-NH2 | (SEQ ID NO.: 77, JM#180) |
| ILRWSRK(Ole)LPCVS | (SEQ ID NO.: 80, JM#183) |
- Group 3 consists of
| | |
|---|---|
| d-LLRWSRK(Pal)MPCVS | (SEQ ID NO.: 53, JM#141) |
| IVRWSK(Pal)KVP-NH2 | (SEQ ID NO.: 63, JM#166) |
| IVRWSKK(Pal)VP-NH2 | (SEQ ID NO.: 64, JM#167) |
| IVRWSKK(Pal)VPCVS | (SEQ ID NO.: 66, JM#169) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRKK(Glu-Pal)-NH2 | (SEQ ID NO.: 88, JM#191) |
| ILRWSRKLK(Glu-Pal)-NH2 | (SEQ ID NO.: 90, JM#193) |
| ILRWSRKK(Glu-Ste)LPCVS | (SEQ ID NO.: 96, JM#199) |
| ILRWSRK(Glu-Dec)LPCVS | (SEQ ID NO.: 98, JM#203) |
| ILRWSRK(Glu-Ste)LPCVS | (SEQ ID NO.: 100, JM#205) |
- Group 4 consists of
| | |
|---|---|
| ILRWSRKVPCVS | (SEQ ID NO.: 10, JM#8) |
| IFRWSRKVPCVS | (SEQ ID NO.: 12, JM#10) |
| MLRWSRKMPCVS | (SEQ ID NO.: 29, JM#27) |
| MMRWSRKMPCVS | (SEQ ID NO.: 36, JM#34) |
| MLRWSRKLPCVS | (SEQ ID NO.: 41, JM#39) |
| ILRWSRKLPSVS | (SEQ ID NO.: 51, JM#122) |
| d-LLRWSRK(Glu-Pal)MPCVS | (SEQ ID NO.: 52, JM#140) |
| ILRWSRK(Pal)MPCLS | (SEQ ID NO.: 59, JM#149) |
| d-LMRWSRKK(Glu-Pal)-NH2 | (SEQ ID NO.: 92, JM#195) |
| ILRWSRK-AcLPCVS | (SEQ ID NO.: 97, JM#200) |
- Group 5 consisting of
| | |
|---|---|
| ILRWSKKVPCVS | (SEQ ID NO.: 3, JM#1) |
| IFRWSKKVPCVS | (SEQ ID NO.: 4, JM#2) |
| IVRWSRKVPCVS | (SEQ ID NO.: 5, JM#3) |
| IVRWSHKVPCVS | (SEQ ID NO.: 6, JM#4) |
| IVRWSKKLPCVS | (SEQ ID NO.: 7, JM#5) |
| IVRWSKKIPCVS | (SEQ ID NO.: 8, JM#6) |
| IVRWSKKFPCVS | (SEQ ID NO.: 9, JM#7) |
| ILRWSHKVPCVS | (SEQ ID NO.: 11, JM#9) |
| IFRWSHKVPCVS | (SEQ ID NO.: 13, JM#11) |
| IVRWSKKMPCVS | (SEQ ID NO.: 14, JM#12) |
| IVRWSKKVPCd-VS | (SEQ ID NO.: 16, JM#14) |
| ILRWSRKVPCd-VS | (SEQ ID NO.: 17, JM#15) |
| IIRWSRKMPCVS | (SEQ ID NO.: 18, JM#16) |
| ILRWSRKVPSVS | (SEQ ID NO.: 25, JM#23) |
| ILRWSRKMPSVS | (SEQ ID NO.: 26, JM#24) |
| Ac-SLRWSRKMPCVS | (SEQ ID NO.: 27, JM#25) |
| d-Ac-SLRWSRKMPCVS | (SEQ ID NO.: 28, JM#26) |
| d-MLRWSRKMPCVS | (SEQ ID NO.: 30, JM#28) |
| d-LLRWSRKMPCVS | (SEQ ID NO.: 31, JM#29) |
| d-FLRWSRKMPCVS | (SEQ ID NO.: 32, JM#30) |
| GLRWSRKMPCVS | (SEQ ID NO.: 33, JM#31) |
| Ac-SMRWSRKMPCVS | (SEQ ID NO.: 34, JM#32) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-FMRWSRKMPCVS | (SEQ ID NO.: 39, JM#37) |
| d-GMRWSRKMPCVS | (SEQ ID NO.: 40, JM#38) |
| d-MLRWSRKLPCVS | (SEQ ID NO.: 42, JM#40) |
| Id-LRWSRKLPCVS | (SEQ ID NO.: 43, JM#41) |
| Id-LRWSRKMPCVS | (SEQ ID NO.: 44, JM#42) |
| Md-LRWSRKLPCVS | (SEQ ID NO.: 45, JM#43) |
| Md-LRWSRKMPCVS | (SEQ ID NO.: 46, JM#44) |
| IVRWSKKVP-NH2 | (SEQ ID NO.: 47, JM#106) |
| IVRWSKK-NH2 | (SEQ ID NO.: 48, JM#110) |
| ILRWSRKLP-NH2 | (SEQ ID NO.: 49, JM#114) |
| ILRWSRK-NH2 | (SEQ ID NO.: 50, JM#118) |
| ILRWSRK(Glu-Pal)LPCVS | (SEQ ID NO.: 54, JM#143) |
| ILRWSRKLPCK(Glu-Pal)S | (SEQ ID NO.: 56, JM#145) |
| IYRWSRKMPCLS | (SEQ ID NO.: 57, JM#146) |
| ILRWSRK(Glu-Pal)MPCLS | (SEQ ID NO.: 58, JM#148) |
| IVRWSKKVPSVS | (SEQ ID NO.: 60, JM#151) |
| IVRWSK(Pal)K-NH2 | (SEQ ID NO.: 61, JM#164) |
| IVRWSKK(Pal)-NH2 | (SEQ ID NO.: 62, JM#165) |
| IVRWSK(Pal)KVPCVS | (SEQ ID NO.: 65, JM#168) |
| d-ILRWSRK-NH2 | (SEQ ID NO.: 70, JM#173) |
| d-ILRWSRKLP-NH2 | (SEQ ID NO.: 71, JM#174) |
| d-ILRWSRK(Pal)LP-NH2 | (SEQ ID NO.: 72, JM#175) |
| d-LMRWSRK(Pal)MPCVS | (SEQ ID NO.: 73, JM#176) |
| Md-LRWSRK(Pal)LPCVS | (SEQ ID NO.: 74, JM#177) |
| Md-LRWSRK(Pal)LP-NH2 | (SEQ ID NO.: 76, JM#179) |
| Md-LRWSRK(Pal)-NH2 | (SEQ ID NO.: 78, JM#181) |
| ILRWSRK(Ste)LPCVS | (SEQ ID NO.: 79, JM#182) |
| ILRWSRK(Chl)LPCVS | (SEQ ID NO.: 81, JM#184) |
| Ac-ILRWSRKLPCVS | (SEQ ID NO.: 82, JM#185) |
| d-Ac-ILRWSRKLPCVS | (SEQ ID NO.: 83, JM#186) |
| Ac-MLRWSRKLPCVS | (SEQ ID NO.: 84, JM#187) |
| d-Ac-MLRWSRKLPCVS | (SEQ ID NO.: 85, JM#188) |
| VLRWSRKLPCVS | (SEQ ID NO.: 86, JM#189) |
| d-VLRWSRKLPCVS | (SEQ ID NO.: 87, JM#190) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197) |
| ILRWSRK(Glu-Ste)LPCVS | (SEQ ID NO.: 95, JM#198) |
| ILRWSRK(Glu-Myr)LPCVS | (SEQ ID NO.: 99, JM#204) |
- Group 6 consists of
| | |
|---|---|
| d-LMRWSRKK(Glu-Pal)-NH2 | (SEQ ID NO.: 92, JM#195) |
| d-MLRWSRK(Glu-Pal)-NH2 | (SEQ ID NO.: 93, JM#196) |
| d-MLRWSRKK(Glu-Pal)-NH2 | (SEQ ID NO.: 94, JM#197) |
| ILRWSRK(Glu-Ste)LPCVS | (SEQ ID NO.: 95, JM#198) |
- Group 7 consists of
| | |
|---|---|
| Md-LRWSRKLPCVS | (SEQ ID NO.: 45, JM#43) |
| Md-LRWSRKMPCVS | (SEQ ID NO.: 46, JM#44) |
| ILRWSRK(Glu-Pal)LPCVS | (SEQ ID NO.: 54, JM#143) |
| ILRWSRK(Pal)LPCVS | (SEQ ID NO.: 55, JM#144) |
| ILRWSRKLPCK(Glu-Pal)S | (SEQ ID NO.: 56, JM#145) |
| ILRWSRK(Pal)MPCLS | (SEQ ID NO.: 59, JM#149) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| d-ILRWSRK-NH2 | (SEQ ID NO.: 70, JM#173) |
| d-ILRWSRKLP-NH2 | (SEQ ID NO.: 71, JM#174) |
- Group 8 consists of
| | |
|---|---|
| d-LLRWSRKMPCVS | (SEQ ID NO.: 31, JM#29) |
| ILRWSRK(Pal)L-NH2 | (SEQ ID NO.: 69, JM#172) |
| ILRWSRK(Glu-Pal)-NH2 | (SEQ ID NO.: 89, JM#192) |
- Group 9 consists of
| | |
|---|---|
| Ac-SLRWSRKMPCVS | (SEQ ID NO.: 27, JM#25) |
| d-MLRWSRKMPCVS | (SEQ ID NO.: 30, JM#28) |
| d-Ac-SMRWSRKMPCVS | (SEQ ID NO.: 35, JM#33) |
| d-MMRWSRKMPCVS | (SEQ ID NO.: 37, JM#35) |
| d-LMRWSRKMPCVS | (SEQ ID NO.: 38, JM#36) |
| d-LLRWSRK(Glu-Pal)MPCVS | (SEQ ID NO.: 52, JM#140) |
| d-LMRWSRK(Pal)MP-NH2 | (SEQ ID NO.: 75, JM#178) |
- Group 10 consists of
| | |
|---|---|
| ILRWSRK(Pal)LPCVS | (SEQ ID NO.: 55, JM#144) |
| IVRWSK(Pal)KVP-NH2 | (SEQ ID NO.: 63, JM#166) |
| IVRWSKK(Pal)VP-NH2 | (SEQ ID NO.: 64, JM#167) |
| ILRWSRK(Pal)-NH2 | (SEQ ID NO.: 67, JM#170) |
| ILRWSRK(Pal)LP-NH2 | (SEQ ID NO.: 68, JM#171) |
| ILRWSRK(Pal)L-NH2 | (SEQ ID NO.: 69, JM#172) |
| d-LMRWSRK(Pal)-NH2 | (SEQ ID NO.: 77, JM#180) |
| ILRWSRKK(Glu-Pal)-NH2 | (SEQ ID NO.: 88, JM#191) |
| ILRWSRK(Glu-Pal)-NH2 | (SEQ ID NO.: 89, JM#192) |
| ILRWSRKLK(Glu-Pal)-NH2 | (SEQ ID NO.: 90, JM#193) |
| d-LMRWSRK(Glu-Pal)-NH2 | (SEQ ID NO.: 91, JM#194) |
wherein d- in front of an amino acid indicates a D-Amino acid, Pal indicates a palmitic acid on the preceding amino acid, Glu-Pal indicates a palmitic acid on the preceding amino acid with a glutamate linker, Dec indicates decanoic acid on the preceding amino acid, Glu-Dec indicates a decanoic acid on the preceding amino acid with a glutamate linker, Myr indicates myristic acid on the preceding amino acid, Glu-Myr indicates a myristic acid on the preceding amino acid with a glutamate linker, Ole indicates oleic acid on the preceding amino acid, Ste indicates stearic acid on the preceding amino acid, Glu-Ste indicates a stearic acid on the preceding amino acid with a glutamate linker, Chl indicates Cholesterol, and Ac indicates a substitution of an amino group by an acetyl group
, wherein the peptide is coupled to a polymer.

4. The peptide according to claim 4, wherein the peptide is coupled to PEG.

5. The peptide according to claim 5, wherein the peptide is coupled to 1,2-distearoyl-sn-glycero-3-phosphoethanolamine.

6. The peptide according to any of claims 6 or 7, wherein the peptide is coupled to a poly(vinyl alcohol).

7. The peptide according to any of claims 5 or 6, wherein the peptide is a poly(vinyl pyrrolidone).

8. A peptide consisting of two identical monomeric peptides according to any of the preceding claims, wherein the monomeric peptides are linked to each other via a cysteine bridge which is formed between the monomeric peptides to form a dimeric peptide.

9. The peptide or the pharmaceutical composition according to any of the preceding claims for use in medicine.

10. Pharmaceutical composition comprising the peptide according to any of the preceding claims, together with at least one pharmaceutically acceptable carrier, mesoporous nanoparticles, cryoprotectant, lyoprotectant, excipient and/or diluent.

11. Use of the peptide or the pharmaceutical composition according to any of the preceding claims for the preparation of a formulation for oral administration, inhalation, intravenous administration, topical administration, intranasal, intraperitoneal administration, subcutaneous administration and/or any other injectable form.

12. Use according to claim 11, wherein the peptide or the pharmaceutical composition are used for the preparation of a lyophilized formulation of a buffered liquid formulation.

13. The peptide or the pharmaceutical composition according to any of the preceding claims for use in theranostics, in the treatment of disorders of hematopoiesis, in particular for support of the mobilization, proliferation and migration of stem cells; in the treatment of wounds, in particular wounds caused by burning; in the treatment of viral diseases, in particular infections with HIV-1, HIV-2, Cytomegalovirus, Herpes simplex virus (type 1 and 2), Varicella zoster virus, Hepatitis A and Hepatitis B virus, Influenza virus, Polio virus, Rhino virus, Rubella virus, Measles virus, Rabies virus, Rous sarcoma virus, Epstein-Barr Virus; in the treatment of infections caused by bacteria and fungi, in particular Pseudomonas, Candida, S. aureus; in the treatment of infectious processes, abnormal infectious processes; in the treatment of inflammation, in particular of periodontal disease; in the treatment of growth disorders; in the treatment of neuronal diseases, disorders of the blood clotting cascade and hematopoiesis, vascular diseases, diseases of the immune system, for improving wound and bone healing, for use in the treatment of neurological diseases, in particular stroke, Parkinson's disease, Alzheimer's disease, multiple sclerosis; in the treatment of the Warts, Hypogammaglobulinemia, Immunodeficiency, and Myelokathexis syndrome and rheumatoide arthritis; in the treatment of cancers, in particular cancers showing the CXCR receptor, preferably cancer of the liver, pancreas, prostate, breast cancer or other solid tumors; in the treatment of lack of mobilization, proliferation and migration of stem cells, T-cell activation as well as support of immunoblasts, preferably of cytotoxic T lymphocytes with programmed cell death receptor 1; in the treatment of antifibrosis; in the treatment or prevention of scars; in the treatment of cardiologic disorders, in particular heart insufficiency; in the treatment of metabolic disorders, in particular diabetes.

14. A method of prophylaxis and/or treatment of cancer, viral diseases, metabolic disorders, neurologic disorders, diseases of the immune system, and disorders of the blood clotting cascade and hematopoiesis in a mammal, including a human.

15. A method for manufacturing the peptide according to any of the preceding claims by solid phase synthesis.
